# EUROPEAN PATENT SPECIFICATION

(11) **EP 0 528 699 B1**
(45) Date of publication and mention of the grant of the patent: **06.11.1996**
(21) Application number: 92307648.3
(22) Date of filing: 21.08.1992
(51) Int. Cl.: C07C 49/753, C07C 69/013, A61K 31/12, A61K 31/215, A61K 31/35, C07D 311/94, C07C 205/57

(54) **Saishin N derivatives, process for their preparation and anti-ulcer agents containing them**
Saishin N-Derivate, ihre Herstellungsverfahren und diese enthaltende Antitumormittel
Dérivés de la Saishin N, leur procédé de préparation et agent anti-ulcère les contenant

(30) Priority: 21.08.1991 JP 291834/91
(43) Date of publication of application: 24.02.1993
(73) Proprietor: TOKYO TANABE COMPANY LIMITED, Chuo-ku Tokyo 103 (JP)
(72) Inventor: Yokura, Susumu, Kawagoe-shi, Saitama (JP); Murakami, Kiyokazu, Yokohama-shi, Kanagawa (JP); Takoi, Nobuo, Musashino-shi, Tokyo (JP); Iizuka, Hiroyuki, c/o Setagaya's Company Residence, Setagaya-ku, Tokyo (JP); Ohtubo, Eiji, Yono-shi, Saitama (JP)
(74) Representative: Dzieglewska, Hanna Eva

(56) References cited:
- EP-A- 0 443 848
- GB-A- 2 179 651

## Description

The present invention relates to a terpenoid compound being a derivative of Saishin N, a process for preparing the derivative and an anti-ulcer agent containing the derivative as an active component. The present invention relates more particularly to a Saishin N derivative useful as a medicament in the treatment of peptic ulcers, a process for preparing the derivative and an anti-ulcer agent containing the derivative as an active component.

Saishin N, 4,5-dihydroxy-2,6,6-trimethyl-2-cycloheptene-1-one has previously been isolated from the crude drug "Saishin" and identified by us as an anti-ulcer agent. A chemical method for preparing Saishin N is described in Japanese Unexamined Patent Publication No. 275640/1991.

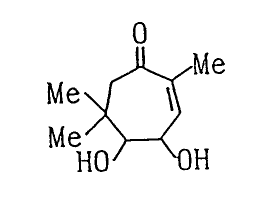

The Saishin N molecule has two hydroxy groups having no significant differences between them. It is therefore difficult to react one of the hydroxy groups selectively, which has up to now presented an obstacle to the study of Saishin N derivatives.

We have now found however that (1) when eucarvone-4,5-oxide (chemical name; 4,5-epoxy-2,6,6-trimethyl-2-cycloheptene-1-one) which is an intermediate of the production of the Saishin N is subjected to an alcoholysis reaction in the presence of an acid catalyst, a Saishin N derivative having an alkoxy group in the 4-position and a hydroxy group in the 5-position may be selectively obtained as the major product; and (2) when Saishin N and a carboxylic acid are subjected to an acylation reaction using carbodiimide, a Saishin N derivative having an acyloxy group in the 4-position and a hydroxy group in the 5-position may be selectively obtained.

The inventors have further studied the modification of the hydroxy groups in the 4- and 5-positions, the reduction of a double bond, the reduction of an oxo group to a hydroxy group and the modification of the hydroxy group and have obtained compounds having an improved anti-ulcer effect over Saishin N.

Viewed from one aspect the invention provides compounds of formula I 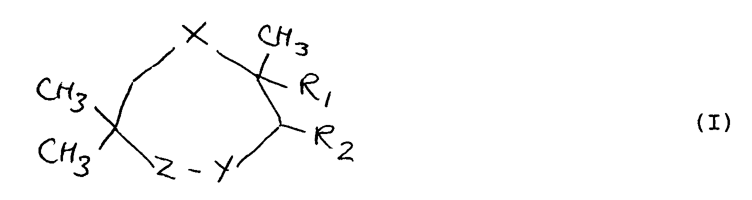 wherein
X represents a group -CR₃R₅-;
R₁ and R₂ each represent hydrogen atoms or R₁ and R₂ together represent a carbon-carbon bond;
Y and Z independently represent carbonyl groups or groups CHR₃;
R₅ represents a hydrogen atom, and each R₃ independently represents a group OR₄, or R₅ and a group R₃ represent an oxygen atom; and
each R₄ independently represents a hydrogen atom or an optionally substituted alkyl, alkenyl, aralkyl, aralkenyl or acyl group; with the proviso that where Y and Z each represent CHOH groups and R₁ and R₂ together represent a carbon-carbon bond, X represents other than a carbonyl group and the isomers and addition salts thereof.

In the R₄ groups of the compounds of formula I each alkyl or alkenyl moiety, which may be straight chain or branched conveniently contains up to 6 carbon atoms, preferably up to 4 carbon atoms, and each aryl moiety conveniently comprises a 5 to 10 membered homocyclic or heterocyclic aromatic ring or fused ring structure, preferably a 5- or 6-membered aromatic ring optionally with a fused benzene ring. Ring heteroatoms are conveniently nitrogen, sulphur or oxygen, preferably nitrogen or sulphur. The R₄ groups are optionally substituted and examples of such substituents include ethers, thioethers, imines or silyl ethers all of which may be linear or cyclic, hydroxy or protected hydroxy (eg. alkoxy), carboxyl, esters (eg. alkoxycarbonyl groups), alkyl, acyl, acyloxy, halogen, nitro, amino, acylamino, alkylamino, and unsaturated heterocyclic rings (such as thiazolyl groups). Alkyl and aryl components of such substituents are conveniently as defined above for such moieties within R₄.

Examples of suitable alkyl groups for R₄ include an alkyl group having eg. 1 to 4 carbon atoms which may have substituents on the chain. Examples of the substituents include a hydroxy group which may be protected by an acyl group having 1 to 4 carbon atoms, an acetal such as tetrahydropyranyl, tetrahydrothiopyranyl, 4-methoxy tetrahydropyranyl, tetrahydrofuranyl, 1-ethoxyethyl, 1-methyl-1-methoxyethyl and 1-isopropyloxyethyl group or a silylether such as trimethylsilylether, tert-butyldimethylsilylether (hereinafter the same definition is applied to the protective group of the hydroxy group), a carboxyl group and an alkoxycarbonyl group having 1 to 4 carbon atoms.

The term alkenyl may be used hereinafter to refer to alkenyl and aralkenyl and examples of suitable such groups for R₄ include an allyl group, 3-methyl-2-butenyl group and cinnamyl group which may have one or more substituents on the ring.

Examples of suitable aralkyl groups for R₄ include benzyl, phenethyl, thenyl or picolyl groups which may have one or more substituents on the ring.

Examples of the substituents on the ring of the cinnamyl or aralkyl group include an alkyl group having 1 to 4 carbon atoms; an alkoxycarbonyl group having 1 to 4 carbon atoms; an acyl group such as acetyl, benzoyl or cinnamoyl group, a hydroxy group which may be protected; an alkoxy group having 1 to 4 carbon atoms; a fluorine, chlorine, bromine or iodine atom; and a nitro group.

Examples of suitable acyl groups for R₄ include aliphatic acyl groups such as an acetyl, propionyl, butyryl, isobutyryl, valeryl, acryloyl, methacryloyl or 2-butenoyl group which may have substituents on the chain or a cinnamoyl group which may have one or more substituents on the ring; an aromatic acyl group such as a benzoyl or naphthoyl group which may have one or more substituents on the ring; or a heterocyclic acyl group such as a furoyl, pyrrolylcarbonyl, thenoyl, imidazolylcarbonyl and benzimidazolylcarbonyl group which may have one or more substituents on the ring.

Examples of the substituents on the chain include hydroxy groups which may be protected, a carboxyl group, an alkoxycarbonyl group having 1 to 4 carbon atoms and a thiazolyl group. Examples of the substituents on the ring include an alkyl group having 1 to 4 carbon atoms; carboxyl group; alkoxycarbonyl group having 1 to 4 carbon atoms; an acyl group such as acetyl, benzoyl and cinnamoyl group which may have one or more substituents on the chain or ring; a hydroxy group which may be protected; an alkoxy group having 1 to 4 carbon atoms; an acyloxy group such as an acetoxy, propionyloxy or butyryloxy group; a fluorine, chlorine, bromine or iodine atom; nitro group; an amino group which may be substituted by an acyl group such as acetyl, benzoyl or cinnamoyl group or an alkyl group having 1 to 4 carbon atoms.

The compound I of the invention also includes each or the mixture of the stereoisomers which exist due to a plurality of asymmetric carbons.

Viewed from a further aspect the invention also provides a process for preparing the compounds of the invention which process comprises at least one of the following steps:
(a) (to prepare compounds of formula I in which X is carbonyl, Z is CHOH and Y is CHOR₄' where R₄' is as defined for R₄ other than hydrogen or acyl) reacting a compound of formula II 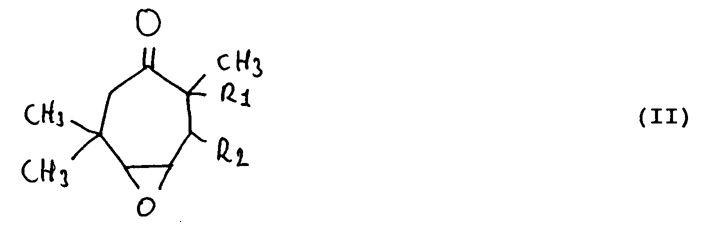 with an alcohol of formula R₄′OH (where R₄′ is as hereinbefore defined);
(b) (to prepare compounds of formula I wherein Y and/or Z represents a group CHOR₄˝ where R₄˝ is an acyl group) acylating a compound of formula I wherein Y and/or Z represents a CHOH group;
(c) (to prepare compounds of formula I in which X is carbonyl, Z is CHOH and Y is CHOR₄′ where R₄′ is as defined for R₄ other than hydrogen or acyl) hydrolysing a compound of formula III 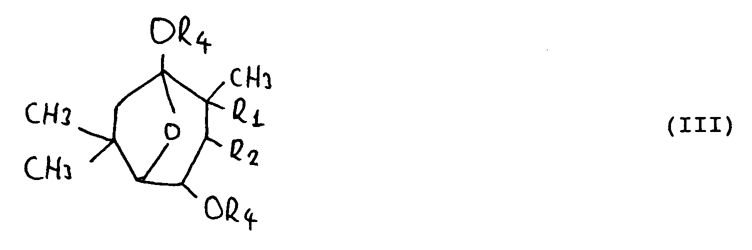
(d) (to prepare compounds of formula I in which X represents a group CR₅OR₄′, Y represents a group CHOR₄′, R₄′ is as hereinbefore defined, Z represents a group CHR₃ and R₅ and the R₃ group of Z together represent an oxygen atom) reacting a compound of formula II 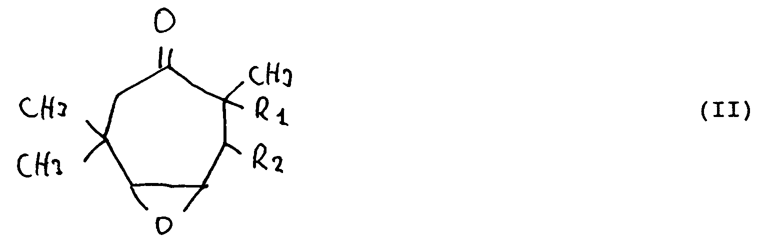 with an alcohol of formula R₄′OH (where R₄′ is as hereinbefore defined);
(e) (to prepare compounds of formula I in which X represents a group CR₅OR₄˝, Y represents a group CHOR₄′, R₄′ and R₄˝ are as hereinbefore defined, Z represents a group CHR₃ and R₅ and the R₃ group of Z together represent an oxygen atom) acylating a compound of formula I wherein X represents a carbonyl group, Y represents a group CHOR₄′ and Z represents a CHOH group;
(f) (to prepare compounds of formula I wherein at least one group R₄ is hydrogen) cleaving a protecting group R₄˝′ (where R₄˝′ is as defined for R₄ other than hydrogen) from a compound of formula I wherein at least one R₄ is a group R₄˝′;
(g) (to prepare compounds of formula I wherein X is carbonyl and Z is a group CHOR′₄) alkylating, alkenylating, aralkylating or aralkenylating a compound of formula IV 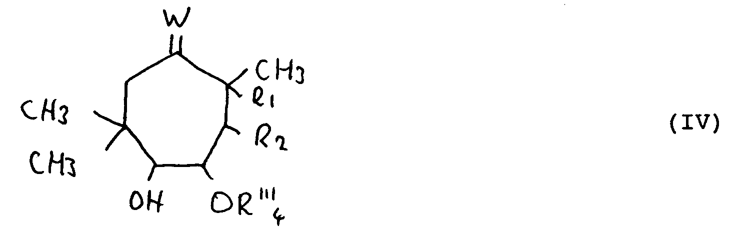 (where R₄˝′ is as hereinbefore defined and W is a carbonyl protecting group), deprotecting the carbonyl group and if desired cleaving protecting group R₄˝′ from the resulting compound of formula I;
(h) (to prepare compounds of formula I wherein X is a group CHOH) reducing a compound of formula I (wherein X is a carbonyl group, Y and Z are both CHOR₄˝′ groups and R₄˝′ is as hereinbefore defined) and if desired cleaving one or both protecting groups R₄˝′ from the resulting compound of formula I;
(i) (to prepare compounds of formula I in which X represents a group CR₅OR₄˝′, one of Y and Z represents a group CHR₃ and the other represents a group CHOR₄ and R₃ and R₅ together represent an oxygen atom) alkylating, alkenylating, aralkylating, aralkenylating or acylating a compound of formula I in which X is a carbonyl group and one of Y and Z is a CHOH group and the other is a CHOR₄ group;
(j) (to prepare compounds of formula I wherein R₁ and R₂ are hydrogen) reducing a compound of formula I (as hereinbefore defined with the exclusion of the proviso and in which R₁ and R₂ together represent a carbon-carbon bond); and
(k) (to prepare compounds of formula I wherein Y or Z represents a carbonyl group) oxidizing a compound of formula I in which Y or Z represents a CHOH group.

In process steps (a) to (k), the starting material may of course be modified to include protecting groups which are split off after the proces is completed.

Examples of the process of the invention are described further below in connection with the preparation of compounds C to S. The starting materials for the process of the invention may be prepared by conventional procedures from Saishin N or known derivatives such as Compound B.

Eucarvone-4,5-oxide (hereinafter referred to as "Compound B") may be prepared by the method described in Japanese Unexamined Patent Publication no. 275640/1991.

### Process 1

The process comprises subjecting compound B to alcoholysis in the presence of an acid catalyst to
obtain Compounds C and K. 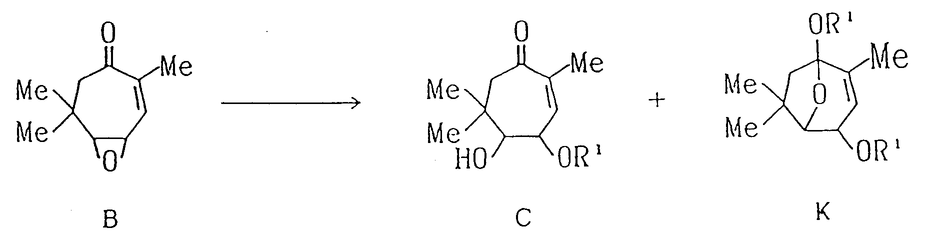 (wherein R¹ represents an alkyl, alkenyl or aralkyl group).

Examples of the alkyl group include an alkyl group having 1 to 4 carbon atoms which may have substituents on the chain. Examples of the substituents on the chain include a hydroxy group which may be protected, a carboxyl group or an alkoxycarbonyl group having 1 to 4 carbon atoms.

Examples of the alkenyl group include an allyl or 3-methyl-2-butenyl group or a cinnamyl group which may have one or more substituents groups on the ring.

Examples of the aralkyl group include a benzyl, phenethyl, furfuryl, thenyl or picolyl group which may have one or more substituents on the ring.

Examples of the substituents on the ring of the cinnamyl of aralkyl group include an alkyl group having 1 to 4 carbon atoms; an alkoxycarbonyl group having 1 to 4 carbon atoms; an acyl group such as acetyl, benzoyl or cinnamoyl group; a hydroxy group which may be protected; an alkoxy group having 1 to 4 carbon atoms; a fluorine, chlorine, bromine or iodine atom; and a nitro group.

The solvent used in the reaction is not critical as long as the mixture of the compound B and the alcohol used in the reaction can dissolve in the solvent, but inert solvents such as aromatic hydrocarbons eg. benzene, toluene, xylene; halogenated hydrocarbons such as dichloromethane, chloroform, carbon tetrachloride; and ethers such as diethylether, tetrahydrofuran, dioxane are preferable. The alcohol itself used in the alcoholysis may be used as a solvent.

Examples of the acid catalyst include inorganic acids such as hydrochloric acid, sulfuric acid, nitric acid, hydrobromic acid and phosphoric acid; Lewis acids such as boron trifluoride etherate, titanium tetrachloride, zinc chloride and tin tetrachloride; or sulfonic acids such as methanesulfonic acid, camphorsulfonic acid, benzenesulfonic acid or toluenesulfonic acid.

Examples of the alcohol include a saturated aliphatic alcohol, an unsaturated aliphatic alcohol or an aralkyl alcohol.

Examples of the saturated aliphatic alcohol include a saturated aliphatic alcohol having 1 to 4 carbon atoms which may have substituents on the chain. Examples of the substituents on the chain include a hydroxy group which may be protected, a carboxyl group or an alkoxycarbonyl group having 1 to 4 carbon atoms.

Examples of the unsaturated aliphatic alcohol include an allylalcohol, 3-methyl-2-buten-1-ol or a cinnamyl alcohol which may have one or more substituents on the ring.

Examples of the aralkylalcohol include a benzylalcohol or phenethyl alcohol, furfurylalcohol, thenylalcohol, or picolylalcahol which may have one or more substituents on the ring.

Examples of the substituents on the ring of cinnamylalcohol or aralkylalcohol include an alkyl group having 1 to 4 carbon atoms; an alkoxycarbonyl group having 1 to 4 carbon atoms; and acyl group such as acetyl, benzoyl or cinnamoyl group; hydroxy group which may be protected; an alkoxy group having 1 to 4 carbon atoms; a fluorine, chlorine, bromine or iodine atom; or a nitro group.

The reaction may be carried out at -70 to 200°C, preferably at 0 to 100°C with or without stirring until the reaction is completed.

After completion of the reaction, the reaction mixture may be subjected to fractionation procedures eg. column chromatography to separate the Compounds C and the bicyclic compounds K.

The compounds K may be converted in high yields to Compound C by acid hydrolysis.

The acid hydrolysis is preferably carried out in water but a polar solvent such as alcohol, tetrahydrofuran, dioxane, acetone, dimethylformamide may be added in order to dissolve the Compound K.

Examples of the acid include an inorganic acid such as hydrochloric acid, sulfuric acid, nitric acid, hydrobromic acid or phosphoric acid, an organic acid such as formic acid, acetic acid, propionic acid or butyric acid; sulfonic acid such as methanesulfonic acid, camphorsulfonic acid, benzenesulfonic acid or toluenesulfonic acid.

The reaction may be carried out at -70 to 200°C, preferably at 0 to 100°C with or without stirring until the reaction is completed.

### Process 2

The process comprises acylating Compound C to obtain Compound D and L. 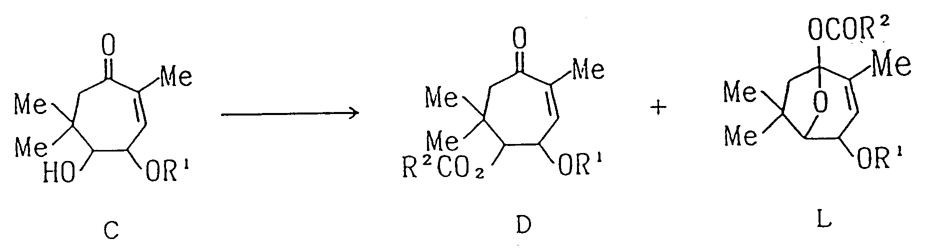 (wherein R¹ has the same meanings as the above and R² represents an alkyl, alkenyl, aromatic hydrocarbon or heterocyclic group.)

Examples of the alkyl group as R² include a lower alkyl group which may have 1 to 4 carbon atoms which may have substituents on the chain. Examples of the substituents on the chain include a hydroxy group which may be protected, a carboxyl group, an alkoxy-carbonyl group having 1 to 4 carbon atoms and a thiazolyl group.

Examples of the alkenyl group as R² include an alkenyl group having 2 to 4 carbon atoms and a styryl group which may have one or more substituents on the ring.

Examples of the aromatic hydrocarbon group as R² include a phenyl or naphthyl group which may have one or more substituents on the ring.

Examples of the heterocyclic group as R² include a furyl, pyrrolyl, thienyl, imidazolyl or benzimidazolyl group which may have one or more substituents on the ring.

Examples of the substituents on the ring of the styryl, aromatic hydrocarbon or heterocyclic group include an alkyl group having 1 to 4 carbon atoms; carboxyl group; an alkoxycarbonyl group having 1 to 4 carbon atoms; an acyl group such as acetyl, benzoyl or cinnamoyl group having one or more substituents on the chain or ring; a hydroxy group which may be protected; an alkoxy group having 1 to 4 carbon atoms; an acyloxy group such as an acetoxy, propionyloxy, butyryloxy, isobutyryloxy or valeryloxy group; a fluorine, chlorine, bromine or iodide atom; a nitro group; an amino group which may be substituted by an acyl group such as an acetyl, benzoyl or cinnamoyl group which may have one or more substituents on the chain or the ring or an alkyl group having 1 to 4 carbon atoms.

The acylation reaction may include a method in which a reaction between a hydroxy group of the Compound C and a carboxylic acid is carried out in the presence of a condensation agent and a method of in which a reaction between a hydroxy group of the Compound C and a carboxylic acid anhydride or a carboxylic acid halide is carried out in the presence of a base.

Examples of carboxylic acid an aliphatic carboxylic acid such as acetic acid, propionic acid, butyric acid, isobutyric acid, valeric acid, isovaleric acid, acrylic acid, methacrylic acid or crotonic acid which may have substituents on the chain or a cinnamic acid which may have one or more substituents on the ring; an aromatic carboxylic acid such as benzoic acid or naphthoic acid which may have one or more substituents on the ring; or a heterocyclic carboxylic acid such as furancarboxylic acid, thiophenecarboxylic acid, pyrrolecarboxylic acid, imidazolecarboxylic acid or benzimidazolecarboxylic acid which may have one or more substituents on the ring. Examples of the substituents on the ring include hydroxy groups which may be protected, an alkoxycarbonyl group having 1 to 4 carbon atoms or a thiazolyl group. Examples of the substituents on the ring include an alkyl group having 1 to 4 carbon atom; an alkoxycarbonyl group having 1 to 4 carbon atoms; an acyl group such as acetyl, benzoyl, cinnamoyl group which may have one or more substituents on the chain or ring; a hydroxy group which may be protected; an alkoxy group having 1 to 4 carbon atoms; an acyloxy group such as acetoxy, propionyloxy, butyryloxy, isobutyryloxy or valeryloxy group; a fluorine, chlorine, bromine or iodine atom; a nitro group; or an amino group which may be protected by an acyl group such as acetyl, benzoyl or cinnamoyl group which may have one or more substituents on the chain or the ring, an alkyl group having 1 to 4 carbon atoms, or a protective group such as benzyl, trityl, or benzyloxycarbonyl group. The activated hydrogen on the heterocyclic ring may be protected by a protective group such as benzyl, trityl or benzyloxycarbonyl group (hereinafter the same is applicable to the heterocyclic ring).

Any condensation agent may be used so long as it is a reagent usually used in a condensation by dehydration reaction. A carbodiimide such a N,N′-dicyclohexylcarbodiimide (hereinafter referred to a "DCC") or 1-cyclohexyl-3-(2-morpholinoethyl) carbodiimide, a 2-halopyridinium salt such as 2-chloro-1-methylpyridinium iodide or 2-bromo-1-ethylpyridinium tetrafluoroborate or 2-chloro-1,3-dimethylimidazolinium chloride may be preferably used.

The carboxylic anhydride is corresponding to the above carboxylic acid or intramolecular acid anhydrides such as phthalic anhydride. The carboxylic halide is corresponding to the above carboxylic acid. The halogen is a chlorine or bromine atom.

Examples of the base include an aromatic amine such as pyridine, picoline, lutidine, 4-dimethylaminopyridine, 4-pyrrolidinopyridine, quinoline, isoquinoline or N,N-dimethylaniline; an aliphatic amine such as trimethylamine, trietylamine, diisopropylethylamine, N-methylpyrrolidine, N-methylpiperidine, or N-methylmorpholine; or an inorganic base such as sodium hydroxide, potassium hydroxide, sodium carbonate, potassium carbonate, sodium bicarbonate and potassium bicarbonate.

The solvent used in the reaction is not limited but may include aromatic hydrocarbons such as benzene, toluene or xylene; alkyl halides such as dichloromethane, chloroform or carbon tetrachloride; ethers such as diethylether, tetrahydrofuran or dioxane; inert solvents such as acetonitrile or ethyl acetate, heterocyclic amines such as pyridine, picoline, lutidine, quinoline or isoquinoline.

The reaction may be carried out at -70 to 200°C, preferably at 0 to 100°C with or without stirring until the reaction is completed.

After completion of the reaction, the product may be subjected to fractionation eg. by column chromatography to separate into the Compound D and the bicylic Compound L.

### Process 3

The process comprises removing oxidatively the alkoxybenzyl group of the Compound D wherein R¹ represents an alkoxybenzyl group to obtain the Compound E. 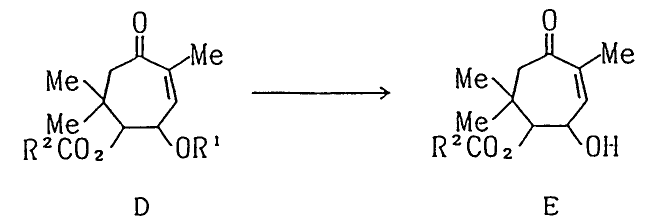 (wherein R¹ represents an alkoxybenzyl group and R² has the same meanings as the above).

Examples of the alkoxybenzyl group include p-methoxybenzyl (hereinafter referred to as "MPM") group and a 2,4-dimethoxybenzyl group.

The solvent used in the reaction include methanol, hydrous dichloromethane, hydrous tetrahydrofuran.

Examples of the oxidizing agent include benzoquinones such as 2,3-dichloro-5,6-dicyanobenzoquinone (hereinafter referred to as "DDQ").

The alkoxybenzyl group may also be removed by an acid hydrolysis reaction using hydrochloric acid, sulfuric acid, nitric acid, tritylfluoroborate, or triarylamine cation or an electrochemical oxidizing reaction.

The reaction may be carried out at -70 to 200°C, preferably at 0 to room temperature with or without stirring until the reaction is completed.

### Process 4

The process comprises acylating Saishin N in a manner corresponding to that described for Process 2 (hereinafter referred to as simply "acylating") to obtain a mixture of the Compound E, F and G. The mixture may be separated into each compound by conventional separating methods such as silica gel column chromatography. 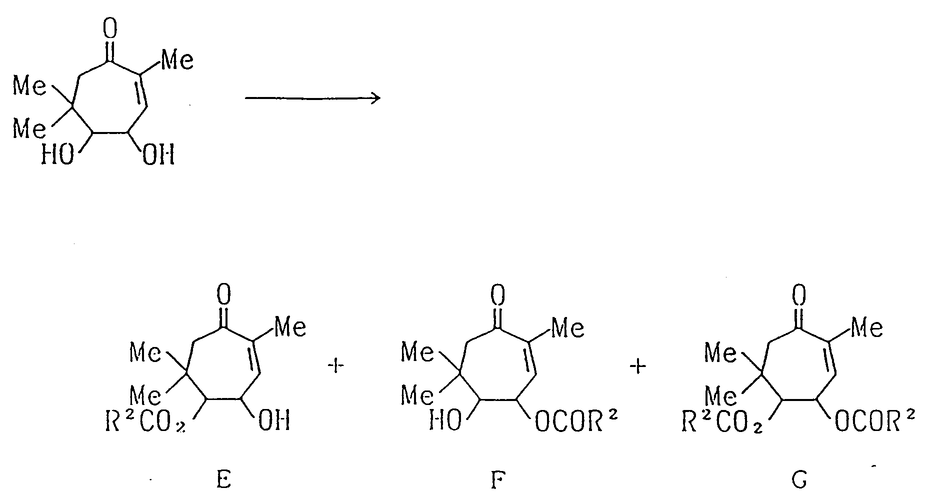 (wherein R² has the same meanings as the above).

The reaction is carried out at -70 to 200°C, preferably at -10 to 10°C with or without stirring until the reaction is completed.

When the reaction is carried out using carboxylic acid and carbodiimide, Compound F may be obtained in which the hydroxy group at the 4-position is selectively acylated.

### Process 5

The process comprises protecting the 1-oxo group of Compound C in which R¹ is alkoxybenzyl group and alkylating, alkenylating or aralkylating the hydroxy group in the 5-position, then deprotecting and removing the alkoxybenzyl group to obtain the Compound H. 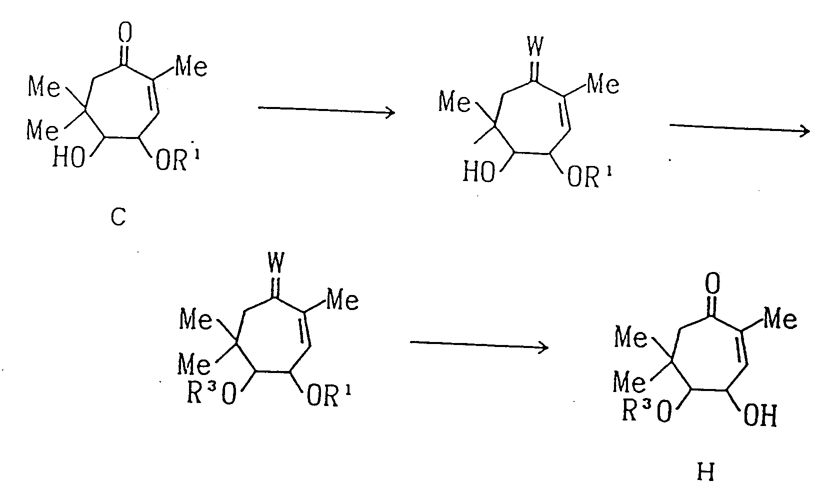
(wherein R¹ represents an alkoxybenzyl group, W represents a protecting group, R³ represents an alkyl, alkenyl or aralkyl group).

Examples of the protecting groups for the 1-oxo group include the usual carbonyl protecting groups, preferably a hydrazone group, more preferably a dimethylhydrazone in a manner according to the method such as described by Newkome (Organic Syntheses Col. Vol., 6, p.12).

The alkylation reaction can be carried out in the presence of a suitable base with the action of an alkyl halide. The alkenylation and aralkylation reaction may be carried out in the same manner as the alkylation reaction.

Examples of the base include metal alkoxides such as sodium methoxide, sodium ethoxide, potassium tert-butoxide; organic amines such as trithylamine, diisopropylethylamine; and sodium hydride, potassium hydride, sodium amide, butyllithium and lithium disopropylamide.

Examples of the alkyl halide include an alkyl halide having 1 to 4 carbon atoms which may have substituents on the chain. Examples of the substituents on the chain include a protected hydroxy group, an alkoxycarbonyl group having 1 to 4 carbon atoms.

Examples of the alkenyl halide include an alkenyl halide such as allyl group or a cinnamyl halide which may have one or more substituents on the ring.

Examples of the aralkyl halide include benzyl halide, phenethyl halide, furfuryl halide, thenyl halide or picolyl halide which may have one or more substituents on the ring.

Examples of the substituents on the cinnamyl or aralkyl group include an alkyl group having 1 to 4 carbon atoms; an alkoxycarbonyl group having 1 to 4 carbon atoms; an acyl group such as acetyl, benzoyl or cinnamoyl group; a hydroxy group which may be protected; an alkoxy group having 1 to 4 carbon atoms; a fluorine, chlorine, bromine or iodine atom; or a nitro group.

The halogen may be a chlorine, bromine or iodine atom.

Examples of the solvent used in the reaction include ethers such as diethylether, dimethoxyethane, tetrahydrofuran, dioxane, non-protonic polar solvents such as dimethylformamide, dimethylsulfoxide or hexamethylphosphoric triamide.

The reaction may be carried out at -70 to 200°C, preferably at 0 to 100°C with or without stirring until the reaction is completed.

The deprotection and the removal of the alkoxybenzyl group may be carriede out simultaneously under the condition of the acid hydrolysis of a hydrazone compound.

### Process 6

The process comprises reducing the 1-oxo group of a Compound I which may be obtained by substituting the 4,5-diol groups of the Saishin N with appropriate substituents, to obtain 1-hydroxy Saishin N derivatives of Formula J (Compound J). 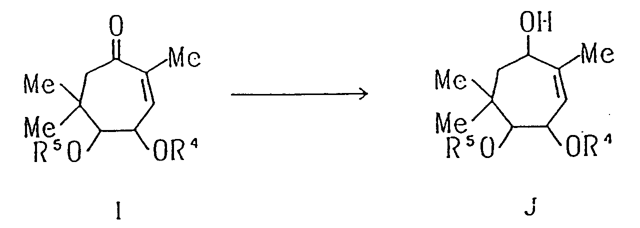
(wherein R⁴ and R⁵ which may be the same or different or combined together, each represent an alkyl, alkenyl, aralkyl or acyl group or a protective group).

Examples of the alkyl group include a lower alkyl group having 1 to 4 carbon atoms which may have substituents on the chain. Examples of the substituents on the chain include a hydroxy group which may be protected, carboxyl group, a lower alkoxycarbonyl group having 1 to 4 carbon atoms.

Examples of the alkenyl group include an allyl group, a 3-methyl-2-butenyl group, a cinnamyl group which may have one or more substituents on the ring.

Examples of the aralkyl group include benzyl, phenethyl, furfuryl, thenyl or picolyl group which may have one or more substituents on the ring.

Examples of the substituents on the ring of the cinnamyl or aralkyl group include a lower alkyl group having 1 to 4 carbon atoms; a lower alkoxycarbonyl group having 1 to 4 carbon atoms; an acyl group such as acetyl, benzoyl, cinnamoyl group; a hydroxy group which may be protected; an alkoxy group having 1 to 4 carbon atoms; a fluorine, chlorine, bromine or iodine atom; or a nitro group.

Examples of the acyl group include an aliphatic acyl group such as acetyl, propionyl, butyryl, isobutyryl, valeryl, acryloyl, methacryloyl or 2-butenoyl which may have substituents on the chain or a cinnamoyl group which may have one or more substituents on the ring; an aromatic acyl group such as benzoyl, naphthoyl group which may have one or more substituents on the ring; or a heterocyclic acyl group such as furoyl, pyrrolylcarbonyl, thenoyl, imidazolylcarbonyl, benzimidazolylcarbonyl group which may have one or more substituents on the ring. Examples of the substituents of the chain include a hydroxy group which may be protected, carboxyl group, an alkoxycarbonyl group having 1 to 4 carbon atoms or thiazolyl group. Examples of the substituents on the ring include an alkyl group having 1 to 4 carbon atoms; carboxyl group; an alkoxycarbonyl group having 1 to 4 carbon atoms; an acyl group such as acetyl, benzoyl, cinnamoyl group which may have one or more substituents on the chain or ring; a hydroxy group which may be protected; an alkoxy group having 1 to 4 carbon atoms; an acyloxy group such as acetoxy, propionyloxy or butyryloxy group; a fluorine, chlorine, bromine or iodine atom; a nitro group; or an amino group which may be substituted by an acyl group such as acetyl, benzoyl or cinnamoyl group which may have one or more substituents on the chain or ring, an alkyl group having 1 to 4 carbon atoms or a benzyl, trityl, benzyloxycarbonyl group.

The protective group is preferably a cyclic acetal and may include isopropylidene, benzylidene, or anisylidene when the Saishin N is reacted directly.

Examples of the protective group of the hydroxy group include a chain acetal such as tetrahydropyranyl, tetrahydrothiopyranyl, 4-methoxytetrahydropyranyl, tetrahydrofuranyl, 1-ethoxyethyl, 1-methyl-1-methoxyethyl or 1-isopropyloxyethyl group, when Compound C, Compound E, Compound F or Compound H is reacted.

The cyclic acetalation reaction may be carried out by using a solvent which is insoluble with water, such as benzene, toluene, carbon tetrachloride, chloroform, dichloromethane, since the reaction is accompanied with the production of water, which was removed azeotropically in the presence of an acid catalyst.

The chain acetalation may be carried out by reacting a corresponding vinylether compound such as dihydropyran in the presence of an acid catalyst. The reaction may be carried out without any solvent or may be carried out by the addition of an inert solvent such as chloroform, dichloromethane, diethylether, tetrahydrofuran, dioxane, dimethylformide, benzene or toluene.

Examples of the acid include an inorganic acid such as sulfuric acid, nitric acid, hydrobromic acid or phosphoric acid; an organic acid such as formic acid, acetic acid, propionic acid or butyric acid; a Lewis acid such as boron trifluoride etherate, titanium tetrachloride, zinc chloride or tin tetrachloride; and a solfonic acid such as methanesulfonic acid, comphorsulfonic acid, benzenesulfonic acid and toluenesulfonic acid.

Examples of the reducing agent include sodium borohydride, lithium borohydride or lithium aluminum-hydride.

The solvent may be selected depending on the reducing agent used, i.e. when sodium borohydride is used, alcohols are preferred and when lithium borohydride or lithium aluminumhyride is used, ethers such as diethylether and tetrahydrofuran are preferred.

### Process 7

The process comprises treating a Saishin N derivative having a hydroxy group in the 4-position with an alcohol in the presence of an acid catalyst or alkylating, alkenylating, aralkylating or acylating the hydroxy group in the 4-position in the presence of a base to obtain the bicyclic Compound M. 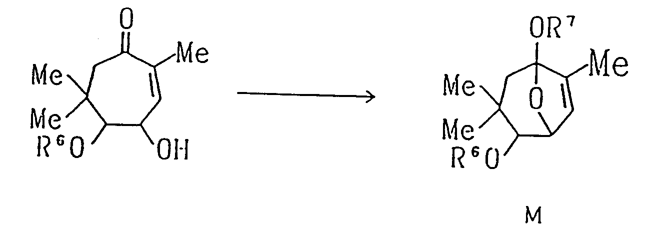
(in which R⁶ and R⁷ may be the same or different and each represents an alkyl, alkenyl, aralkyl or acyl group).

### Process 8

The proces comprises treating a Saishin N derivative having a hydroxy group in the 5-position in the same manner as described for Process 7 to obtain a bicyclic Compound N. 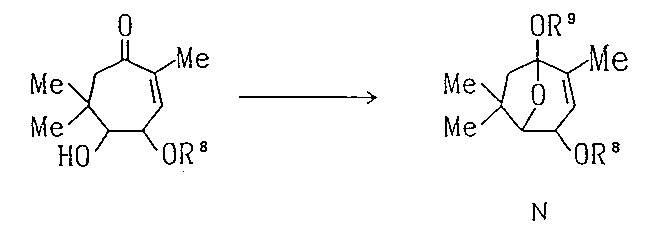
(wherein R⁸ and R⁹ may be the same or different and each represents an alkyl, alkenyl, aralkyl or acyl group).

The definition of the alkyl, alkenyl, aralkyl and acyl groups in the Process 7 and 8 are as follows.

Examples of the alkyl group include an alkyl group having 1 to 4 carbon atoms which may have substituents on the chain. Examples of the substituents on the chain include a hydroxy group which may be protected, carboxyl group or an alkoxycarbonyl group having 1 to 4 carbon atoms.

Examples of the alkenyl group include an allyl group, 3-methyl-2-butenyl group or cinnamyl group which may have one or more substituents on the ring.

Examples of the aralkyl group include benzyl, phenethyl, furfuryl, thenyl or picolyl group which may have one or more substituents on the ring.

Examples of the substituents on the ring of the cinnamyl or aralkyl group include an alkyl group having 1 to 4 carbon atoms; an alkoxycarbonyl group having 1 to 4 carbon atoms; an acyl group such as acetyl, benzoyl or cinnamoyl group; a hydroxy group which may be protected; an alkoxy group having 1 to 4 carbon atoms; a fluorine, chlorine, bromine or iodine atom; or a nitro group.

Examples of the acyl group include an aliphatic acyl group such as acetyl, propionyl, butyryl, isobutyryl, valeryl, acryloyl, methacryloyl and 2-butenoyl group which may have substituents on the chain or cinnamoyl group which may have one or more substituents on the chain or cinnamoyl group which may have one or more substituents on the ring; an aromatic acyl group such as benzoyl or naphthoyl group which may have one or more substituents on the ring; or a heterocyclic acyl group such as furoyl, pyrrolylcarbonyl, thenoyl, imidazolylcarbonyl or benzimidazolylcarbonyl group which may have one or more substituents on the ring. Examples of the substituents on the chain include a hydroxy group which may be protected, a carboxyl group, an alkoxycarbonyl group having 1 to 4 carbon atoms or thiazolyl group. Examples of the substituents on the ring include an alkyl group having 1 to 4 carbon atoms; carboxyl groups, an alkoxycarbonyl group having 1 to 4 carbon atoms; an acyl group such as acetyl, benzoyl or cinnamoyl group which may have one or more substituents on the chain or ring; a hydroxy group which may be protected; an alkoxy group having 1 to 4 carbon atoms; an acyloxy group such as acetoxy, propionyloxy and butyryloxy groups; a fluorine, chlorine, bromine or iodine group; a nitro group; and an amino group which may be substituted by a protective group such as an acyl group such as an acetyl, benzoyl or cinnamoyl group which may have one or more substituents on the chain or ring, an alkyl group having 1 to 4 carbon atoms or benzyl, trityl or benzyloxycarbonyl group.

The alcoholysis may be carried out as described for Process 1, the acylation may be carried out as described for Process 2 and the alkylation, alkenylation or aralkylation may be carried out as described for Process 5.

When the Saishin N is alcoholyzed, a mixture of the compounds corresponding to the compound M and N (R⁶ or R⁸ represents a hydrogen atom) is obtained.

### Process 9

The process comprises reducing catalytically Saishin N or a Saishin N derivative of formula O (Compound O) obtained in the Processes 1 to 7 to obtain a 2,3-dihydro Saishin N derivative (Compound P). 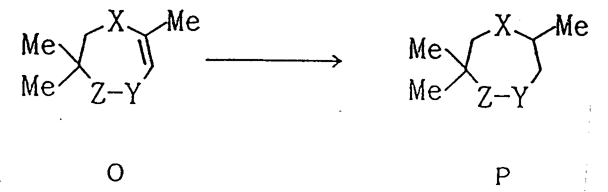
(wherein X, Y and Z are as defined for formula I with the exclusion of the proviso).

The Compound Q having a hydroxy group at the 5-position exists as an equilibrium mixture with the bicyclic compound S represented by the following formula: 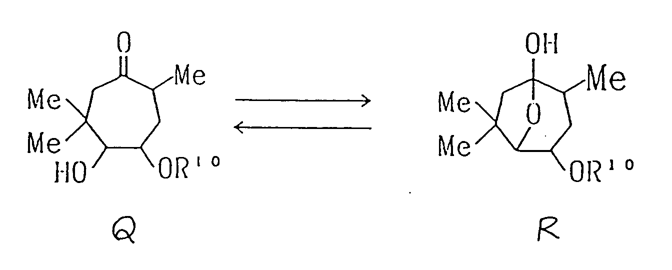
(wherein R¹⁰ represents a hydrogen atom, an alkyl, alkenyl, aralkyl or acyl group).

The definitions of the alkyl, alkenyl, aralkyl and acyl groups are the same as given above.

The solvent used in the reaction is not limited but may include an inert solvent such as an aromatic hydrocarbons such as benzene, toluene or xylene, alkyl halides such as dichloromethane, chloroform, or carbon tetrachloride, ethers such as diethylether, tetrahydrofuran or dioxane, alcohols such as methanol or ethanol, or esters such as ethyl acetate.

Any usual metal catalyst may be used as a catalyst used in the catalytic hydrogenation. Platinum, palladium, Raney nickel or rhodium may be preferably used.

The reaction may be carried out with stirring at room temperature until the given amount of the hydrogen is taken up.

### Process 10

The process comprises treating a Compound Q with an alcohol in the presence of an acid catalyst according to the procedures described for Process 8 or by alkylating, alkenylating, aralkylating or acylating a Compound Q in the presence of a base to obtain a bicyclic compound S. 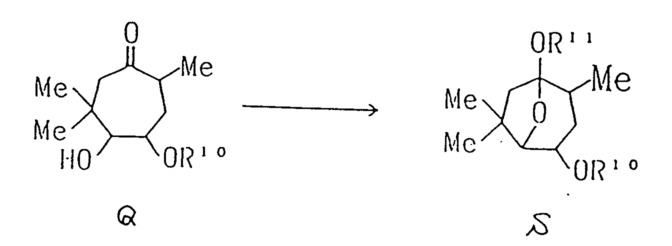
(wherein R¹¹ represents an alkyl, alkenyl, aralkyl or acyl group).

The definitions of the alkyl, alkenyl, aralkyl and acyl groups are the same as given for the above Process 8.

When the alkylation, alkenylation, aralkylation, acylation of the hydroxy group, the oxidation of the hydroxy group to an oxo group, the hydrolysis of the ester group, the removal of the alkoxybenzyl group, the reduction of the oxo group to a hydroxy group, the hydrolysis of the substituents on the chain or ring, the conversion of the substituents by a reduction and oxidizing reaction are carried out on a Saishin N derivative obtained in the processes according to any one of 1 to 10, a Saishin N derivative may be obtained having desirable substituents in the 1-, 4- or 5-positions.

The oxidation of a hydroxy group to an oxo group may be carried out using conventional methods for oxidising hydroxy groups.

Examples of appropriate oxidizing agents include an activated manganese dioxide, chromic anhydride, pyridinium chlorochromate, dimethylsulfoxidetrifluoroacetic anhydride and dimethylsulfoxide-oxalyl chloride.

The configuration of the hydroxy group in the 1-, 4- or 5-position of the compound A may be inverted by conventional inversion methods such as the Mitsunobu reaction.

Where the substituents in the compounds of formula I contain groups which can form acid or base addition salts, such salts can be formed by conventional procedures by reaction with organic or inorganic acids or bases, preferably physiologically tolerable acids or bases.

The inhibitory effects of Saishin N derivatives according to the inventin on experimentally-induced ulcers will be detailed below.

Experiments on Hydrochloric acid-Ethanol-Induced Ulcers, Aspirin-Induced Ulcers, Water-Immersion stress-Induced Ulcers, Shay's Ulcers and Serotonin-Induced Ulcers were carried out using male Wister rats weighing about 200g in groups of six. Experiments on Ischemia-Reperfusion -Induced Gastric Lesions were carried out using male SD rats weighing about 250g in groups of six. Other than for the tests on Ischemia-Reperfusion-Induced Gastric Lesions Models, the test compounds were prepared by mixing thoroughly with polyethyleneglycol and 0.5% sodium carboxymethyl cellulose solution and emulsifying vigorously. Activities against respective ulcer models were evaluated by an inhibiting ratio obtained by dividing the difference between the ulceration indexes of the non-administered group and the test compound group by the non-administered group.

### 〈Hydrochloric acid-Ethanol-Induced Ulcers〉

To each rat which had been fasted for 24 hours was orally administered 0.5ml of a mixed solution of 150 mM hydrochloric acid - 60% ethanol per 100g of weight. Each rat was slaughtered after one hour, a length of ulcer formed at the fundus gland area of stomach was measured and ulceration index was calculated based on the length. The test compound was administered intraduodenally one hour or three hours before the administration of mixed solution of hydrochloric acid - 60% ethanol.

The results of before three hours are shown in Table 1, and those of before an hour are shown in Table 2.

**[Table 2]**

| Example No. | Compound | Inhibition ratio(%) |
|---|---|---|
| 2 | AU156 | 97(10)** |
| 3 | AU155 | 96(10)** ;93(5)** |
| 4 | AU154 | 67(10)** |
| 29 | AU102 | 75(10)** |
| 96 | AU152 | 87(20)** |
| 96 | AU153 | 55(10)* |
| 97 | AU159 | 94 (5)** ;83(10)** |
| 98 | AU157 | 64 (5)** |
| 108 | AU158 | 33 (5) |
| 116 | AU105B | 86(10)** |
| 118 | AU108A | 75(10)** |
| ref. | Saishin N | 88(20)** ;35(10)* |

| | | |
|---|---|---|
| The numerals in the parenthesis show the dose(mg/kg). *;p<0.05, | | |
| **;p<0.01 | | |

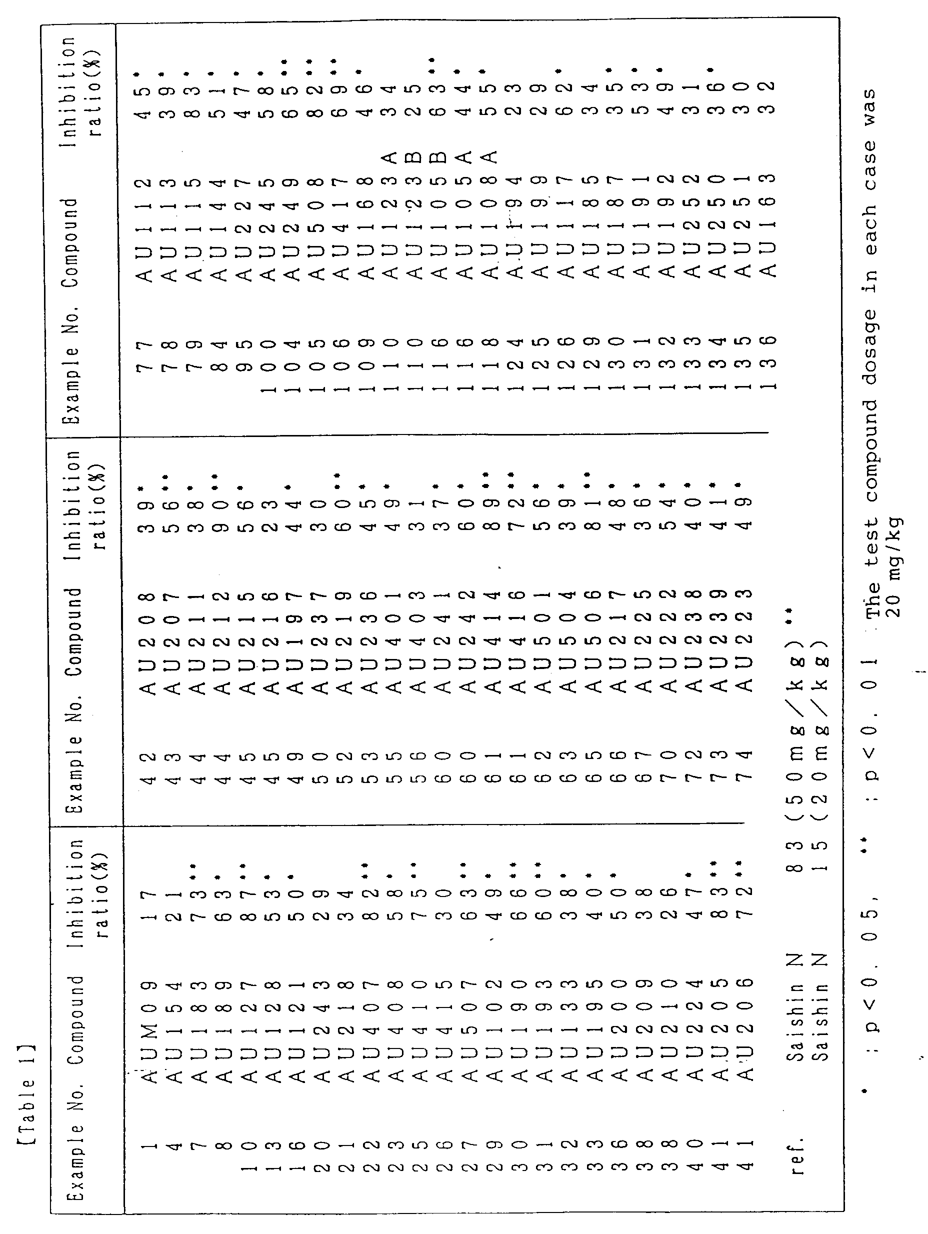

As clearly shown in Table 1 and Table 2, the Saishin N derivatives demonstrate excellent anti-ulcer activity.

### 〈Aspirin-Induced Ulcers〉

In each rat which had been fasted for 24 hours, the pyloric end of the stomach was ligated, and simultaneously the test compound was administered intraduodenally, and after 5 minutes, 150 mg/kg of aspirin was administered orally, respectively. After 9 hours from ligation, each rat was slaughtered, a length of ulcer formed at the fundus gland area of stomach was measured and ulceration index was calculated based on the length.

### 〈Water-Immersion stress-Induced Ulcers〉

Each rat which had been fasted for 15 hours was immobilized in a stress cage and immersed to chest depth in a water bath at 21°C. Each rat was slaughtered after 10 hours, a length of ulcer formed at the fundus gland area of stomach was measured and ulceration index was calculated based on the length. The test compound was administered orally 10 minutes before the exposure to stress.

### 〈Shay's Ulcers〉

In each rat which had been fasted for 48 hours, the pyloric end of stomach was ligated, and each was kept without giving any food or water for 14 hours. Each rat was then slaughtered, and area of ulcer formed in the forestomach was measured and ulceration inded was calculated based on the area. The test compound was administered intraduodenally immediately after the ligation.

### 〈Serotonin-Induced Ulcers〉

To each rat which had been fasted for 24 hours, 30 mg/kg of a solution of serotonin creatinine sulfate in a saline solution was administered subcutaneously. Each rat was then slaughtered after 4 and a half hours and area of gastric mucosal lesion formed facing the center line of greater curvature of the corpus ventriculi of stomach was measured and ulceration index was calculated based on the area. The test compound was administered intraduodenally 30 minutes before the administration of the Serotonin creatinine sulfate.

### 〈Ischemia-Reperfusion-Induced Gastric Lesions〉

Each rat which had been fasted for 24 hours was anesthetized intraperitoneally with Nembutal and immobilized on a constant temperature pad in a dorsal position. After tracheotomy was performed, blood pressure was measured via a transducer for blood pressure from a cannula inserted in right carotid. The abdomen was opened, and the gastroesophageal junction was ligated. A tube was then passed through the duodenostomy into the stomach and the gastric lumen was lavaged with warm saline. 1 ml of 0.1N hydrochloric acid per 100g body weight was instilled into the stomach via the gastric tube and the pylorus was ligated. When the blood pressure became stable, 2% of weight (w/w) of the blood was collected from a cannula inserted in the left carotid into a syringe containing a heparin added saline solution. After 20 minutes, the collecting blood was retransfused and each rat was left to stand for 20 minutes, and slaughtered and the area of gastric mucosal lesions was measured and ulceration index was calculated based on the area. The test compound was dissolved in dimethylsulfoxide and administered at a dose of 40 mg/kg from the caudal artery 30 minutes before beginning collection of the blood.

The results of Aspirin-Induced Ulcers, Water-immersion stress-Induced Ulcers, Shay's Ulcers, Serotonin-induced Ulcers and Ischemia-Reperfusion-Induced Gastric lesions are shown in Table 3.

Considering the rsults from each above test, it can be seen that the Saishin N derivatives have an excellent anti-ulcer activity and that a medicament containing such Saishin N derivatives as an active component thus provides an excellent anti-ulcer agent.

The dose of the Saishin N derivatives to a patient may vary depending on age, conditions, etc., but is generally a total of 1 to 1000 mg, preferably 10 to 600 mg per day for an adult in oral administration, administered 1 to 6 times, more preferably 1 to 3 times a day.

In the present invention, by admixing conventional pharmaceutical carriers with Saishin N derivatives, pharmaceutical compositions according to the invention may be formed into solid preparations such as tablets, hard or soft capsules, granules, powder, fine particles or of suppositories; or into liquid preparations such as injection syrups, elixirs, suspensions or emulsions, etc. Solid preparations may be prepared in the form of enteric preparations or gradually releasing preparations. The pharmaceutical carriers for these preparations may be chosen suitably, depending on the desired type of preparations, for excipients, binders, disintegrants, lubricants, coating agents, dissolving adjuvants, emulsifiers, suspending agents, surfactants, absorption adjuvants, stabilizers or solvents, etc.

Viewed from further aspects the invention provides the use of a compound of Formula I as defined above, for the preparation of a composition for use in combatting ulcers and pharmaceutical compositions containing a compound of formula I together with at least one pharmaceutical carrier or excipient.

**[Table 3]**

| Example No. | Compound | Water-Immersion Stress | Shay | Aspirin | Serotonin | Ischemia-Reperfusion |
|---|---|---|---|---|---|---|
| 4 | AU154 | 17(50) | 22(50) | ― | 46(20)* | ― |
| 22 | AU407 | 50(50)* | ― | 71(50)** | 41(20)* | ― |
| 23 | AU408 | 56(50)* | 43(50)* | 73(50)** | 44(20)* | 75(40)** |
| 30 | AU190 | 45(50)* | 55(50)* | 55(50)* | 69(20)** | ― |
| 41 | AU205 | 42(20)* | 50(50)* | 53(50)* | 80(20)* | |
| 44 | AU212 | 48(20)* | 75(50)** | 46(20)* | 71(20)** | 63(40)** |
| 45 | AU215 | 45(20)* | 73(50)** | 66(50)** | 81(20)** | 90(40)** |
| 55 | AU401 | ― | ― | ― | 68(20)** | ― |
| 61 | AU414 | 41(20)* | ― | ― | ― | ― |
| 62 | AU501 | 22(50) | ― | 30(50) | 55(50)* | 77(40)** |
| 70 | AU222 | 34(20)* | 58(50)* | 28(50) | 84(20)** | ― |
| 100 | AU245 | 38(20)* | 35(50) | 63(50)** | 38(20) | 93(40)** |
| 104 | AU249 | 33(20) | ― | ― | ― | ― |
| 116 | AU105B | 65(20)** | 44(50)* | ― | 80(20)* | 52(40)* |
| 126 | AU117 | ― | ― | ― | 60(20)** | ― |
| ref. | Saishin N | 49(50)* | 49(100)* | 52(50)* | 13(20) | |
| | | 62(100)** | 58(300)* | 60(100)** | 36(50) | |
| | Cetraxatehydrochloride | 45(300)* | 11(300) | 33(300) | | |

| | | | | | | |
|---|---|---|---|---|---|---|
| The numerals in the parenthesis show the dose(mg/kg). *;p<0.05, | | | | | | |
| **p<0.01 | | | | | | |

The present invention will hereinafter be explained in more detail with reference to the following non-limitative working Examples.

The stereochemistry of the oxygen functional group in the 1-, 4- or 5-position and the methyl group in the 2-position in a reductant of a double in the 2- or 3-position represents 1-cis, 2-cis, 5-trans based on the 4-position unless otherwise mentioned. When specifying a stereochemistry, the prefix was written conveniently such as 2,4-trans. The word in parentheses after a chemical name represents a temporary name in the specification.

### Example 1

To a solution of eucarvone-4,5-oxide (50g, 0.3M) in tetrahydrofuran (150ml), 4-methoxybenzyl alcohol (55g, 0.4M) and p-toluenesufonic acid (2g) were added, and heated under reflux for eight hours, concentrated, and then purified by silica gel column chromatography using hexane-ethyl acetate (10:1) as an eluent to obtain 46.7g (50%) of 5-hydroxy-4-(4-methoxybenzyloxy)-2,6,6-trimethyl-2-cyclohepten-1-one (AUM09) as a light yellow oily product.
IR (KBr, cm⁻¹): 3528, 1672, 1612, 1514, 1249, 1099, 1034, 823.
1H-NMR (CDCl₃, ppm): 0.97 (3H, s), 1. 12 (3H, s), 1. 84 (3H, t, J=2Hz), 2.33(1H, d, J=13Hz),2.44 (1H, d, J=13Hz), 3.12 (1H, brs), 3.30 (1H, d, J=9Hz), 3.82 (3H, s), 4.04 (1H, dm, J=9Hz), 4.58 (1H, d, J=11Hz), 4.76 (1H, d, J=11Hz), 6.50 (1H, m), 6.91 (2H, d, J=8Hz), 7.31 (2H, d, J=8Hz).

### Example 2

To a solution of eucarvone-4,5-oxide (3.32g, 20mM) in tetrahydrofuran (10ml), benzyl alcohol (4.12ml, 40mM) and concentrated sulfuric acid (0.2ml) were added and stirred at 0°C for an hour. The mixture was then treated as disclosed in Example 1 to obtain 1.09g (20%) of 4-benzyloxy-5-hydroxy-2,6,6-trimethyl-2-cyclohepten-1-one (AU156) as a colorless oily product.
IR (KBr, cm⁻¹): 3534, 1672, 1100, 1072, 738, 698.
1H-NMR (CDCl₃, ppm): 0.97 (3H, s) 1.12 (3H, s), 1.85 (3H, t, J=2Hz), 2.34 (1H, d, J=12Hz), 2.45 (1H, d, J=12Hz), 3.09 (1H, brs), 3.34 (1H, d, J=9Hz), 4.06 (1H, dm, J=9Hz), 4.65 (1H, d, J=12Hz), 4.83 (2H, d, J=12Hz), 6.51 (1H, m), 7.36 (5H, m).

### Example 3

To a solution of eucarvone-4,5-oxide (1.66g, 10mM) in methanol (10ml), concentrated sulfuric acid (0.2ml) was added under ice-cooling and stirring for 30 minutes, followed by stirring at room temperature for two hours. The reaction liquid was diluted by ethyl acetate, washed with an aqueous potassium carbonate solution and with brine, dried over magnesium sulfate, filtered and then concentrated. The residue was purified by silica gel column chromatography using hexaneethyl acetate (20:1) as an eluent to obtain 0.99g (46.6%) of 1,4-dimethoxy-2,6,6-trimethyl-8-oxabicyclo [3. 2. 1] oct-2-ene (AU155) as a colorless oily product.
1H-NMR (CDCl₃, ppm): 1.22 (3H, s), 1.25 (3H, s), 1.67 (3H, t, J=2Hz), 1.80 (2H, s), 3.36 (6H, s), 3.05 (1H, dd, J=5, 2Hz), 4.35 (1H, m), 5.48 (1H, m).

### Example 4

To a solution of eucarvone-4,5-oxide (4.98g, 30mM) in methanol (30ml), p-toluenesufonic acid (0.1g) was added under ice-cooling and stirring for three hours. The mixture was then concentrated. To the mixture was added acetone (20ml) and 1N HCl (3ml), and stirred for a day, diluted by ethyl acetate, washed with a saturated sodium bicarbonate solution and with brine, dried over magnesium sulfate, filtered and concentrated. The residue was purified by silica gel column chromatography using hexane-ethyl acetate (10:1) as an eluent to obtain 4.94g (83%) of 5-hydroxy-4-methoxy-2,6,6-trimethyl-2-cyclohepten-1-one (AU154) as a yellow oily product.
IR (KBr, cm⁻¹): 3446, 1670, 1458, 1104.
1H-NMR (CDCl₃, ppm): 1.02 (3H, s), 1.13 (3H, s) 1.85 (3H, t, J=2Hz), 2.35 (1H, d J=12Hz), 2.47 (1H, d, J=12Hz), 3.11 (1H, brs), 3.28 (1H, d, J=9Hz), 3.55 (3H, s), 3.79 (1H, dm, J=9Hz), 6.43 (1H, m).

### Example 5

To a solution of 5-hydroxy-4-(4-methoxybenzyloxy)-2,6,6-trimethyl-2-cyclohepten-1-one (11.47g, 37mM) obtained in Example 1 in pyridine (30ml), acetic anhydride (10ml) was added and stirred overnight at room temperature. The reaction solution was then poured onto ice-water and extracted with ethyl acetate, washed with a saturated sodium bicarbonate and with brine, dried over magnesium sulfate, filtered and concentrated. The residue was purified by silica gel column chromatography using hexane-ethyl acetate (30:1) as an eluent to obtain 10.06g (78%) of 5-acetoxy-4-(4-methoxybenzyloxy)-2,6,6-trimethyl-2-cyclohepten-1-one (AU181) as a colorless oily product.
IR(KBr, cm⁻¹): 1743, 1673, 1514, 1247, 1098, 1035, 824.
1H-NMR (CDCl₃, ppm): 1.02 (3H, s), 1.03 (3H, s), 1.85 (3H, t, J=2Hz), 2.07 (3H, s), 2.45 (1H, d, J=13Hz), 2.53 (1H, d, J=13Hz), 3.82 (3H, s), 4.19 (1H, dm, J=9Hz), 4.58 (1H, d, J=12Hz), 4.65 (1H, d, J=12Hz), 4.90 (1H, d, J=9Hz), 6.50 (1H, m), 6.90 (2H, d, J=9Hz), 7.25 (2H, d. J=9Hz).

### Example 6

To a solution of 5-hydroxy-4-(4-methoxybenzyloxy)-2,6,6-trimethyl-2-cyclohepten-1-one (2.44g, 8mM) obtained in Example 1 in pyridine (10ml), benzoic acid anhydride (3.04g, 10mM) and 4-dimethyl aminopyridine (0.1g) were added and stirred overnight at room temperature. The mixture was then after treated according to the procedures described in Example 5 and purified by silica gel column chromatography using hexane-ethyl acetate (10:1) as an eluent to obtain 3.10g (95%) of 5-benzoyloxy-4-(4-methoxybenzyloxy)-2,6,6-trimethyl-2-cyclohepten-1-one (AU182) as a colorless oily product.
IR (KBr, cm⁻¹): 1723, 1673, 1514, 1273, 1114, 712.
1H-NMR(CDCl₃, ppm) 1.09 (3H, s), 1.13 (3H, s), 1.88 (3H, t, J=2Hz), 2.53 (1H, d, J=13Hz), 2.60 (1H, d, J=13Hz), 3.75 (3H, s), 4.31 (1H, dm, J=9Hz), 4.49 (1H, d, J=12Hz), 4.58 (1H, d, J=12Hz), 5.15 (1H, d, J=9Hz), 6.54 (1H, m), 6.72 (2H, d, J=9Hz), 7.05 (2H, d, J=9Hz), 7.46 (2H, t, J=7Hz), 7.59 (1H, m), 8.05 (2H, d, J=7Hz).

### Example 7

To a solution of the product of Example 6 (3.69g, 9.0mM) in methylene chloride (40ml), water (0.8ml) and DDQ (2.72g, 12mM) were added and stirred for two hours at room temperature, filtered and concentrated. The residue was purified by silica gel column chromatography using hexane-ethyl acetate (10:1) as an eluent and crystallized from a mixed solvent of ethyl acetate and hexane to obtain 1.82g (70%) of 5-benzoyloxy-4-hydroxy-2,6,6-trimethyl-2-cyclohepten-1-one (AU183) as colorless needles . melting point: 99 to 100 °C.
IR (KBr, cm⁻¹): 3446, 1720, 1677, 1279, 1118, 716.
1H-NMR (CDCl₃, ppm): 1.10 (3H, s), 1.23 (3H, s), 1.90 (3H, t, J=2Hz), 2.49 (1H, d, J=13Hz), 2.66 (1H, d, J=13Hz), 4.66 (1H, m), 4.95 (1H, d, J=9Hz), 6.55 (1H, m), 7.47 (2H, m), 7.62 (1H, m), 8.08 (2H, m).

### Example 8

The product obtained In Example 7 (1.1g, 3.5mM) was acetylated by a conventional method and crystallized from a mixed solvent of ethyl acetate and hexane to obtain 0.83g (72%) of 4-acetoxy-5-benzoyloxy-2,6,6-trimethyl-2-cyclohepten-1-one (AU189) as colorless needles. melting point: 86 to 87 °C.
IR (KBr, cm⁻¹): 1750, 1723, 1668, 1269, 1224, 1113, 1027, 715.
1H-NMR (CDCl₃, ppm): 1.09 (3H, s), 1.24 (3H, s), 1.77 (3H, s), 1.90 (3H, t, J=2Hz), 2.54 (1H, d, J=13Hz), 2.74 (1H, d, J=13Hz), 5.10 (1H, d, J=9Hz), 5.97 (1H, dm, J=9Hz), 6.36 (1H, m), 7.47 (2H, m), 7.58 (1H, m), 8.03 (2H, m).

### Example 9

5-hydroxy-4-(4-methoxybenzyloxy)-2,6,6-trimethyl-2-cyclohepten-1-one (4.7g, 15.4mM) obtained in Example 1 was treated with 2-chlorobenzoic anhydride (16g, 54.2mM) according to the procedures described in Example 6 to obtain 4.33g (63%) of 5-(2-chlorobenzyloxy)-4-(4-methoxybenzyloxy)-2,6,6-trimethyl-2-cyclohepten-1-one (AU124) as a colorless oily product.
IR (KBr, cm⁻¹): 1736, 1673, 1514, 1249, 1049, 749.
1H-NMR (CDCl₃, ppm): 1.13 (3H, s), 1.15 (3H, s), 1.88 (3H, t, J=2Hz), 2.52 (1H, d, J=13Hz), 2.60 (1H, d, J=13Hz), 3.78 (3H, s), 4.31 (1H, dm, J=8Hz), 4.51 (1H, d, J=12Hz), 4.61 (1H, d, J=12Hz), 5.16 (1H, d, J=9Hz), 6.54 (1H, m), 6.76 (2H, d, J=9Hz), 7.12 (2H, d, J=9Hz), 7.29 (1H, m), 7.43 (2H, m), 7.75 (1H, m).

### Example 10

The product of Example 9 (1.5g, 3.4mM) was treated with DDQ as described in Example 7 and was crystallized from a mixed solvent of ethyl acetate and hexane to obtain 0.78g (81%) of 5-(2-chlorobenzoyloxy)-4-hydroxy-2,6,6-trimethyl-2-cyclohepten-1-one (AU 127) as colorless prisms. melting point: 92.5 to 94°C.
IR (KBr, cm⁻¹): 3507, 1734, 1656, 1252, 1115, 1050, 748.
1H-NMR (CDCl₃, ppm): 1.13 (3H, s), 1.15 (3H, s), 1.89 (3H, t, J=2Hz), 2.48 (1H, d, J=13Hz), 2.64 (1H, d, J=13Hz); 4.65 (1H, dm, J=9Hz), 5.00 (1H, d, J=9Hz), 6.54 (1H, m), 7.36 (1H, m), 7.47 (2H, m), 7.87 (1H, m).

### Example 11

5-hydroxy-4(4-methoxybenzyloxy)-2,6,6-trimethyl-2-cyclohepten-1-one (4.3g, 14mM) obtained in Example 1 was treated with 3-chlorobenzoic anhydride (14.4g, 48.8mM) according to the procedures described in Example 6 to obtain 3.56g (57%) of 5-(3-chlorobenzoyloxy)-4-(4-methoxybenzyloxy)-2,6,6-trimethyl-2-cyclohepten-1-one (AU125) as a colorless oily product.
IR (KBr, cm⁻¹): 3507, 1725, 1674, 1271, 1117, 1104, 757.
1H-NMR (CDCl₃, ppm): 1.08 (3H, s), 1.11 (3H, s), 1.89 (3H, t, J=2Hz), 2.51 (1H, d, J=13Hz), 2.59 (1H, d, J=13Hz), 3.77 (3H, s), 4.30 (1H, dm, J=9Hz), 4.45 (1H, d, J=12Hz),4.60 (1H, d, J=12Hz), 5.11 (1H, d, J=9Hz), 6.54 (1H, m), 6.74 (2H, d, J=8Hz), 7.05 (2H, d, J=8Hz), 7.41 (1H, t, J=8Hz), 7.56 (1H, m), 7.92 (1H, m), 7.97 (1H, m).

### Example 12

5-hydroxy-4-(4-methoxybenzyloxy)-2,6,6-trimethyl-2-cyclohepten-1-one (5.0g, 16.4mM) obtained in Example 1 was treated with 4-chlorobenzoic anhydride (17g, 57.6mM) according to the procedures described in Example 6 to obtain 3.45g (48%) of 5-(4-chlorobenzoyloxy)-4-(4-methoxybenzyloxy)-2,6,6-trimethyl-2-cyclohepten-1-one (AU126) as a colorless oily product.
IR (KBr, cm⁻¹): 1725, 1674, 1271, 1117, 757.
1H-NMR (CDCl₃, ppm): 1.08 (3H, s), 1.11 (3H, s), 1.88 (3H, t, J=2Hz), 2.52 (1H, d, J=13Hz), 2.59 (1H, d, J=13Hz), 3.77 (3H, s), 4.30 (1H, dm, J=9Hz), 4.46 (1H, d, J=12Hz), 4.58 (1H, d, J=12Hz), 5.11 (1H, d, J=9Hz), 6.53 (1H, m), 6.73 (2H, d, J=8Hz), 7.04 (2H, d, J=8Hz), 7.43 (2H, d, J=8Hz), 7.95 (2H, d. J=8Hz).

### Example 13

The product of Example 12 (3.00g, 6.78mM) was treated with DDQ according to the procedures described in Example 7 and crystallized from a mixed solvent of ethyl acetate and hexane to obtain 1.09g (48%) of 5-(4-chlorobenzoyloxy)-4-hydroxy-2,6,6-trimethyl-2-cyclohepten-1-one (AU128) as colorless plates. melting point: 114.5 to 115.5 °C.
IR (KBr, cm⁻¹): 3480, 1714, 1676, 1274, 1123, 1094, 848, 758.
1H-NMR (CDCl₃, ppm): 1.09 (3H, s), 1.21 (3H, s), 1.90 (3H, t, J=2Hz), 2.23 (1H, d, J=8Hz; disappeared by the addition of heavy water), 2.48 (1H, d, J=13Hz), 2.65 (1H, d, J=13Hz), 4.63 (1H, m; dm by the addition of heavy water, J=9Hz), 4.92 (1H, d, J=9Hz), 6.54 (1H, m), 7.46 (2H, d, J=9Hz), 8.01 (2H, d, J=9Hz).

### Example 14

The Product of Example 13 (0.79g, 2.5mM) was acetylated by a conventional method and crystallized from a mixed solvent of ethyl acetate and hexane to obtain 0.54g (61%) of 4-acetoxy-5-(4-chlorobenzoyloxy)-2,6,6-trimethyl-2-cyclohepten-1-one (AU130) as colorless prisms. melting point: 86 to 87 °C.
IR (KBr, cm⁻¹): 1747, 1725, 1675, 1592, 1265, 760.
1H-NMR (CDCl₃, ppm): 1.08 (3H, s), 1.22 (3H, s), 1.79 (3H, s), 1.90 (3H, t, J=2Hz), 2.53 (1H, d, J=13Hz), 2.73 (1H, d, J=13Hz), 5.09 (1H, d, J=9Hz), 5.95 (1H, dm, J=9Hz), 6.34 (1H, m), 7.44 (2H, d, J=9Hz), 7.95 (2H d, J=9Hz).

### Example 15

5-hydroxy-4-methoxy-2,6,6-trimethy-2-cyclohepten-1-one (5.18g, 26mM) obtained in Example 4 was acetylated by a conventional method and purified by silica gel column chromatography using benzene-ethyl acetate (20:1) as an eluent to obtain 4.16g (65%) of 5-acetoxy-4-methoxy-2,6,6-trimethyl-2-cyclohepten-1-one (AU161) as a colorless oily product.
IR (KBr, cm⁻¹): 1744, 1674, 1238.
1H-NMR (CDCl₃, ppm): 1.02 (3H, s), 1.05 (3H, s) 1.86 (3H, t, J=2Hz), 2.12 (3H, s), 2.46 (1H, d, J=13Hz), 2.56 (1H, d, J=13Hz), 3.45 (3H, s), 3.96 (1H, dm, J=9Hz), 4.85 (1H, d, J=9Hz), 6.45 (1H, m).
13C-NMR (CDCl₃, ppm): 19.0 (q), 20.9 (q), 23.6 (q), 27.3 (q), 53.9 (t), 58.6 (q), 78.6 (d), 79.3 (d), 138.4 (s), 141.8 (d), 170.2 (s), 200.3 (s).

### Example 16

5-hydroxy-4-methoxy-2,6,6-trimethyl-2-cyclohepten-1-one (1.0g, 5.0mM) obtained in Example 4 was benzoylated according to the procedures described in Example 6 and crystallized from hexane to obtain 0.95g (63%) of 5-benzoyloxy-4-methoxy-2,6,6-trimethyl-2-cyclohepten-1-one (AU121) as colorless needles. melting point: 91.5 to 93.5 °C.
IR (KBr, cm⁻¹): 1716, 1670, 1276, 1114, 714.
1H-NMR (CDCl₃, ppm): 1.10 (3H, s), 1.19 (3H, s), 1.89 (3H, t, J=2Hz), 2.56 (1H, d, J=13Hz), 2.64 (1H, d, J=13Hz), 3.41 (3H, s), 4.11 (1H, dm, J=9Hz), 5.12 (1H, d, J=9Hz), 6.50 (1H, m), 7.47 (2H, m), 7.58 (1H, m), 8.07 (2H, m).

### Example 17

To a solution of 5-hydroxy-4-methoxy-2,6,6-trimethyl-2-cyclohepten-1-one (1.0g, 5.04mM) obtained in Example 4 in methylene chloride (5ml), 4-methoxybenzoyl chloride (1.3g, 7.62mM) was added at 0 °C, stirred for 30 minutes, and stirred overnight at room temperature. The mixture was then treated according to the procedures described in Example 6 and purified by silica gel column chromatography using hexane-ethyl acetate (20:1) as an eluent and crystallized from a mixed solvent of ethyl acetate and hexane to obtain 0.53g (32%) of 4-methoxy-5-(4-methoxybenzoyloxy)-2,6,6-trimethyl-2-cyclohepten-1-one (AU129) as colorless needles. melting point: 100 to 101 °C.
IR (KBr, cm⁻¹): 1711, 1669, 1605, 1281, 1258, 1112, 852, 770.
1H-NMR (CDCl₃, ppm): 1.09 (3H, s), 1.17 (3H, s), 1.89 (3H, t, J=2Hz), 2.56 (1H, d, J=13Hz), 2.62 (1H, d, J=13Hz), 3.40 (3H, s), 3.87 (3H, s), 4.08 (1H, dm, J=9Hz), 5.09 (1H, d, J=9Hz), 6.49 (1H, m), 6.94 (2H d, J=9Hz), 8.02 (2H, d, J=9Hz).

### Example 18

5-hydroxy-4-methoxy-2,6,6-trimethyl-2-cyclohepten-1-one (9.9g, 50mM) obtained in Example 4 was treated with 4-nitrobenzoyl chloride according to the procedures described in Example 17 and fractionated by silica gel column chromatography using hexane-ethyl acetate (20:1 to 10:1) as an eluent. The first eluted portion was crystallized from ethyl-acetate-hexane to obtain 5.75g (33%) of 4-methoxy-1-(4-nitrobenzoyloxy)-2,6,6-trimethyl-8-oxabicyclo [3. 2. 1]oct-2-ene (AU231) as light yellow needles. melting point: 95.0 to 95.5 °C.
IR (KBr, cm⁻¹):1749, 1527, 1261, 1141, 715.
1H-NMR (CDCl₃, ppm): 1.30 (3H, s), 1.34 (3H, s), 1.67 (3H, t, J=2Hz), 2.19 (2H, s), 3.40 (3H, s), 4.24 (1H, dd, J=5,2Hz), 4.54 (1H, m), 5.52 (1H, m), 8.22 (2H, d, J=9Hz), 8.30 (2H, d, J=9Hz).

Further, the successive eluted portion was crystallized from ethyl acetate-hexane to obtain 3.90g (22%) of 4-methoxy-5-(4-nitrobenzoyloxy)-2,6,6-trimethyl-2-cyclohepten-1-one (AU230) as light yellow needles. melting point: 122 to 123 °C.
IR (KBr, cm⁻¹): 1728, 1670, 1522, 1269, 1114, 714.
1H-NMR (CDCl₃, ppm): 1.11 (3H, s), 1.20 (3H, s), 1.90 (3H, t, J=2Hz), 2.57 (1H, d, J=13Hz), 2.64 (1H, d, J=13Hz), 3.40 (3H, s), 4.12 (1H, dm, J=9Hz), 5.12 (1H, d, J=9Hz), 6.50 (1H, m), 8.23 (2H, d, J=9Hz), 8.33 (2H, d, J=9Hz).

### Example 19

The mixture of 4-methoxy-5-(4-nitrobenzoyloxy)-2,6,6-trimethyl-2-cyclohepten-1-one (2.78g, 8mM) obtained in Example 18, ammonium chloride (0.17g), Fe (1.2g), dimethylformamide (9ml) and water (4ml) was stirred for 20 minutes at 80°C, filtered and washed with ethyl acetate. The filtrate and the wash liquid were combined and washed with water, dried over magnesium sulfate, filtered and crystallized from a mixed solution of ethyl acetate and hexane to obtain 2.29g (90%) of 5-(4-aminoben zoyloxy)-4-methoxy-2,6,6-trimethyl-2-cyclohepten-1-one (AU235) as colorless needles. melting point: 138.5 to 161°C.
IR (KBr, cm⁻¹): 3432, 3349, 3238, 1684, 1664, 1602, 1275, 1168, 1115.
1H-NMR (CDCl₃, ppm): 1.08 (3H, s), 1.16 (3H, s), 1.88 (3H, t, J=2Hz), 2.56 (1H, d, J=13Hz), 2.61 (1H, d, J=13Hz), 3.41 (3H, s), 4.11 (3H, m), 5.08 (1H, d, J=9Hz), 6.50 (1H, m), 6.66 (2H, d, J=9Hz), 7.88 (2H, d, J=9Hz).

### Example 20

To a mixed solution of 5-hydroxy-4-methoxy-2,6,6-trimethyl-2-cyclohepten-1-one (0.99g, 5mM) obtained in Example 4 in acetonitrile (15ml) and pyridine (4ml), 2-tritylaminothiazole-4-acetic acid (2.6g, 6.5mM), DCC (1.34g, 6.5mM) and dimethyl aminopyridine (0.1g) were added under ice-cooling and stirring and stirred overnight. The reaction solution was filtered and the filtrate was concentrated. The residue was diluted with ethyl acetate and washed successively with water, a sodium bicarbonate solution and a saturated saline solution, dried over magnesium sulfate, filtered and concentrated. The residue was purified by silica gel column chromatography using hexane-ethyl acetate (5:1) to obtain 1.31g of 4-methoxy-2,6,6-trimethyl-5-(2-tritylaminothiazol-4-yl) acetoxy-2-cyclohepten-1-one as a colorless oily product.
1H-NMR (CDCl₃, ppm): 0.97 (6H, s), 1.84 (3H, t, J=2Hz), 2.45 (1H, d, J=13Hz), 2.52 (1H, d, J=2Hz), 3.35 (3H, s), 3.60 (2H, s), 3.93 (1H, dm, J=9Hz), 4.85 (1H, d, J=9Hz), 6.20 (1H, s), 6.43 (1H, m), 6.68 (1H, brs), 7.29 (15H, m).

To the product so obtained was added 80% acetic acid (5ml), warmed to 70 °C for three hours and concentrated. The residue was purified by silica gel column chromatography using hexane-ethyl acetate (1:1) as an eluent and purified from a mixed solvent of ethyl acetate and hexane to obtain 0.40g (23%) of 5-(2-aminothiazol-4-yl) acetoxy-4-methoxy-2,6,6-trimethyl-2-cyclohepten-1-one (AU243) as yellow plates. melting point: 116 to 117 °C.
IR (KBr, cm⁻¹): 3376, 3296, 3127, 1733, 1660, 1532, 1262.
1H-NMR (CDCl₃, ppm): 0.99 (6H, s), 1.85 (3H, t, J=2Hz), 2.45 (1H, d, J=13Hz), 2.53 (1H, d, J=13Hz), 3.40 (3H, s), 3.61 (2H, s), 3.95 (1H, dm, J=9Hz), 4.86 (1H, d, J=9Hz), 5.49 (2H, brs), 6.35 (1H, s), 6.44 (1H, m).

### Example 21

To a solution of 5-hydroxy-4-methoxy-2,6,6-trimethyl-2-cyclohepten-1-one obtained in Example 4 (1.98g, 10mM) in pyridine (10ml), phthalic anhydride (1.78g, 12mM) and dimethyl aminopyridine (0.05g) were added and warmed to 90 °C for three hours, left to cool, poured onto ice-water, alkalified with potassium carbonate and extracted with ethyl acetate. The aqueous layer was acidified with hydrochloric acid and extracted with ethyl acetate, washed with brine, dried over magnesium sulfate, filtered and concentrated. The residue was crystallized from a mixed solvent of ethyl acetate and hexane to obtain 1.80g (53%) of 5-(2-carboxybenzoyloxy)-4-methoxy-2,6,6-trimethyl-2-cyclohepten-1-one (AU218) as colorless plates. melting point: 158.0 to 158.5 °C.
IR (KBr, cm⁻¹): 3600-2300, 1726, 1694, 1672, 1294, 748.
1H-NMR (CDCl₃, ppm): 1.11 (3H, s), 1.15 (3H, s), 1.88 (3H, t, J=2Hz), 2.53 (1H, d, J=13Hz), 2.61 (1H, d, J=13Hz), 3.45 (3H, s), 4.09 (1H, dm, J=9Hz), 5.12 (1H, d, J=9Hz), 6.51 (1H, m), 7.61 (2H, m), 7.75 (1H, m), 7.90 (1H, m).

### Example 22

5-hydroxy-4-methoxy-2,6,6-trimethyl-2-cycloheptene-1-one obtained in Example 4 (5.95g, 30mM) was treated with 2,4-dinitrobenzoic acid according to the procedures described in Example 20. The mixture was re-crystallized twice from a mixed solution of ethyl acetate and hexane to obtain 7.24g (61.5%) of 5-(2,4-dinitrobenzoyloxy)-4-methoxy-2,6,6-trimethyl-2-cyclohepten-1-one (AU407) as reddish needles. melting point: 147.5 to 149°C.
IR (KBr, cm⁻¹): 1741, 1669, 1541, 1348, 1284, 1101, 735.
1H-NMR (CDCl₃, ppm): 1.06 (3H, s), 1.17 (3H, s), 1.90 (3H, t, J=2Hz), 2.51 (1H, d, J=13Hz), 2.59 (1H, d, J=13Hz), 3.45 (3H, s), 4.06 (1H, dm, J=9Hz), 5.10 (1H, d, J=9Hz), 6.49 (1H, m), 7.99 (1H, d, J=8Hz), 8.55 (1H, dd, J=8, 2Hz), 8.78 (1H, d, J=8Hz).

### Example 23

The product of Example 22 (5.0g, 12.7mM) was reduced at nitro groups according to the procedure described in Example 19 to obtain 2.27g (55.4%) of 5-(2,4-diaminobenzoyloxy)-4-methoxy-2,6,6- trimethyl-2-cyclohepten-1-one (AU408) as yellow plates. melting point: 178 to 179°C.
IR (KBr, cm⁻¹): 3498, 3451, 3366, 1665, 1624, 1585, 1542, 1258, 1148, 770.
1H-NMR (CDCl₃, ppm): 1.07 (3H, s), 1.16 (3H, s), 1.87 (3H, t, J=2Hz), 2.52 (1H, d, J=13Hz), 2.61 (1H, d, J=13Hz), 3.43 (3H, s), 3.92 (2H, brs; disappeared by the addition of heavy water), 4.06 (1H, dm, J=9Hz), 5.02 (1H, d, J=9Hz), 5.74 (2H, brs; disappeared by the addition of heavy water), 5.87 (1H, d, J=2Hz), 5.99 (1H, dd, J=9, 2Hz), 6.52 (1H, m), 7.69 (1H, d, J=9Hz).

The above product so obtained (1.3g, 4.0mM) was dissolved in ethyl acetate and added by a solution of 4N hydrogen chloride in ethyl acetate. The precipitate was collected by filtration to obtain 1.5g (95%) of a hydrochloride as a colorless powder. melting point: 115 °C(decomposition).
IR (KBr, cm ):3450, 3326, 3200-2400, 1673, 1634, 1247, 1109, 769.
1H-NMR (DMSO-d6, ppm): 0.92 (3H, s), 1.09 (3H, s), 1.79 (3H, brt), 2.39 (1H, d, J=13Hz), 2.73 (1H, d, J=13Hz), 3.33 (3H, s), 4.29 (1H, brm), 4.79 (1H, d, J=9Hz), 6.29 (1H, m), 6.40 (1H, m), 6.58 (1H, m), 7.71 (1H, m).

### Example 24

5-hydroxy-4-methoxy-2,6,6-trimethyl-2-cyclohepten-1-one obtained in Example 4 (25.0g, 126mM) was treated with 3,4-dinitrobenzoic acid according to the procedures described in Example 20, and crystallized from methanol to obtain 42.1g (85%) of 5-(3,4-dinitrobenzoyloxy)-4-methoxy-2,6,6-trimethyl-2-cyclohepten-1-one (AU409) as light yellow needles. melting point:103 to 104 °C.
IR (KBr, cm⁻¹): 1734, 1668, 1545, 1369, 1354, 1251, 847.
1H-NMR (CDCl₃, ppm): 1.10 (3H, s), 1.19 (3H, s), 1.91 (3H, t, J=2Hz), 2.56 (1H, d, J=13Hz), 2.65 (1H, d, J=9Hz), 3.40 (3H,s), 4.13 (1H, dm, J=9Hz), 5.11 (1H, d, J=9Hz), 6,49 (1H, m), 8.01 (1H, d, J=9Hz), 8.43 (1H, dd, J=9, 2Hz), 8.57 (1H, d, J=2Hz).

### Example 25

The product of Example 24 (22g, 56mM) was reduced at nitro groups according to the procedures described in Example 19 and crystallized from methanol to obtain 2.95g (16%) of 5-(3,4-diaminobenzoyloxy)-4-methoxy-2,6,6-trimethyl-2-cyclohepten-1-one (AU410) as light red needles. melting point: 136 °C (decomposion).
IR (KBr, cm⁻¹):3472, 3362, 1684, 1662, 1310, 1288, 1224, 764.
1H-NMR (CDCl₃, ppm): 1.07 (3H, s), 1.16 (3H, s), 1.88 (3H, t, J=2Hz), 2.55 (1H, d, J=13Hz), 2.63 (1H, d, J=13Hz), 3.41 (3H, s), 4.09 (1H, dm, J=9Hz), 5.08 (1H, d, J=9Hz), 5.50 (1H, m), 6.69 (1H, d, J=8Hz), 7.43 (1H, d, J=2Hz), 7.50 (1H, dd, J=8, 2Hz).

### Example 26

To a solution of the product of Example 25 (1.44g, 4.77mM) in tetrahydrofuran (10ml), cyanogen bromide (0.47g, 4.78mM) was added at room temperature under stirring and after five hours, cyanogen bromide (0.26g, 2.60mM) was further added and stirred overnight. The reaction mixture was added by a saturated sodium bicarbonate solution and extracted with ethyl acetate and reverse extracted with diluted hydrochloric acid. The extracted solution was added by sodium bicarbonate to basify, extracted with ethyl acetate, dried over magnesium sulfate, filtered and concentrated to obtain 1.21g (76%) of 5-(2-aminobenzimidazolyl-5-carbonyloxy)-4-methoxy-2,6,6-trimethyl-2-cyclohepten-1-one (AU415) as a glassy material. melting point: 131 to 132.5 °C.
IR (KBr, cm⁻¹): 3400-3100, 1709, 1646, 1559, 1289, 1198.
1H-NMR (CDCl₃, ppm): 1.08 (3H, s), 1.19 (3H, s), 1.86 (3H, t, J=2Hz), 2.54 (1H, d, J=13Hz), 2.62 (1H, d, J=13Hz), 3.41 (3H, s), 4.12 (1H, dm, J=9Hz), 5.09 (1H, d, J=9Hz), 5.51 (2H, br; disappeared by the addition of heavy water), 6.47 (1H, m), 7.29 (1H, d, J=8Hz), 7.82 (1H, d, J=8Hz), 7.98 (1H, brs).

### Example 27

The product of Example 25 (1g, 3mM) was added to dimethylforrmamide dimethylacetal (1.5ml) and was stirred at 50°C for one hour. The reaction solution was concentrated, the residue was purified by silica gel column chlomatography using ethyl acetate-hexane (3:1) as an eluent and crystallized from a mixed solution of ethyl acetate and hexane to obtain 0.1g (10%) of 5-(5-benzimidazolylcarbonyloxy)-4-methoxy-2,6,6-trimethyl-2-cyclohepten-1-one (AU507) as a brown glassy product. melting point: 155 to 157 °C.
IR (KBr, cm⁻¹): 3600-3100, 1705, 1654, 1302, 1124, 750
1H-NMR (CDCl₃, ppm): 1.11 (3H, s), 1.22 (3H, s), 1.89 (3H, t, J=2Hz), 2.56 (1H, d, J=13Hz), 2.66 (1H, d, J=13Hz), 3.41 (3H, s), 4.13 (1H, dm, J=9Hz), 5.14 (1H, d, J=9Hz), 6.52 (1H, m), 7.69 (1H, d, J=8Hz), 8.05 (1H, d, J=8Hz), 8.23 (1H, s), 8.47 (1H, s).

### Example 28

4-benzyloxy-5-hydroxy-2,6,6-trimethyl-2-cyclohepten-1-one obtained in Example 2 (50mg, 0.18mM) was acetylated by a conventional method to obtain 49mg (91%) of 5-acetoxy-4-benzyloxy-2,6,6-trimethyl-2-cyclohepten-1-one (AU156 acetate) as a colorless oily product.
1H-NMR (CDCl₃, ppm): 1.01 (3H, s), 1.02 (3H, s), 1.84 (3H, t, J=2Hz), 2.04 (3H, s), 2.43 (1H, d, J=13Hz), 2.53 (2H, d, J=13Hz), 4.20 (1H, dm, J=9Hz), 4.63 (1H, d, J=12Hz), 4.71 (2H, d, J=12Hz), 4,91 (1H, d, J=9Hz), 6,52 (1H, m), 7.31 (5H, m).

### Example 29

Saishin N (5.53g, 30mM) was acetylated by a conventional method and re-crystallized from a mixed solvent of ethyl acetate and hexane to obtain 6.58g (81%) of 4,5-diacetoxy-2,6,6-trimethyl-2-cyclohepten-1-one (AU102) as colorless needles. melting point: 68 to 69 °C.
IR (KBr, cm⁻¹): 1741, 1681, 1252, 1041.
1H-NMR (CDCl₃, ppm): 1.02 (3H, s), 1.09 (3H, s), 1.86(3H, t, J=2Hz), 2.09(3H,s), 2.10 (3H, s), 2.48 (1H, d, J=13Hz), 2.66 (1H, d, J=13Hz), 4.89 (1H, d, J=9Hz), 5.75 (1H, dm, J=9Hz), 6.26 (1H, m).

### Example 30

Saishin N (1.84g, 10mM) was benzoylated according to the procedures described in Example 6 and fractionated by silica gel column chromatography using hexane-ethyl acetate (10:1) as an eluent. The first eluted portion was crystallized from a mixed soluent of ethyl acetate and hexane to obtain 0.98g (34.1%) of 4-benzoyloxy-5-hydroxy-2,6,6-trimethyl-2-cyclohepten-1-one (AU190) as colorless plates. melting point: 127 to 128°C.
IR (KBr, cm⁻¹): 3551, 1718, 1674, 1333, 1272, 1111, 705.
1H-NMR (CDCl₃+D₂O, ppm): 1.12 (3H, s), 1.16 (3H, s), 1.87 (3H, t, J=2Hz), 2.45 (1H, d, J=13Hz), 2.67 (1H, d, J=13Hz), 3.55 (1H, d, J=9Hz), 5.85 (1H, dm, J=9Hz), 6.42 (1H, m), 7.48 (2H, m), 7.61 (1H, m), 8.09 (2H, m).

0.7g (26%) of 5-benzoyloxy-4-hydroxy-2,6,6-trimethyl-2-cyclohepten-1-one (AU183) as that in Example 7 was obtained from the successive eluted portion.

### Example 31

4-benzoyloxy-5-hydroxy-2,6,6-trimethyl-2-cyclohepten-1-one obtained in Example 30 (0.60g, 2.08mM) was acetylated by a conventional method and crystllized from water to obtain 0.47g (67%) of 5-acetoxy-4-benzoyloxy-2,6,6-trimethyl-2-cyclohepten-1-one (AU193) as colorless crystals. melting point: 94 to 95 °C.
IR (KBr, cm⁻¹): 1735, 1722, 1672, 1273, 1232, 714.
1H-NMR (CDCl₃, ppm): 1.06 (3H, s), 1.15 (3H, s), 1.82 (3H, s), 1.90 (3H, t, J=2Hz), 2.52 (1H, d, J=13Hz), 2.73 (1H, d, J=13Hz), 5.03 (1H, d, J=9Hz), 6.04 (1H, dm, J=9Hz), 6.41 (1H, m), 7.47 (2H, brt, J=7Hz), 7.59 (1H, brt, J=7Hz), 8.04 (2H, brd, J=7Hz).

### Example 32

Saishin N (1.84g, 10mM) was treated with 3,4-dimethoxybenzoyl chloride (2.41g, 12mM) according to the procedures described in Example 17 and fractionated by silica gel column chromatography using benzene-ethyl acetate (5:1) as an eluent. The first eluted portion was crystallized from a mixed solution of ethyl acetate and hexane to obtain 1.01g (32%) of 4-(3,4-dimethoxybenzoyloxy)-5-hydroxy-2,6,6-trimethyl-2-cyclohepten-1-one (AU133) as colorless plates. melting point: 161 to 163.5 °C.
IR (KBr, cm⁻¹): 3517, 1719, 1680, 1517, 1271, 1215, 758.
1H-NMR (CDCl₃+D₂O, ppm): 1.13 (3H, s), 1.17 (3H, s), 1.89 (3H, t, J=2Hz), 2.46 (1H, d, J=13Hz), 2.67 (1H, d, J=13Hz), 3.54 (1H, d, J=9Hz), 3.95 (3H, s), 3.96 (3H, s), 5.84 (1H, dm, J=9Hz), 6.44 (1H, m), 6.92 (1H, d, J=8Hz), 7.58 (1H, d, J=2Hz), 7.74 (1H, dd, J=8, 2Hz).

The successive eluted portion was crystallized from a mixed solvent of ethyl acetate and ethanol to obtain 0.87g (28%) of 5-(3,4-dimethoxybenzoyloxy)-4-hydroxy-2,6,6-trimethyl-2-cyclohepten-1-one (AU134) as colorless plates. melting point: 150 to 151.5 °C.
IR (KBr, cm⁻¹): 3485, 1707, 1669, 1598, 1518, 1279, 1224, 762.
1H-NMR (CDCl₃+D₂O, ppm): 1.09 (3H, s), 1.21 (3H, s), 1.90 (3H, t, J=2Hz), 2.48 (1H, d, J=13Hz), 2.65 (1H, d, J=13Hz), 3,94 (3H, s), 3.95 (3H, s), 4.63 (1H, dm, J=9Hz), 4.91 (1H, d, J=9Hz), 6.55 (1H, m), 6.92 (1H, d, J=8Hz), 7.57 (1H, d, J=2Hz), 7.72 (1H, dd, J=8, 2Hz).

### Example 33

Saishin N (3.68g, 20mM) was treated with 4-methoxybenzoylchloride (4.27g, 25mM) as described in Example 17 and fractionated by silica gel column chromatography using hexane-ethyl acetate (10:1) as an eluent. The first eluted portion was crystallized from a mixed solvent of ethyl acetate and hexane to obtain 2.44g (38%) of 5-hydroxy-4-(4-methoxybenzoyloxy)-2,6,6-trimethyl-2-cyclohepten-1-one (AU195) as colorless plates. melting point: 108.5 to 110 °C.
IR (KBr, cm⁻¹): 3517, 1682, 1608, 1342, 1290, 1182, 1106, 774.
1H-NMR (CDCl₃, ppm): 1.12 (3H, s), 1.17 (3H, s), 1.88 (3H, t, J=2Hz), 2.45 (1H, d, J=13Hz), 2.67 (1H, d, J=13Hz), 3.55 (1H, dd, J=9, 6Hz), 3.89 (3H, s), 5.83 (1H, dm, J=9Hz), 6.43 (1H, m), 6.96 (2H, d, J=9Hz), 8.06 (2H, d, J=9Hz).

1.63g (25%) of 4-hydroxy-5-(4-methoxybenzoyloxy)-2,6,6-trimethyl-2-cyclohepten-1-one (AU202) as a light yellow oily product from the successive eluted portion.
IR (KBr, cm⁻¹): 3474, 1716, 1671, 1605, 1258, 1169, 1102, 768.
1H-NMR (CDCl₃+D₂O, ppm): 1.07 (3H, s), 1.19 (3H, s), 1.88 (3H, t, J=2Hz), 2.46 (1H, d, J=13Hz), 2.63 (1H, d, J=13Hz), 3.87 (3H, s), 4.61 (1H, dm, J=9Hz), 4.89 (1H, d, J=9Hz), 6.54 (1H, m), 6.94 (2H, d, J=9Hz), 8.02 (2H, d, J=9Hz).

### Example 34

5-hydroxy-4(4-methoxybenzoyloxy)-2,6,6-trimethyl-2-cyclohepten-1-one (796mg, 2.5mM) obtained in Example 33 was acetylated by a conventional method and crystallized from water to obtain 867mg (96%) of 5-acetoxy-4-(4-methoxybenzoyloxy)-2,6,6-trimethyl-2-cyclohepten-1-one (AU198) as colorless plates. melting point: 93 to 94 °C.
IR (KBr, cm⁻¹): 1749, 1719, 1673, 1605, 1256, 770.
1H-NMR (CDCl₃, ppm) 1.04 (3H, s), 1.14 (3H, s), 1.81 (3H, s), 1.88 (3H, t, J=2Hz), 2.51 (1H, d, J=13Hz), 2.72 (1H, d, J=13Hz), 3.87 (3H, s), 4.99 (1H, d, J=9Hz), 6.01 (1H, dm, J=9Hz), 6.41 (1H, m), 6.94 (2H, d, J=9Hz), 7.98 (2H, d, J=9Hz ).

### Example 35

4-hydroxy-5-(4-methoxybenzoyloxy)-2,6,6-trimethyl-2-cyclohepten-1-one (1.46g, 4.5mM) obtained in Example 33 was acetylated by a conventional method and crystallized from a mixed solvent of ethyl acetate and hexane to obtain 0.91g (56%) of 4-acetoxy-5-(4-methoxybenzoyloxy)-2,6,6-trimethyl-2-cyclohepten-1-one (AU203) as colorless prisms. meling point: 78 to 80°C.
IR (KBr, cm⁻¹): 1762, 1715, 1672, 1604, 1259, 1214, 1176, 1101, 1031, 850, 768.
1H-NMR (CDCl₃, ppm): 1.08 (3H, s), 1.23 (3H, s), 1.78 (3H, s), 1.89 (3H, t, J=2Hz), 2.52 (1H, d, J=13Hz), 2.73 (1H, d, J=13Hz), 3.87 (3H, s), 5.07 (1H, d, J=9Hz), 5.97 (1H, dm, J=9Hz), 6.36 (1H, m), 6.94 (2H, d, J=9Hz), 7.98 (2H, d, J=9Hz).

### Example 36

Saishin N (3.68g, 20mM) was treated with 3-nitrobenzoyl chloride (4.45g, 24mM) as described in Example 17 and purified by silica gel column chromatography using hexane-ethyl acetate (5:1) as an eluent and crystallized from a mixed solvent of ethyl acetate and hexane to obtain 1.79g (24%) of 5-hydroxy-4-(3-nitrobenzoyloxy)-2,6,6-trimethyl-2-cyclohepten-1-one (AU200) as colorless prisms. melting point: 131 to 133 °C.
IR (KBr, cm⁻¹): 3517, 1718, 1668, 1534, 1355, 1288, 1268, 1144, 719.
1H-NMR (CDCl₃+D₂O, ppm): 1.14 (3H, s), 1.17 (3H, s), 1.90 (3H, t, J=2Hz), 2.48 (1H, d, J=13Hz), 2.68 (1H, d, J=13Hz), 3.57 (1H, d, J=9Hz), 5.93 (1H, dm, J=9Hz), 6.45 (1H, m), 7.71 (1H, t, J=8Hz), 8.46 (2H, m), 8.91 (1H, t, J=2Hz),

### Example 37

The product of Example 36 (750mg, 2.8mM) was acetylated by a conventional method and crystallized from a mixed solvent of ethyl acetate and hexane to obtain 1,79g (24%) of 5-acetoxy-4-(3-nitrobenzoyloxy)-2,6,6-trimethyl-2-cyclohepten-1-one (AU204) as colorless needles. melting point: 108.5 to 110 °C. IR (KBr, cm⁻¹): 1742, 1666, 1538, 1350, 1263, 1133, 714.
1H-NMR (CDCl₃, ppm): 1.07 (3H, s), 1.16 (3H, s), 1.87 (3H, s), 1.91 (3H, t, J=2Hz), 2.56 (1H, d, J=13Hz), 2.73 (1H, d, J=13Hz), 5.07 (1H, d, J=9Hz), 6.03 (1H, dm, J=9Hz), 6.38 (1H, m), 7.70 (1H, t, J=8Hz), 8.35 (1H, m), 8.47 (1H, m), 8.88 (1H, m).

### Example 38

To a solution of 5-hydroxy-4-(3-nitrobenzoyloxy)-2,6,6-trimethyl-2-cyclohepten-1-one (3.03g, 8mM) obtained in Example 36 in benzene (200ml), activated iron (13g) was added and ethanol (2.2ml) was added and heated under reflux. The mixture was further added by water (0.5ml) little by little and heated under reflux for four hours and filtered. The filtrate was then concentrated and the residue was fractionated by silica gel column chromatography using hexane-ethyl acetate (2:1) as an eluent. From the first eluted portion 1.27g (52%) of 4-(3-aminobenzoyloxy)-5-hydroxy-2, 6,6-trimethyl-2-cyclohepten-1-one (AU210) was obtained as a yellow oily product.
IR (KBr, cm⁻¹): 3450, 3375, 1718, 1670, 1291, 1234, 752.
1H-NMR (CDCl₃, ppm): 1.11 (3H, s), 1.15 (3H, s), 1.87 (3H, s), 2.35 (1H, br), 2.44 (1H, d, J=13Hz), 2.66 (1H, d, J=13hz), 3.53 (1H, d, J=9Hz), 3.85 (2H, br), 5.82 (1H, dm, J=9Hz), 6.40 (1H, m), 6.90 (1H, m), 7.24 (1H, m), 7.38 (1H, m), 7.46 (1H, m).

The successive eluted portion was crystallized from a mixed solvent of ethyl acetate and hexane to obtain 0.89(37%) of 5-(3-aminobenzoyloxy)-4-hydroxy-2,6,6-trimethyl-2-cyclohepten-1-one (AU209) as light yellow prisms. melting point: 121 to 123 °C.
IR (KBr, cm⁻¹): 3386, 3326, 1717, 1654, 1285, 1233, 752.
1H-NMR (CDCl₃, ppm): 1.07 (3H, s), 1.18 (3H, s), 1.87 (3H, t, J=2Hz), 2.46 (1H, d, J=13Hz), 2.52 (1H, br), 2.63 (1H, d, J=13Hz), 3.84 (2H, br), 4.61 (1H, dm, J=9Hz), 4.91 (1H, d, J=9Hz), 6.51 (1H, m), 6.89 (1H, m), 7.23 (1H, t, J=8Hz), 7.36 (1H, m), 7.45 (1H, m).

### Example 39

4-(3-aminobenzoyloxy)-5-hydroxy-2,6,6-trimethyl-2-cyclohepten-1-one (970mg, 3.2mM) obtained in Example 38 was dissolved in ethyl acetate and was added by a solution of 4N hydrogen chloride in ethyl acetate. The mixture was left to stand overnight and the precipitate was collected by filtration to obtain 990mg (91%) of a hydrochloride of the above compound. melting point: 204 to 204.5 °C(decomposition).
IR (KBr, cm⁻¹): 3282, 3000-2500, 1712, 1664, 1290, 1276, 755.

### Example 40

5-(3-aminobenzoyloxy)-4-hydroxy-2,6,6-trimethyl-2-cyclohepten-1-one (152mg, 0.5mM) obtained in Example 38 was acetylated by a conventional method to obtain 125mg (65%) of 5-(3-acetamidobenzoyloxy)-4-acetoxy-2,6,6-trimethyl-2-cyclohepten-1-one (AU224) as a colorless oily product.
IR (KBr, cm⁻¹): 3362, 1734, 1675, 1558, 1285, 1229, 754.
1H-NMR (CDCl₃, ppm): 1.07 (3H, s), 1.23 (3H, s), 1.79 (3H, s), 1.89(3H, t, J=2Hz), 2.20 (3H, s), 2.53 (1H, d, J=13Hz), 2.73 (1H, d, J=13Hz), 5.08 (1H, d, J=9Hz), 5.96 (1H, dm, J=9Hz), 6.37 (1H, m), 7.42 (1H, brt, J=8Hz), 7.74 (1H, brd, J=8Hz), 7.95 (1H, brs). 8.06 (1H, brd, J=8Hz).

### Example 41

Saishin N (1.84g, 10mM) was treated with cinnamoyl chloride (2.00g, 12mM) as described in Example 17 and fractionated by silica gel column chlomatography using hexane-ethyl acetate (5:1) as an eluent. From the first eluted portion 1.28g (41%) of 4-cinnamoyloxy-5-hydroxy-2,6,6-trimethyl-2-cyclohepten-1-one (AU206) was obtained as a colorless oily product.
IR (KBr, cm⁻¹): 3492, 1711, 1674, 1636, 1165, 768.
1H-NMR (CDCl₃+D₂O, ppm): 1.10 (3H, s), 1.15 (3H, s), 1.87 (3H, t, J=2Hz), 2.43 (1H, d, J=13Hz), 2.64 (1H, d, J=13Hz), 3.48 (1H, d, J=9Hz), 5.72 (1H, dm, J=9Hz), 6.38 (1H, m), 6.53 (1H, d, J=16Hz), 7.41 (3H, m), 7.56 (2H, m), 7.80 (1H, d, J=16Hz).

The successive eluted portion was crystallzed from a mixed solvent of ethyl acetate and hexane to obtain 0.72g (22%) of 5-cinnamoyloxy-4-hydroxy-2,6,6-trimethyl-2-cyclohepten-1-one (AU205) as colorless needles. melting point: 85.5 to 87°C.
IR (KBr, cm⁻¹): 3482, 1711, 1674, 1279, 769.
1H-NMR (CDCl₃+D₂O, ppm): 1.08 (3H, s), 1. 15 (3H, s), 1.89 (3H, t, J=2Hz), 2.46 (1H, d, J=13Hz), 2.62 (1H, d, J=13Hz), 4.58 (1H, dm, J=9Hz), 4.82 (1H, d, J=9Hz), 6.51 (1H, d, J=16Hz), 6.53 (1H, m), 7.42 (3H, m), 7.55 (2H, m), 7.77 (1H, d, J=16Hz).

### Example 42

4-cinnamoyloxy-5-hydroxy-2,6,6-trimethyl-2-cyclohepten-1-one (0.98g, 3.1mM) obtained in Example 41 was acetylated by a conventional method and crystallized from a mixed solvent of ethyl acetate and hexane to obtain 0.98g (86%) of 5-acetoxy-4-cinnamoyloxy-2,6,6-trimethyl-2-cyclohepten-1-one (AU208) as colorless plates. melting point: 138 to 139°C.
IR (KBr, cm⁻¹): 1743, 1718, 1675, 1633, 1310, 1232, 1159, 773.
1H-NMR (CDCl₃+D₂O, ppm): 1.05 (3H, s), 1.14 (3H, s), 1.89 (3H, t, J=2Hz), 2.00 (3H, s), 2.51 (1H, d, J=13Hz), 2.71 (1H, d, J=13Hz), 4.95 (1H, d, J=9Hz), 5.93 (1H, dm, J=9Hz), 6.37 (1H, m), 6.44 (1H, d, J=16Hz), 7.42 (3H, m), 7.55 (2H, m), 7.74 (1H, d, J=16Hz).

### Example 43

5-cinnamoyloxy-4-hydroxy-2,6,6-trimethyl-2-cyclohepten-1-one (0.39g, 1.2mM) obtained in Example 41 was acetylated by a conventional method and crystallized from a mixed solvent of ethyl acetate and hexane to obtain 0.19g (44%) of 4-acetoxy-5-cinnamoyloxy-2,6,6-trimethyl-2-cyclohepten-1-one (AU207) as colorless prisms. melting point: 64 to 65 °C.
IR (KBr, cm⁻¹): 1756, 1716, 1672, 1633, 1312, 1225, 1158, 769.
1H-NMR (CDCl₃, ppm): 1.07 (3H,s), 1.18 (3H, s), 1.89 (3H, t, J=2Hz), 2.01 (3H, s), 2.52 (1H, d, J=13Hz), 2.70 (1H, d, J=13Hz), 5.01 (1H, d, J=9Hz), 5.88 (1H, dm, J=9Hz), 6.32 (1H, m), 6.45 (1H, d, J=16Hz), 7.42 (3H, m), 7.56 (2H, m), 7.72 (1H, d, J=16Hz).

### Example 44

Saishin N (1.84g, 10mM) was treated with 4-nitrobenzoyl chloride (2.23g, 12mM) according to the procedures described in Example 17. Crystallization of the mixture from ethyl acetate was repeated twice to obtain 0.97g (26%) of 5-hydroxy-4-(4-nitrobenzoyloxy)-2,6,6-trimethyl-2-cyclohepten-1-one (AU212) as light yellow prisms. melting point: 151 to 152.5 °C.
IR (KBr, cm⁻¹): 3556, 1731, 1660, 1524, 1354, 1273, 1105, 722.
1H-NMR (CDCl₃+D₂O, ppm): 1.14 (3H, s), 1.17 (3H, s), 1.90 (3H, t, J=2Hz), 2.48 (1H, d, J=13Hz), 2.68 (1H, d, J=13Hz), 3,55 (1H, d, J=9Hz), 5.91 (1H, dm, J=9Hz), 6.43 (1H, m), 8.27 (2H, d, J=9Hz), 8.34 (2H, d, J=9Hz).

The mother liquor of the crystal was concentrated. Then crystallization of the residue was repeated twice from ethyl acetate to obtain 0.99g (26%) of 4-hydroxy-5-(4-nitrobenzoyloxy)-2,6,6-trimethyl-2-cyclohepten-1-one (AU211) as light yellow needles. melting point: 148 to 150 °C.
IR (KBr, cm⁻¹): 3552, 1716, 1674, 1522, 1349, 1275, 719.
1H-NMR (CDCl₃+D₂O, ppm): 1.10 (3H, s), 1.23 (3H, s), 1.91 (3H, t, J=2Hz), 2.50 (1H, d, J=13Hz), 2.66 (1H, d, J=13Hz), 4.65 (1H, dm, J=9Hz), 4.97 (1H, d, J=9Hz), 6.53 (1H, m), 8.25 (2H, d, J=9Hz), 8.32 (2H, d, J=9Hz).

### Example 45

5-hydroxy-4-(4-nitrobenzoyloxy)-2,6,6-trimethyl-2-cyclohepten-1-one (751mg, 2mM) obtained in Example 44 was reduced at nitro group according to the procedures described in Example 38 and fractionated by silica gel column chromatography using hexane-ethyl acetate (2:1) as an eluent. The first eluted portion was crystallized from a mixed solvent of ethyl acetate and hexane to obtain 320mg (53%) of 4-(4-aminobenzoyloxy)-5-hydroxy-2,6,6-trimethyl-2-cyclohepten-1-one (AU215) as colorless prisms. melting point: 152 to 153°C.
IR (KBr, cm⁻¹): 3648, 3524, 3437, 3360, 1685, 1672, 1605, 1273, 770.
1H-NMR (CDCl₃, ppm): 1.11 (3H, s), 1.16 (3H, s), 1.86 (3H, t, J=2Hz), 2.30 (1H, d, J=5Hz), 2.43 (1H, d, J=12Hz), 2.66 (1H, d, J=12Hz), 3.52 (1H, dd, J=9, 5Hz), 4.14 (2H, brs), 5.79 (1H, dm, J=9Hz), 6.42 (1H, m), 6.66 (2H, d, J=9Hz), 7.90 (2H, d, J=9Hz).

The successive eluted portion was crystallized from a mixed solvent of ethyl acetate and hexane to obtain 225mg (37%) of 5-(4-aminobenzoyloxy)-4-hydroxy-2,6,6-trimethyl-2-cyclohepten-1-one (AU216 ) as colorless needles. melting point: 169.5 to 171°C.
IR (KBr, cm⁻¹): 3460, 3366, 3234, 1676, 1633, 1600, 1279, 1170.
1H-NMR (CDCl₃,ppm): 1.07 (3H, s), 1.19 (3H, s), 1.88 (3H, t, J=2Hz), 2.45 (1H, d, J=13Hz), 2.51 (1H, brd, J=7Hz), 2.63 (1H, d, J=13Hz), 4.14 (2H, brs), 4.61 (1H, brs), 4.87 (1H, d, J=9Hz), 6.55 (1H, m), 6.66 (2H, d, J=9Hz), 7.88 (2H, d, J=9Hz).

### Example 46

4-hydroxy-5-(4-nitrobenzoyloxy)-2,6,6-trimethyl-2-cyclohepten-1-one obtained in Example 44 was treated as described in Example 45 to obtain 4-(4-aminobenzoyloxy-5-hydroxy-2,6,6-trimethyl-2-cyclohepten-1-one (AU215) and 5-(4-aminobenzoyloxy)-4-hydroxy-2,6,6-trimethyl-2-cyclohepten-1-one (AU216) as in Example 45.

### Example 47

A solution of 5-hydroxy-4-(4-nitrobenzoyloxy)-2,6,6-trimethyl-2-cyclohepten-1-one (3.33g, 10mM) obtained in Example 44 in ethyl acetate (30ml) was added by platinum oxide (0.1g), and catalytically reduced. After the catalyst was filtered off, the filtrate was treated as described in Example 45 to obtain 2.53g (76%) of 4-(4-aminobenzoyloxy)-5-hydroxy-2,6,6-trimethyl-2-cyclohepten-1-one (AU215) as in Example 45.

### Example 48

5-hydroxy-4-(4-nitrobenzoyloxy)-2,6,6-trimethyl-2-cyclohepten-1-one (638mg, 2mM) obtained in Example 44 was acetylated by a conventional method and crystallized from a mixed solvent of ethyl acetate and hexane to obtain 683mg (91%) of 5-acetoxy-4-(4-nitrobenzoyloxy)-2,6,6-trimethyl-2-cyclohepten-1-one (AU196) as colorless needles. melting point: 147 to 147.5 °C.
IR (KBr, cm⁻¹): 1735, 1722, 1685, 1523, 1282, 1234, 718.
1H-NMR (CDCl₃, ppm): 1.06 (3H, s), 1.15 (3H, s), 1.84 (3H, s), 1.91 (3H, t, J=2Hz), 2.55 (1H, d, J=13Hz), 2.72 (1H, d, J=13Hz), 5.05 (1H, d, J=9Hz), 6.03 (1H, dm, J=9Hz), 6.36 (1H, m), 8.21 (2H, d, J=9Hz), 8.33 (2H, d, J=9Hz).

### Example 49

The product of Example 48 (210mg, 0.56mM) was reduced at nitro group as described in Example 38 and crystallized from a mixed solution of benzene and hexane to obtain 175mg (94%) of 5-acetoxy-4-(4-aminobenzoyloxy)-2,6,6-trimethyl-2-cyclohepten-1-one (AU197). melting point: 158.5 to 160°C.
IR (KBr, cm⁻¹): 3463, 3380, 3246, 1747, 1701, 1673, 1628, 1601, 1267.
1H-NMR (CDCl₃, ppm): 1.05 (3H, s), 1.14 (3H, s), 1.83 (3H, s), 1.89 (3H, t, J=2Hz), 2.50 (1H, d, J=13Hz), 2.73 (1H, d, J=13Hz), 4.13 (2H, brs: disappeared by the addition of heavy water), 4.98 (1H, d, J=9Hz), 6.00 (1H, dm, J=9Hz), 6.42 (1H, m), 6.66 (2H, d, J=9Hz), 7.84 (2H, d, J=9Hz).

### Example 50

To a solution of 5-hydroxy-4-(4-nitrobenzoyloxy)-2,6,6-trimethyl-2-cyclohepten-1-one (1.67g, 5mM) obtained in Example 44 in tetrahydrofuran (5ml), dihydropyran (4.5ml) and p-toluenesulfonic acid (0.1g) were added and stirred at room temperature overnight. The reaction mixture was concentrated, then diluted with ethyl acetate, washed with an aqueous dilute pottasium carbonate solution and with brine, dried over magnesium sulfate, filtered, concentrated and crystallized from hexane to obtain 1.97g (94%) of 5-tetrahydropyranyloxy-4-(4-nitrobenzoyloxy)-2,6,6-trimethyl-2-cyclohepten-1-one as colorless needles crystal. melting point: 133.5 to 135 °C.
IR (KBr, cm⁻¹): 1721, 1678, 1524, 1280, 720.

The product so obtained (1.79g, 4.7mM) was reduced at nitro group as described in Example 19 and purified by silica gel column chromatography using hexane-ethyl acetate (5:1) as an eluent to obtain 1.28g (62%) of 4-(4-aminobenzoyloxy)-5-tetrahydropyranyloxy-2,6,6-trimethyl-2-cyclohepten-1-one as a colorless oily product.

The product so obtained (2.88g, 66mM) was dissolved in acetic anhydride (15ml) and added by concentrated sulfuric acid (two drops) under ice-cooling, then warmed to room temperature and stirred for two hours. The mixture was added by ice-water and stirred, extracted with ethyl acetate, washed with a saturated sodium bicarbonate solution and dried over magnesium sulfate. After filtration, the mixture was concentrated and the residue was crystallized from a mixed solvent of ethyl acetate and hexane to obtain 1.47g (62%) of 4-(4-acetamidobenzoyloxy)-5-hydroxy-2,6,6-trimethyl-2-cyclohepten-1-one (AU237) as light yellow needles. melting point: 133.5 to 135.0 °C.
IR (KBr, cm⁻¹): 3355, 1711, 1678, 1600, 1538, 1273, 1110.
1H-NMR (CDCl₃, ppm): 1,10 (3H, s), 1.15 (3H, s), 1.86 (3H, t, J=2Hz), 2.21 (3H, s), 2.32 (1H, d, J=6Hz), 2.44 (1H, d, J=13Hz), 2.66 (1H, d, J=13Hz), 3,53 (1H, dd, J=9, 6Hz), 5.81 (1H, dm, J=9Hz), 6.41 (1H, m), 7.46 (1H, brs), 7.62 (2H, d, J=9Hz), 8,04 (2H, d, J=9Hz).

### Example 51

Saishin N (1.84g, 10mM) was treated with 2-methoxybenzoyl chloride (2.05g, 25mM) according to the procedures described in Example 17 and fractionated by silica gel column chromatography using hexane-ethyl acetate (5:1) as an eluent. From the first eluted portion 1.57g (49%) of 5-hydroxy-4-(2-methoxybenzoyloxy)-2,6,6-trimethyl-2-cyclohepten-1-one (AU214) was obtained as a colorless oily product.
IR (KBr, cm⁻¹): 3510, 1719, 1675, 1602, 1299, 1250, 1099, 757,
1H-NMR (CDCl₃+D₂O, ppm): 1.10 (3H, s), 1.19 (3H, s), 1.85 (3H, t, J=2Hz), 2.42 (1H, d, J=12Hz), 2.64 (1H, d, J=12Hz), 3,54 (1H, d, J=9Hz), 3.94 (3H, s), 5,75 (1H, dm, J=9Hz), 6.38 (1H, m), 7.02 (1H, brd, J=8Hz), 7.08 (1Hr, brd, J=7Hz) 7.53 (1H, m), 7.86 (1H, dd, J=7,2Hz).

1.53g (47%) of 4-hydroxy-5-(2-methoxybenzoyloxy)-2,6,6-trimethyl-2-cyclohepten-1-one (AU213) as a colorless oily product from the successive eluted portion.
IR (KBr, cm⁻¹): 3501, 1718, 1670, 1602, 1302, 1253, 758.
1H-NMR (CDCl₃+D₂O, ppm): 1.06 (3H, s), 1.13 (3H, s), 1.88 (3H, t, J=2Hz), 2.44 (1H, d, J=13Hz), 2.65 (1H, d, J=13Hz), 3.93 (3H, s), 4.66 (1H, dm, J=9Hz), 4.93 (1H, d, J=9Hz), 6.55 (1H, m), 7.01 (1H, brd, J=8Hz), 7.05 (1H, brd, J=7Hz), 7.51 (1H, m), 7.75 (1H, dd, J=7, 2Hz).

### Example 52

Saishin N (2.40g, 13mM) was treated with 2-chloro-4-nitrobenzoyl chloride (3.74g, 17mM) according to the procedures described in Example 17, fractionated by silica gel column chromatography using hexane-ethyl acetate (5:1) as an eluent. The first eluted portion was crystallized from a mixed solvent of ethyl acetate and hexane to obtain 1.45g (30%) of 4-(2-chloro-4-nitrobenzoyloxy)-5-hydroxy-2,6,6-trimethyl-2-cyclohepten-1-one (AU219) as a colorless powder. melting point: 108 to 109.5°C.
IR (KBr, cm⁻¹): 3502, 1738, 1676, 1528, 1351, 1242, 734.
1H-NMR (CDCl₃+D₂O, ppm): 1.13 (3H, s), 1.17 (3H, s), 1.89 (3H, t, J=2Hz), 2.47 (1H, d, J=13Hz), 2.65 (1H, d, J=13Hz), 3.55 (1H, d, J=9Hz), 5.90 (1H, dm, J=9Hz), 6.40 (1H, m), 8.07 (1H, d, J=8Hz), 8.20 (1H, dd, J=8, 2Hz), 8.36 (1H, d, J=2Hz).

The successive eluted portion was crystallized from a mixed solvent of ethyl acetate and hexane to obtain 1.06g (22%) of 5-(2-chloro-4-nitrobenzoyloxy)-4-hydroxy-2,6,6-trimethyl-2-cyclohepten-1-one (AU220) as a colorless powder. melting point: 221 to 222°C.
IR (KBr, cm⁻¹): 3568, 1718, 1675, 1523, 1354, 1273, 735.
1H-NMR (CDCl₃+D₂O, ppm): 1.14 (3H, s), 1.15 (3H, s), 1.90 (3H, t, J=2Hz), 2.50 (1H, d, J=13Hz), 2.64 (1H, d, J=13Hz), 4.64 (1H, dm, J=9Hz), 5.02 (1H, d, J=9Hz), 6.51 (1H, m), 8.00 (1H, d, J=8Hz), 8.19 (1H, dd, J=8, 2Hz), 8.36 (1H, d, J=2Hz).

### Example 53

Saishin N (3.68g, 20mM) was treated with hydrochloride of 4-dimethylaminobenzoylchloride according to the procedures described in Example 17, purified by silica gel column chromatography using hexane-ethyl acetate (5:1) as an eluent and crystallized from a mixed solvent of ethyl acetate and hexane to obtain 0.67g (10%) of 4-(4-dimethylaminobenzoyloxy)-5-hydroxy-2,6,6-trimethyl-2-cyclohepten-1-one (AU236) as colorless needles. melting point: 162 to 163 °C.
IR (KBr, cm⁻¹): 3517, 1673, 1616, 1286, 1190, 1116, 828, 770.
1H-NMR (CDCl₃, ppm): 1.12 (3H, s), 1.16 (3H, s), 1.87 (3H, t, J=2Hz), 2.39 (1H, brd), 2.44 (1H, d, J=13Hz), 2.67 (1H, d, J=13Hz), 3.07 (6H, s), 3.51 (1H, dd, J=9,5Hz), 5,79 (1H, dm, J=9Hz), 6.44 (1H, m), 6.67 (2H, d, J=9Hz), 7.95 (2H, d, J=9Hz).

### Example 54

Saishin N (5g, 27mM) was treated with 4-nitrocinnamoylchloride according to the procedures described in Example 17 and crystallized from a mixed solvent of ethyl acetate and hexane to obtain 7.50g (77%) of 5-hydroxy-4-(4-nitrocinnamoyloxy)-2,6,6-trimethyl-2-cyclohepten-1-one (AU150) as colorless needles. melting point: 172 to 173 °C.
IR (KBr, cm⁻¹): 3529, 1709, 1669, 1516, 1345, 1178, 848.
1H-NMR (CDCl₃, ppm): 1.11 (3H, s), 1.16 (3H, s), 1.88 (3H, t, J=2Hz), 2.16 (1H, brd. J=5Hz; disappeared by the addition of heavy water), 2.45 (1H, d, J=13Hz), 2.65 (1H, d, J=13Hz), 3.48 (1H, dd, J=9, 5Hz; d by the addition of heavy water, J=9Hz), 5.75 (1H, dm, J=9Hz), 6.36 (1H, m), 6.64 (1H, d, J=16Hz), 7.71 (2H, d, J=9Hz), 7.82 (1H, d, J=16Hz), 8.28 (2H, d, J=9Hz).

### Example 55

The product of Example 54 (6.5g, 18.1mM) was reduced at nitro group as described in Example 19 and fractionated by silica gel column chromatography using hexane-ethyl acetate (2:1) as an eluent. From the first eluted portion 2.55g (43%) of 4-(4-aminocinnamoyloxy)-5-hydroxy-2,6,6-trimethyl-2-cyclohepten-1-one (AU401) was obtained as a yellow glassy product. melting point: 72 to 74°C.
IR (KBr, cm⁻¹): 3452, 3365, 3234, 1699, 1675, 1596, 1518, 1156.
1H-NMR (CDCl₃, ppm): 1.09 (3H, s), 1.15 (3H, s), 1.85 (3H, t, J=2Hz), 2.34 (1H, brd, J=4Hz; disappeared by the addition of heavy water), 2.42 (1H, d, J=12Hz), 2.64 (1H, d, J=12Hz), 3.46 (1H, dd, J=9, 4Hz; d by the addition of heavy water, J=9Hz), 5.69 (1H, dm, J=9Hz), 6.29(1H, d, J=16Hz), 6.37 (1H, m), 6.66 (1H, d, J=9Hz), 7.37 (1H, d, J=9Hz), 7.69 (1H, d, J=16Hz).

From the successive eluted portion 2.55g (43%) of 5-(4-aminocinnamoyloxy)-4-hydroxy-2,6,6-trimethyl-2-cyclohepten-1-one (AU402) as a yellow glassy product. melting point: 49 to 50°C.
IR (KBr, cm⁻¹):3459, 3365, 3233, 1699, 1669, 1623, 1597, 1518, 1159.
1H=NMR (CDCl₃, ppm): 1.06 (3H, s), 1.13 (3H, s), 1.87 (3H, t, J=2Hz), 2.43 (1H, d, J=13Hz), 2.61 (1H, d, J=13Hz), 4.04 (1H, brd, J=4Hz; disappeared by the addition of heavy water), 4.56 (1H, dm, J=9Hz), 4.79 (1H, d, J=9Hz), 6.29 (1H, d, J=16Hz), 6.54 (1H, m), 6.66 (1h, d, J=8Hz), 7.37 (1H, d, J=8Hz), 7.66 (1H, d, J=16Hz).

### Example 56

Saishin N (5.0g, 27mM) was treated with 2-furoyl chloride according to the procedures described in Example 17 and fractionated by silica gel column chromatography using benzene-ethyl acetate (25:1) as an eluent. From the first eluted portion 1.36g (18%) of 4-(2-furoyloxy)-5-hydroxy-2,6,6-trimethyl-2-cyclohepten-1-one (AU403) was obtained as a colorless oily product.
IR (KBr, cm⁻¹): 3500, 1732, 1715, 1682, 1668, 1471.
1H-NMR (CDCl₃, ppm): 1.10 (3H, s), 1.16 (3H, s), 1.86 (3H, t, J=2Hz), 2.29 (1H, d. J=5Hz; disappeared by the addition of heavy water), 2.44 (1H, d, J=13Hz), 2.65 (1H, d, J=13Hz), 3.53 (1H, dd, J=9, 5Hz; d by the addition of heavy water, J=9Hz), 5.80 (1H, dm, J=9Hz), 6.41 (1H, m), 6.57 (1H, dd, J=3.2Hz), 7.30 (1H, dd, J=3, 1Hz), 7.62 (1H, dd, J=2, 1Hz).

The successive eluted portion was crystallized from a mixed solvent of ethyl acetate and hexane to obtain 1.36g (18%) of 5-(2-furoyloxy)-4-hydroxy-2,6,6-trimethyl-2-cyclohepten-1-one (AU404) as colorless needles, melting point: 102.5 to 103.5 °C.
IR (KBr, cm⁻¹): 3508, 3118, 1712, 1674, 1474, 1306, 1186, 1129.
1H-NMR (CDCl₃, ppm): 1.09 (3H, s), 1.16 (3H, s), 1.89 (3H, t, J=2Hz), 2.31 (1H, d, J=7Hz; disappeared by the addition of heavy water), 2.46 (1H, d, J=13Hz), 2.64 (1H, d, J=13Hz), 4.63 (1H, m; dm by the addition of heavy water), 4.88 (1H, d, J=9Hz), 6.52 (1H, m), 6.55 (1H, m), 7,25 (1H, m), 7.63 (1H, m).

### Example 57

DCC (6.81g, 33mM) and 4-dimethylaminopyridine (50mg) were added to a solution of Saishin N(5.53g, 30mM) in methylene chloride (50ml). The mixture was then added by 4-nitrobenzoic acid (5.51g, 33mM) with stirring at 0 °C, stirred for four hours and filtered to have a filtrate. The residue was extracted with hot ethyl acetate (200ml) at 40 °C and was combined with the above filtrate which were concentrated and crystallized from ethyl acetate to obtain 6.67g (66%) of 5-hydroxy-4-(4-nitrobenzoyloxy)-2,6,6-trimethyl-2-cyclohepten-1-one (AU212) as obtained in Example 44.

### Example 58

To a solution of Saishin N (5.53g, 30mM) in methylene chloride (30ml), 4-aminobenzoic acid (5.35g, 39mM), DCC(8.05g, 39mM) and 4-dimethylaminopyridine (0.15g) were added and stirred for 10 hours at 0 °C. The mixture was then filtered and washed with ethyl acetate. The filtrate and wash liquid were combined and concentrated. The residue was purified by silica gel column chromatography using hexane-ethyl acetate (2:1) as an eluent and crystallized from ethyl acetate to obtain 5.52g (61%) of 4-(4-aminobenzoyloxy)-5-hydroxy-2,6,6-trimethyl-2-cyclohepten-1-one (AU215) as obtained in Example 45.

### Example 59

Saishin N (9.21g, 50mM) was treated with 3,4-diaminobenzoic acid (12.93g, 85mM) according to the procedures described in Example 20, purified by silica gel column chromatography using hexane-ethyl acetate (1:1) as an eluent and crystallized from a mixed solution of ethyl acetate and hexane to obtain 6.21g (41%) of 4-(3,4-diaminobenzoyloxy)-5-hydroxy-2,6,6-trimethyl-2-cyclohepten-1-one (AU240) as light orange crystals. melting point: 136 to 137°C.
IR (KBr, cm⁻¹): 3475, 3354, 3272, 1690, 1668, 1314, 1280, 1218.
1H-NMR (CDCl₃, ppm): 1.10 (3H, s), 1.14 (3H, s), 1185 (3H, t, J=2Hz), 2.42 (1H, d, J=13Hz), 2.65 (1H, d, J=13Hz), 3.50 (1H, d, J=9Hz), 5.78 (1H, dm, J=9Hz), 6.40 (1H, m), 6.69 (1H, d, J=8Hz), 7.43 (1H, d, J=2Hz), 7.51 (1H, dd, J=8, 2Hz).

### Example 60

Saishin N (3.68g, 20mM) was treated with 2,4-dinitrobenzoic acid (6.79g, 32mM) according to the procedures described in Example 20, reduced at nitro groups according to the procedures described in Example 19 and fractionated by silica gel column chromatography using hexane-ethyl acetate (3:2) as an eluent. The first eluted portion was crystallized from a mixed solution of ethyl acetate and hexane to obtain 1.65g (26%) of 4-(2,4-diaminobenzoyloxy)-5-hydroxy-2,6,6-trimethyl-2-cyclohepten-1-one (AU242) as a brown powder. melting point: 154 to 156 °C.
IR (KBr, cm⁻¹): 3467, 3378, 1668, 1619, 1257.
1H-NMR (CDCl₃, ppm): 1.10 (3H, s), 1.15 (3H, s), 1.86 (3H, t, J=2Hz), 2.40 (1H, brs; disappeared by the addition of heavy water), 2.43 (1H, d, J=12Hz), 2.65 (1H, d, J=12Hz), 3.52 (1H, dd, J=9, 2Hz; d by the addition of heavy water, J=9Hz), 3,99 (2H, br; disappeared by the addition of heavy water), 5.72 (2H, br; disappeared by the addition of heavy water). 5,75 (1H, dm, J=9Hz), 5.87 (1H, d, J=2Hz), 6.00 (1H, dd, J=9, 2Hz), 6.40 (1H, m), 7.71 (1H, d, J=9Hz).

The successive eluted portion was crystallized from ethyl acetate to obtain 1.63g (26%) of 5-(2,4-diaminobenzoyloxy)-4-hydroxy-2,6,6-trimethyl-2-cyclohepten-1-one (AU241) as yellow needles. melting point: 185 to 186.5 °C.
IR (KBr, cm⁻¹): 3481, 3363, 3300, 1672, 1661, 1625, 1251.
1H-NMR (CDCl₃, ppm): 1.07 (3H, s), 1.18 (3H, s), 1.88 (3H, t, J=2Hz), 2.44 (1H, d, J=13Hz), 2.55 (1H, d, J=6Hz), 2.63 (1H, d, J=13Hz), 3.97 (2H, br), 4.62 (1H, m), 4.84 (1H, d, J=9Hz), 5.71 (2H, br), 5.87 (1H, d, J=2Hz), 6.00 (1H, dd, J=9, 2Hz), 6.57 (1H, m), 7.71 (1H, d, J=9Hz).

### Example 61

Saishin N (5.0g, 27.1mM) was treated with 3-methoxy-4-tetrahydropyranyloxycinnamic acid (9.82g, 35.3mM) according to the procedures described in Example 20, fractionated by reversed phase chromatography (chromatorex ODS: Fuji Divison) using 50 to 70% methanol as an eluent and further by silica gel column chromatograpy using hexane-ethyl acetate (4:1) as an eluent. From the first eluted portion 4.10g (34%) of 5-hydroxy-4-(3-methoxy-4-tetrahydropyranyloxy cinnamoyloxy)-2,6,6-trimethyl-2-cyclohepten-1-one was obtained as a colorless glassy product. 4.10g (34%) of 4-hydroxy-5-(3-methoxy-4-tetrahydropyranyloxycinnamoyloxy)-2,6,6-trimethyl-2-cyclohepten-1-one as a colorless glassy product was obtained from the successive eluted portion.

The first eluted portion (2.0g, 4.5mM) was dissolved in tetrahydroduran (20ml) and added by 1N hydrochloric acid (10ml) under ice-cooling and stirring and stirred for three hours. Then the mixture was diluted with ethyl acetate, washed with water, dried, filtered and concentrated. The residue was purified by silica gel column chromatography using hexane-ethyl acetate (4:1) as an eluent and crystallized from a mixed solvent of ethyl acetate and hexane to obtain 1.21g, (74%) of 5-hydroxy-4-(4-hydroxy-3-methoxycinnamoyloxy)-2,6,6-trimethyl-2-cyclohepten-1-one (AU416) as colorless needles. melting point: 141.5 to 142.5 °C.
IR (KBr, cm⁻¹): 3482, 3283, 1730, 1719, 1650, 1631, 1595, 1521, 1155.
1H-NMR (CDCl₃, ppm): 1.10 (3H, s), 1.15 (3H, s), 1.86 (3H, t, J=2Hz), 2.26 (1H, d, J=5Hz; disappeared by the addition of heavy water), 2.43 (1H, d, J=12Hz), 2.65 (1H, d, J=12Hz), 3.47 (1H, dd, J=9, 5Hz; d by the addition of heavy water, J=9Hz), 3.94 (3H, s), 5.71 (1H, dm, J=9Hz), 5.91 (1H, s; disappeared by the addition of heavy water), 6.36 (1H, d, J=16Hz), 6.37 (1H, t, J=2Hz), 6.94 (1H, d, J=8Hz), 7.05 (1H, d, J=2Hz), 7.10 (1H, dd, J=8, 2Hz), 7.47 (1H, t, J=16Hz).

The second eluted component (3.4g, 7.6mM) was treated in the same manner as above to obtain 2.19g (79%) of 4-hydroxy-5-(4-hydroxy-3-methoxycinnamoyloxy)-2,6,6-trimethyl-2-cyclohepten-1-one (AU414) as colorless needles. melting point: 125 to 127 °C.
IR (KBr, cm⁻¹): 3500-3200, 1737, 1710, 1668, 1631, 1602, 1514, 1281, 1159.
1H-NMR (CDCl₃, ppm): 1.07 (3H, s), 1.14 (3H, s), 1.88 (3H, t, J=2Hz), 2.35 (1H, d, J=7Hz; disappeared by the addition of heavy water), 2.44 (1H, d, J=13Hz), 2.62 (1H, d, J=13Hz), 3.95 (3H, s), 4.57 (1H, m), 4.81 (1H, d, J=9Hz), 5.90 (1H, s; disappeared by the addition of heavy water), 6.35 (1H, d, J=16Hz), 6.54 (1H, m, J=2Hz), 6.93 (1H, d, J=8Hz), 7.05 (1H, d, J=2Hz), 7.11 (1H, dd, J=8, 2Hz), 7.68 (1H, d, J=16Hz).

### Example 62

To a mixed solution of Saishin N (1.84g, 10mM), methylene chloride (10ml) and pyridine (1.12ml), 1-methyl-2-pyrrolecarboxylic acid (1.25g, 12mM) and 2-chloro-1,3-dimethylimidazolinium chloride (2.03g, 12mM) were added, and stirred at room temperature overnight. The reaction mixture was filtered and the residue obtained by the concentration of the filtrate was purified by silica gel column chromatography using a mixed liquid of benzene-ethyl acetate as an eluent and crystallized from a mixed solvent of ethyl acetate and hexane to obtain 1.97g (67.7%) of 4-(1-methyl-2-pyrrolylcarbonyloxy)-5-hydroxy-2,6,6-trimethyl-2-cyclohepten-1-one (AU501) as glassy crystals. melting point: 141.0 to 141.5 °C.
IR (KBr, cm⁻¹):3406, 1684, 1656, 1115, 740.
1H-NMR (CDCl₃, ppm): 1.10 (3H, s), 1.15 (3H, s), 1.86 (3H, t, J=2Hz), 2.34 (1H, d, J=4Hz), 2.43 (1H, d, J=12Hz), 2.64 (1H, d, J=12Hz), 3.51 (1H, dd, J=9, 5Hz), 3.96 (3H, s), 5.74 (1H, dm, J=9Hz), 6.15 (1H, dd, J=4, 2Hz), 6.38 (1H, m), 6.86 (1H, t, J=2Hz), 7.02 (1H, dd, J=4, 2Hz).

### Example 63

The crude product obtained by the condensation of Saishin N (1.84g, 10mM) and 5-methyl-4-imidazolylcarboxylic acid (1.51g, 12mM) according to the procedures described in Example 20 was stirred overnight in a mixed liquid (40ml) of equal amount of acetic acid-tetrahydrofuran water, extracted with ethyl acetate, washed with a saturated sodium bicarbonate solution and with brine, dried over magnesium sulfate, filtered and concentrated. The residue was fractionated by silica gel column chromatography using hexane-ethyl acetate (3:1) as an eluent. The first eluted portion was crystallized from a mixed solvent of ethyl acetate and hexane to obtain 0.81g (28%) of 5-hydroxy-4-(5-methyl-4-imidazolylcarbonyloxy)-2,6,6-trimethyl-2-cyclohepten-1-one (AU503) as a colorless powder. melting point: 99 to 101°C.
IR (KBr, cm⁻¹) : 3600-2500, 1709, 1673, 1100.
1H-NMR (CDCl₃, ppm) 1.13 (3H, s), 1.16 (3H, s), 1.83 (3H, t, J=2Hz), 2.43 (1H, d, J=12Hz), 2.64 (1H, d, J=12Hz), 3.52 (1H, d, J=9Hz), 5.72 (1H, brd), 6.34 (1H, m), 7.50 (1H, s).

The successive eluted portion was crystallized from a mixed solvent of ethyl acetate and hexane to obtain 0.32g (11%) of 4-hydroxy-5-(5-methyl-4-imidazolylcarbonyloxy)-2,6,6-trimethyl-2-cyclohepten-1-one (AU504) as a colorless powder. melting point: 201 to 202 °C.
IR (KBr, cm⁻¹): 3448, 3100-2400, 1708, 1668.
1H-NMR (CDCl₃, ppm): 1.10 (3H, s), 1.15 (3H, s), 1.88 (3H, t, J=2Hz), 2.54 (1H, d, J=13Hz), 2.56 (3H, s), 2.62 (1H, d, J=13Hz), 4.10 (1H, dm, J=9Hz), 5.12 (1H, d, J=9Hz), 6.47 (1H, m), 7.65 (1H, s).

### Example 64

Saishin N (3.68g, 20mM) and 4-tetrahydropyranyloxybenzoic acid (7.0g, 30mM) were condensed according to the procedures described in Example 20 to obtain 4.93g (63.5%) of 5-hydroxy-4-(4-tetrahydropyranyloxybenzoyloxy)-2,6,6-trimethyl-2-cyclohepten-1-one. The product so obtained (2.0g, 5mM) was hydrolyzed according to the procedures described in Example 61 and crystallized from a mixed solvent of ethyl acetate and hexane to obtain 0.3g (20%) of 5-hydroxy-4-(4-hydroxybenzoyloxy)-2,6,6-trimethyl-2-cyclohepten-1-one (AU505) as a colorless powder. melting point: 123 to 125 °C.
IR (KBr, cm⁻¹): 3405, 1712, 1656, 1611, 1267, 1099.
1H-NMR (DMSO-d₆, ppm): 0.96 (3H, s), 1.00 (3H, s), 1.77 (3H, brs), 2.32 (1H, d, J=13Hz), 2.68 (1H, d, J=13Hz), 3.31 (1H, dd, J=9, 3Hz), 5.46 (1H, d, J=6Hz), 5.72 (1H, dm, J=9Hz), 6.48 (1H, m), 6.86 (2H, d, J=9Hz), 7.92 (2H, d, J=9Hz), 10.3 (1H, brs).

### Example 65

Saishin N (1.84g, 10mM) and 4-tetrahydropyranyloxycinnamic acid (2.98g, 12mM) were treated as described in Example 61 and crystallised from a mixed solvent of ethyl acetate and hexane, 0.29g (18%) of 5-hydroxy-4-(4-hydroxycinnamoyloxy)-2,6,6-trimethyl-2-cyclohepten-1-one (AU506) as colorless crystals was obtained. melting point: 176 to 177 °C.
IR (KBr, cm⁻¹): 3386, 3100-2400, 1713, 1654, 1608, 1516, 1279, 1158.
1H-NMR (CDCl₃, ppm): 1.10 (3H, s), 1.15 (3H, s), 1.87 (3H, t, J=2Hz), 2.43 (1H, d, J=12Hz), 2.65 (1H, d, J=12Hz), 3.48 (1H, d, J=9Hz), 5.72 (1H, dm, 9Hz), 6.33 (1H, d. J=16Hz), 6.39 (1H, m), 6.86 (2H, d, J=9Hz), 7.42 (2H, d, J=9Hz), 7.71 (1H, d, J=16Hz).

### Example 66

4-acetoxybenzoic acid (2.16g, 12mM), 2-chloro-1-methylpyridinium iodide (3.07g, 12mM) and triethyamine (3.37ml, 24mM) were added to a solution of Saishin N (1.84g, 10mM) in methylene chloride (20ml). The mixture was stirred for seven days at room temperature and added by water, extracted with ethyl acetate, washed successively with dilute hydrochloric acid, brine, a saturated sodium bicarbonate solution and brine and dried over magnesium sulfate After filtration, the filtrate was concentrated, purified by silica gel column chromatography using hexane-ethyl acetate (5:1) as an eluent and crystallized from a mixed solvent of hexane-ethyl acetate to obtain 0.85g (25%) of 4-(4-acetoxybenzoyloxy)-5-hydroxy-2,6,6-trimethyl-2-cyclohepten-1-one (AU217) as colorless needles. melting point: 132 to 133 °C.
IR (KBr, cm⁻¹): 3514, 1744, 1731, 1675, 1262, 1228, 1102.
1H-NMR (CECl₃+D₂O, ppm): 1.13 (3H, s), 1.17(3H, s), 1.89 (3H, t, J=2Hz), 2.34 (3H, s), 2.46 (1H, d, J=13Hz), 2.67 (1H, d, J=13Hz), 3.54 (1H, d, J=9Hz), 5.86 (1H, dm, J=9Hz), 6.42 (1H, m), 7.23 (2H, d, J=9Hz), 8.13 (2H, d, J=9Hz).

### Example 67

Manganese dioxide (5.5g) was added to a solution of 5-benzoyloxy-4-hydroxy-2,6,6-trimethyl-2-cyclohepten-1-one (0.9g, 2.4mM) obtained in Example 7 in methylene chloride (20ml). The mixture was stirred for two days at room temperature, filtered and concentrated. The residue was purified by silica gel column chromatography using hexane-ethyl acetate (10:1) as an eluent to obtain 0.47g (6M%) of 5-benzoyloxy-2,6,6-trimethyl-2-cyclohepten-1,4-dione (AU225) as colorless plates. melting point: 68 to 68.5 °C.
IR (KBr, cm⁻¹): 1720, 1687, 1274, 1110, 716.
1H-NMR (CDCl₃, ppm): 1.27 (6H, s), 2.01 (3H, t, J=2Hz), 2.69 (1H, d, J=13Hz), 2.71 (1H, d, J=13Hz), 5.10 (1H, s), 6.46 (1H, m), 7.47 (2H, m), 7.60 (1H, m), 8.01 (2H, m).

### Example 68

Pyridinium chlorochromate (1.72g, 8mM) and cerite (1.5g) were added into methylene chloride (40ml). A solution of 4-benzoyloxy-5-hydroxy-2,6,6-trimethyl-2-cyclohepten-1-one (0.577g, 2mM) obtained in Example 30 in methylene chloride (5ml) was added dropwise to the mixture with stirring at room temperature and stirred for eight hours. The reaction mixture was added by ether (50ml) and magnesium sulfate, left to stand for 30 minutes, filtered and concentrated. The residue was extracted with ether, separated by thin-layer chromatography (Merc k 5744) using hexane-ethyl acetate (3:1) as a developing solvent and crystallized from a mixed solution of ethyl acetate and hexane to obtain 0.30g (69%) of 4-benzoyloxy-2,6,6-trimethyl-2-cyclohepten-1,5-dione (AU226) as colorless plates. melting point: 91 to 92°C.
IR (KBr, cm⁻¹): 1736, 1721, 1670, 1274, 1122.
1H-NMR (CDCl₃, ppm): 1.17 (3H, s), 1.36 (3H, s), 1.94 (3H, d, J=2Hz), 2.74 (1H, d, J=14Hz), 2.27 (1H, d, J=14Hz), 6.63 (2H, m), 7.47 (2H, m), 7.62 (1H, m), 8.13 (2H, m).

### Example 69

N,N-dimethylhydrazine (15.9ml, 210mM) and acetic acid (3ml) were added to a solution of 5-hydroxy-4-(4-methoxybenzyloxy)-2,6,6-trimethyl-2-cyclohepten-1-one (21.3g, 70mM) obtained in Example 1 in ethanol (60ml). The mixture was heated under reflux for five hours, concentrated and purified by silica gel column chromatography using hexane-ethyl acetate (10:1) as an eluent to obtain 13.35g (55%) of dimethylhydrazone as an orange oily product.

55% sodium hydride (0.79g, 18mM) and methyl iodide (1.43ml, 23mM) were added to a solution of the above dimethylhydrazone (5.20g, 15mM) in tetrahydrofuran (30ml) and stirred for six hours at room temperature. The reaction solution was then poured onto ice-water, extracted with ethyl acetate, washed with brine, dried over magnesium sulfate, filtered and concentrated. The residue was fractionated by silica gel column chromatography using hexane-ethyl acetate (20:1 to 5:1) as an eluent to obtain two kinds of stereoisomers, 5-methoxy-4-(4-methoxybenzyloxy)-2,6,6-trimethyl-2-cyclohepten-1-one dimethylhydrazone, 3.14g (58%) and 0.78g (14.4%), respectively.
1H-NMR (CDCl₃, ppm): 0.99 (3H, s), 1.02 (3H, s), 1.92 (3H, t, J=2Hz), 2.05 (1H, d, J=14Hz), 2.41 (3H, s), 2.92 (1H, d, J=8Hz), 3.00(1H, d, J=14Hz), 3.55 (3H, s), 3.80 (3H, s), 4.08 (1H, m), 4.59 (1H, d, J=11Hz), 4.68 (1H, d, J=11Hz), 6.01 (1H, m), 6.87 (2H, d, J=9Hz), 7.31 (2H, d, J=9Hz).
1H-NMR (CDCl₃, ppm): 0.98 (3H, s), 1.10 (3H, s), 1.94 (3H, t, J=2Hz), 2.11 (1H, d, J=13Hz), 2.21 (1H, d, J=13Hz), 2.47 (3H, s), 2.90 (1H, d, J=8Hz), 3.56 (3H, s), 3.80 (3H, s), 3.96 (1H, m), 4.56 (1H, d, J=11Hz), 4.67 (1H, d, J=11Hz), 5.56 (1H, m), 6.87 (2H, d, J=9Hz), 7.29 (2H, d, J=9Hz).

The mixture of the above isomers (3.14g, 8.7mM) was dissolved in a mixed solution of ethanol (19ml) and water (1ml), and was added by methyl iodide (0.8ml, 13mM), heated under reflux for eight hours and concentrated. The residue was purified by silica gel column chromatography using hexane-ethyl acetate (20:1) as an eluent to obtain 1.14g (41%) of 5-methoxy-4-(4-methoxybenzyloxy)-2,6,6-trimethyl-2-cyclohepten-1-one (AU221) as a yellow oily product.
IR (KBr, cm⁻¹): 1673, 1514, 1249, 1113.
1H-NMR (CDCl₃, ppm): 0.99 (3H, s), 1.06 (3H, s), 1.82 (3H, t, J=2Hz), 2.29 (1H, d, J=13Hz), 2.41 (1H, d, J=13Hz), 2.88 (1H, d, J=8Hz), 3.57 (3H, s), 3.82 (3H, s), 4.17 (1H, dm, J=8Hz), 4.64 (1H, d, J=11Hz), 4.80 (1H, d, J=11Hz), 6.56 (1H, m), 6.90 (2H, d, J=9Hz), 7.33 (2H, d, J=9Hz).

### Example 70

5-hydroxy-4-(4-methoxybenzyloxy)-2,6,6-trimethyl-2-cyclohepten-1-one dimethylhydrazone (13.35g, 38mM) obtained in Example 69 as an intermediate was methylated as described in Example 69 and then dissolved in a mixed solution of 6N hydrochloric acid (40ml) and tetrahydrofuran (20ml), warmed to 50° C for six hours, then extracted with ethyl acetate, washed with a saturated sodium bicarbonate solution and with brine, dried over magnesium sulfate and filtered. The residue was purified by silica gel column chromatography using hexane-ethyl acetate (10:1) as an eluent and crystallized from a mixed solution of ethyl acetate and hexane to obtain 4.26g (56%) of 4-hydroxy-5-methoxy-2,6,6-trimethyl-2-cyclohepten-1-one (AU222) as colorless plates. melting point: 71 to 72 °C.
IR (KBr, cm⁻¹): 3461, 1669, 1103, 1082.
1H-NMR (CDCl₃, ppm): 0.99 (3H, s), 1.12 (3H, s), 1,83 (3H, t, J=2Hz), 2.27 (1H, d, J=12Hz), 2.50 (1H, d, J=12Hz), 2.78 (1H, d, J=9Hz), 2.96 (1H, brs), 3.57 (3H, s), 4.41 (1H, dm, J=9Hz), 6.57 (1H, m).

### Example 71

5-methoxy-4-(4-methoxybenzyloxy)-2,6,6-trimethyl-2-cyclohepten-1-one (2.5g, 7.8mM) obtained in Example 69 was treated with DDQ according to the procedures described in Example 7 to obtain 1.21g (78%) of 4-hydroxy-5-methoxy-2-cyclohepten-1-one (AU222) as in Example 70.

### Example 72

The product of Example 71 (1.39g, 7.0mM) was treated with 4-nitrobenzoyl chloride as described in Example 17, purified with silica gel column chromatography using a benzene-ethyl acetate (20:1) as an eluent and crystallized from a mixed solvent of benzene and hexane to obtain 2.11g (87%) of 5-methoxy-4-(4-nitrobenzoyloxy)-2,6,6-trimethyl-2-cyclohepten-1-one (AU238) as light yellow crystals. melting point: 136.5 to 138.0 °C.
IR (KBr, cm⁻¹): 1725, 1682, 1527, 1285, 1124, 1104, 717.
1H-NMR (CDCl₃, ppm): 1.11 (3H, s), 1.13 (3H, s), 1.90 (3H, t, J=2Hz), 2.41 (1H, d, J=13Hz), 2.65 (1H, d, J=13Hz), 3.02 (1H, d, J=9Hz), 3.42 (3H, s), 6.06 (1H, dm, J=9Hz), 6.49 (1H, m), 8.30 (2H, d, J=9Hz), 8.34 (2H, d, J=9Hz).

### Example 73

The product of Example 72 (1.68g, 4.8mM) was reduced at nitro group as described in Example 19 and crystallized from a mixed solvent of ethyl acetate and hexane to obtain 0.79g (51.1%) of 4-(4-aminobenzoyloxy)-5-methoxy-2,6,6-trimethyl-2-cyclohepten-1-one (AU239) as colorless plates. melting point: 159 to 161°C.
IR (KBr, cm⁻¹): 3476, 3377, 1687, 1602, 1276, 1170, 1114.
1H-NMR (CDCl₃, ppm): 1.09 (3H, s), 1.11 (3H, s), 1.87 (3H, t, J=2Hz), 2.38 (1H, d, J=13Hz), 2.66 (1H, d, J=13Hz), 2.98 (1H, d, J=9Hz), 3.45 (3H, s), 4.11 (2H, br), 5.99 (1H, dm, J=9Hz), 6.47 (1H, m), 6.67 (2H, d, J=9Hz), 7.93 (2H, d, J=9Hz).

### Example 74

4-hydroxy-5-methoxy-2,6,6-trimethyl-2-cyclohepten-1-one (397mg, 2mM) obtained in Example 71 was benzoylated according to the procedures described in Example 6 and crystallized from hexane to obtain 510mg (84%) of 4-benzoyloxy-5-methoxy-2,6,6-trimethyl-2-cyclohepten-1-one (AU223) as colorless plates. melting point: 97 to 98 °C.
IR (KBr, cm⁻¹): 1724, 1676, 1272, 1117, 712.
1H-NMR (CDCl₃, ppm): 1.10 (3H, s), 1.12 (3H, s), 1.89 (3H, t, J=2Hz), 2.40 (1H, d, J=13Hz), 2.67 (1H, d, J=13Hz), 3.01 (1H, d, J=9Hz), 3.45 (3H, s), 6.04 (1H, dm, J=9Hz), 6.49 (1H, m), 7.48 (2H, m), 7.60 (1H, m), 8.13 (2H, m).

### Example 75

p-Toluenesulfonic acid (0.3g) was added to a solution of Saishin N (2.00g, 11mM) in acetone (10ml), stirred overnight at room temperature, diluted with ethyl acetate, washed with a saturated sodium bicarbonate solution and with brine, dried over magnesium sulfate, filtered and concentrated. The residue was crystallized from hexane to obtain 2.30g (95.7%) of 4,5-0-isopropylidene Saishin N as colorless needles. melting point: 65 to 65.5 °C.
IR (KBr, cm⁻¹): 1664, 1374, 1242, 1091, 877.
1H-NMR (CDCl₃, ppm): 1.06 (3H, s), 1.15 (3H, s), 1.41 (3H, s), 1.43 (3H, s), 1.85 (3H, t, J=2Hz), 2.37 (1H, d, J=12Hz), 2.47 (1H, d, J=12Hz), 3.42 (1H, d, J=9Hz), 4.53 (1H, dm, J=9Hz), 6.57 (1H, t, J=1Hz).

To a solution of the above product (2.24g, 10mM) in tetrahydrofuran (5ml), lithium aluminum hydride (0.3g, 8mM) was added under ice-cooling and stirring and stirred for two hours. Ethyl acetate (5ml) and water (4ml) were added to the mixture, stirred for two hours and then filtered. The filtrate was concentrated to obtain 1.85g (82%) of 4,5-0-isopropylidene-2,6,6-trimethyl-2-cyclohepten-1,4,5-triol as a colorless crystalline material.
IR (KBr, cm⁻¹): 3365, 1450, 1372, 1240, 1069, 1006.
1H-NMR (CDCl₃+D₂O, pm): 1.07 (3H, s), 1.12 (3H, s), 1.37 (3H, s), 1.40 (3H, s), 1.48 (1H, dd, J=13, 2Hz), 1.71 (1H, dd, J=13, 11Hz), 1.79 (3H, brs), 3.23 (1H, d, J=9Hz), 4.39 (1H, dm, J=9Hz), 4.49 (1H, brd, J=11Hz), 5.56 (1H, m).

2N hydrochloric acid (5ml) was added to a mixed solution of the above prodcut (2.33g, 10.3mM) in methanol (5ml) and tetrahydrofuran (5ml) and warmed at 40 C for six hours, diluted with ethyl acetate, washed with a saturated sodium bicarbonate solution and with brine, dried over magnesium sulfate, filtered, concentrated, purified by silica gel column chromatography using benzene-ethyl acetate (3:1) as an eluent and crystallized from ethyl acetate to obtain 0.99g (54%) of 2,6,6-trimethyl-2-cyclohepten-1,4,5-triol (AU103) as colorless needles. melting point: 138 to 139°C.
IR (KBr, cm⁻¹): 3600-3100, 1447, 1435, 1280, 994.
1H-NMR (DMSO-d₆, ppm): 0.95 (3H, s), 0.97 (3H, s), 1.36 (1H, dd, J=13, 2Hz), 1.47 (1H, dd, J=13, 10Hz), 1.66 (3H, brs), 2.78 (1H, dd, J=10, 3Hz; d by the additio of heavy water, J=10Hz), 3.99 (1H, m; brd by the addition of heavy water, J=10Hz), 4.26 (1H, m; brd disappeared by the addition of heavy water, J=10Hz), 4.36 (1H, d, J=3Hz; disappeared by the addition of heavy water), 4.62 (1H, d, J=3Hz; disappeared by the addition of heavy water), 4.68 (1H, d, J=4Hz; disappeared by the addition of heavy water), 5.17 (1H, m).

### Example 76

4-Methoxybenzaldehyde (3.5ml, 28mM) and p-toluenesulfonic acid (0.25g) were added to a solution of Saishin N (5.0g, 27mM) in benzene (60ml) and heated under reflux for an hour, diluted with benzene, washed with a saturated sodium bicarbonate solution, dried over magnesium sulfate, filtered and concentrated to obtain 4,5-0-(4-methoxybenzylidene) Saishin N in a quantitative yield as a partly crystalline product.
IR (KBr, cm⁻¹): 1676, 1243, 1093, 1029.

The above product was reduced by lithium aluminum hydride (1g, 26mM) according to the procedures described in Example 75 and purified by silica gel column chromatography using toluene-ethyl acetate (20:1) as an eluent to obtain 4,5-0-(4-methoxybenzylidene)-2,6,6-trimethyl-2-cyclohepten-1,4,5-triol as a partly crystalline product.
IR (KBr, cm⁻¹):3482, 1613, 1516, 1248, 1089.

The above product was methylated according to the procedures described in Example 69, added by 80% acetic acid (80ml) and stirred overnight. The reaction solution was concentrated, neutralized with a sodium bicarbonate solution extracted with ethyl acetate, washed with brine, dried over magnesium sulfate, filtered and concentrated. The residue was purified by silica gel column chromatography using hexane-ethyl acetate (20:1) as an eluent to obtain 2.98g (55%) of 1-methoxy-2,6,6-trimethyl-2-cyclohepten-4,5-diol (AU110) as a colorless oily material.
IR (KBr, cm⁻¹): 3418, 1103, 1002.
1H-NMR (CDCl₃+CD₃OD, ppm): 1.07 (3H, s), 1.09 (3H, s), 1.51 (1H, dd, J=14, 3Hz), 1.58 (1H, dd, J=14, 9Hz), 1.75 (3H, m), 3.07 (1H, d, J=10Hz), 3.32 (3H, s), 3.84 (1H, brd, J=9Hz), 4.20 (1H, brd, J=10Hz), 5,33 (1H, m).

### Example 77

1-methoxy-2,6,6-trimethyl-2-cyclohepten-4,5-diol (0.3g, 1.3mM) obtained in Example 76 was acetylated by a conventional method to obtain 0.41g (94%) of 4,5-diacetoxy-1-methoxy-2,6,6-trimethyl-2-cycloheptene (AU112) as a colorless oily product.
IR (KBr, cm): 1746, 1372, 1244, 1102, 1028.
1H-NMR (CDCl , ppm): 0.95 (3H, s), 1.19 (3H, s), 1.59 (1H, d, J=13Hz), 1.63 (1H, d, J=13Hz), 1.76 (3H, brt, J=1Hz), 2.03 (3H, s), 2.04 (3H, s), 3.33 (3H, s), 3.94 (1H, brd, J=8Hz), 4.75 (1H, d, J=10Hz), 5.22 (1H, m), 5.58 (1H, dm, J=10Hz).

### Example 78

1-methoxy-2,6,6-trimethyl-2-cyclohepten-4,5-diol (0.35g, 17mM) obtained in Example 76 was methylated according to the procedures described in Example 69 to obtain 0.325g (81.4%) of 1,4,5-trimethoxy-2,6,6-trimethyl-2-cycloheptene (AU113) as a colorless oily product.
IR (KBr, cm⁻¹): 1446, 1385, 1104.
1H-NMR (CDCl₃, ppm): 1.05 (3H, s), 1.07 (3h, s), 1.50 (2H, m), 1.75 (3H, brs), 2.69 (1h, d, J=10Hz), 3.31 (3H, s), 3.43 (3H, s), 3.49 (3H, s), 3.84. (1H, brd, J=10Hz), 3.87 (1H, dm, J=10Hz), 5.30 (1H, m).

### Example 79

To a solution of 1-methoxy-2,6,6-trimethyl-2-cyclohepten-4,5-diol (0.3g, 1.5mM) obtained in Example 76 in tetrahydrofuran (3ml), 55% sodium hydride (0.2g, 4.6mM) was added under ice-cooling and stirring. After stirring for an hour, the mixture was added by benzoyl chloride (0.53ml, 4.55mM) and stirred overnight at room temperature. The reaction mixture was poured onto ice-water, diluted with ethyl acetate, washed with brine, dried over magnesium sulfate, filtered and concentrated. The residue was fractionated by silica gel column chromatography using hexane-ethyl acetate (10:1). From the first eluted portion 0.11g (17.2%) of 4,5-dibenzoyloxy-1-methoxy-2,6,6-trimethyl-2-cycloheptene (AU114) was obtained as colorless crystals.
melting point: 78 to 80 °C.
IR (KBr, cm⁻¹): 1724, 1602, 1451, 1279, 1095, 711.
1H-NMR (CDCl₃, ppm): 1.06 (3H, s), 1.39 (3H, s), 1.70 (1H, dd, J=14, 2Hz), 1.80 (1H, dd, J=14, 10Hz), 1.83 (3H, brs), 3.39 (3H, s), 4.10 (1H, d, J=7Hz), 5.16 (1H, d, J=10Hz), 5.43 (1H, m), 6.00 (1H, m), 7.23 (4H, m), 7.38 (2H, m), 7.84 (4H, m).

86mg (19%) of 5-benzoyloxy-1-methoxy-2,6,6-trimethyl-2-cyclohepten-4-ol (AU115) was obtained as colorless crystals from the successive eluted portion.
1H-NMR (CDCl₃, ppm): 1.02 (3H, s), 1.30 (3H, s), 1.61 (1H, dd, J=13, 2Hz), 1.72 (1H, dd, J=13, 10Hz), 1.80 (3H, brs), 3.35 (3H, s), 3.91 (1H, d, J=10Hz), 4.49 (1H, brd, J=10Hz), 4.84 (1H, d, J=10Hz), 5.41 (1H, m), 7.46 (2H, m), 7.58 (1H, m), 8.06 (2H, brd, J=9Hz).

### Example 80

1-methoxy-2,6,6-trimethyl-2-cyclohepten-4,5-diol (2.73g, 13.6mM) obtained in Example 76 was benzoylated as described in Example 6 and crystallized from a mixed solution of hexane and ethyl acetate to obtain 1.01g (33%) of 4-benzoyloxy-1-methoxy-2,6,6-trimethyl-2-cyclohepten-5-ol (AU145) as colorless needles. melting point: 98 to 99 °C.
IR (KBr, cm⁻¹): 3517, 1694, 1280, 1095, 720.
1H-NMR (CDCl₃+D₂O, ppm): 1.16 (3H, s), 1.18 (3H, s), 1.61 (1H, dd, J=14. 2Hz), 1.65 (1H, dd, J=14, 8Hz), 1.78 (3H, brs), 3.36 (3H, s), 3.46 (1H, d, J=10Hz), 3.98 (1H, brd, J=8Hz), 5.32 (1H, m), 5.67 (1H, dm, J=10Hz), 7.46 (2H, t, J=7Hz), 7.59 (1H, t, J=7Hz), 8.06 (1H, d, J=7Hz).

### Example 81

4,5-0-(4-methoxybenzylidene)-2,6,6-trimethyl-2-cyclohepten-1,4,5-triol (918mg, 3.0mM) obtained as an intermediate in Example 76 was acetylated by a conventional method to obtain 1.05g (94.7%) of acetate. The acetate (961mg, 2.77mM) was hydrolyzed with 80% acetic acid (5ml), and purified by silica gel column chromatography to obtain 0.615g (97.2%) of 1-acetoxy-2,6,6-trimethyl-2-cyclohepten-4,5-diol (AU111) as a colorless oily product.
IR (KBr, cm⁻¹): 3444, 1738, 1374, 1240.
1H-NMR (CDCl₃, ppm): 1.09 (3H, s), 1.11 (3H, s), 1.49 (1H, dd, J=14, 2Hz), 1.69 (3H, brs), 1.74 (1H, dd, J=14, 10Hz), 2.07 (3H, s), 2.48 (1H, d, J=3Hz; disappeared by the addition of heavy water), 2.29 (1H, d, J=4Hz; disappeared by the addition of heavy water), 3.14 (1H, dd, J=10, 4Hz; d by the addition of heavy water, J=10Hz), 4.30 (1H, brd, J=10Hz), 5.40 (1H, q, J=1Hz), 5.50 (1H, brd, J=10Hz).

### Example 82

To a solution of 4,5-0-(4-methoxybenzylidene)-2,6,6-trimethyl-2-cyclohepten-1,4,5-triol (1.0g, 3.29mM) obtained as an intermediate in Example 76 in tetrahydrofuran (10ml), 55% sodium hydride (0.2g) was added under ice-cooling and stirring. After stirring for 90 minutes, benzyl chloride (0.53ml, 4.6mM) was added, heated under reflux for 19 hours, diluted with water, extracted with ethyl acetate, washed with brine, dried over magnesium sulfate, filtred and concentrated. The residue was purified by silica gel column chromatography using hexaneethyl acetate (40:1) as an eluent to obtain 816mg (63%) of 1-benzylether as an colorless oily product. The product so obtained was hydrolyzed with 80% acetic acid to obtain 266mg (43.2%) of 1-benzyloxy-2,6,6-trimethyl-2-cyclohepten-4,5-diol (AU116) as a colorless oily product.
IR (KBr, cm⁻¹): 3600-3200, 1456, 1094, 745, 697.
1H-NMR (CDCl₃+D₂O, ppm): 1.01 (3H, s), 1.09 (3H, s), 1.59 (1H, dd, J=14, 2Hz), 1.68 (1H, dd, J=14, 10Hz), 1.82 (3H, t, J=2Hz), 3.10 (1H, d, J=10Hz), 4.08 (1H, dm, J=9Hz), 4.21 (1H, brd, J=10Hz), 4.50 (2H, s), 5.34 (1H, m), 7.33 (5H, m).

### Example 83

4,5-0-(4-methoxybenzylidene)-2,6,6-trimethyl-2-cyclohepten-1,4,5-triol (4.98g, 16.4mM) obtained in Example 76 was treated with 55% sodium hydride (0.93g, 21.3mM) and 1-bromo-3-methyl-2-butene (2.5ml, 21.3mM) according to the procedures described in Example 69 and fractionated by silica gel column chromatography using hexane-ethyl acetate (2:1) as an eluent to obtain 1.56g (38%) of 1-(3-methyl-2-buten-1-yloxy)-2,6,6-trimethyl-2-cyclohepten-4,5-diol (AU143) as a colorless oily product.
IR (KBr, cm⁻¹): 3434, 1714, 1450, 1379, 1069.
1H-NMR (CDCl₃+D₂O, ppm): 1.07 (3H, s), 1.09 (3H, s), 1.52 (1H, dd, J=13, 2Hz), 1.63 (1H, dd, J=13, 10Hz), 1.67 (3H, brs), 1.75 (3H, brs), 1.77 (3H, brs), 3.10 (1H, d, J=10Hz), 3.94 (2H, m), 3.99 (1H, brd, J=10Hz), 4.25 (1H, dm, J=9Hz), 5.32 (2H, m).

### Example 84

The product of Example 83 (1.25g, 4.9mM) was benzoylated as described in Example 6 and purified by silica gel column chromatography using benzene-ethyl acetate (10:1) as an eluent to obtain 0.52g (30%) of 4-benzoyloxy-1-(3-methyl-2-buten-1-yloxy)-2,6,6-trimethyl-2-cyclohepten-5-ol (AU144) as a colorless oily product.
IR (KBr, cm⁻¹): 3479, 1732, 1715, 1698, 1451, 1272, 712.
1H-NMR (CDCl₃+D₂O, ppm): 1.15 (3H, s), 1.17 (3H, s), 1.61 (1H, dd, J=13, 2Hz), 1.70 (3H, s), 1.76 (3H, s), 1.79 (3H, t, J=2Hz), 3.46 (1H, d, J=10Hz), 3.98 (2H, m). 4.13 (1H, brd, J=11Hz), 5.33 (2H, m), 5.67 (1H, dm, J=9Hz), 7.43 (2H, m). 7.56 (1H, m), 8.06 (2H, m).

### Example 85

4,5-0-(4-methoxybenzylidene)-2,6,6-trimethyl-2-cyclohepten-1,4,5-triol (10.0g, 32.9mM) obtained as an intermediate in Example 76 was treated with bromoethyl acetate (5.6ml, 42.6mM) according to the procedures described in Example 83 and hydrolyzed with 80% acetic acid to obtain 1.84g (21%) of 1-ethoxycarbonylmethyloxy-2,6,6-trimethyl-2-cyclohepten-4,5-diol (AU146) as a colorless oily product.
1H-NMR (CDCl₃+D₂O, ppm): 1.10 (3H, s), 1.12 (3H, s), 1.29 (3H, t, J=7Hz), 1.63 (2H, m), 1.80 (3H, t, J=2Hz), 3.10 (1H, d, J=10Hz), 4.04 (1H, d, J=16Hz), 4.08 (1H, dm, J=10Hz), 4.22 (2H, q, J=7Hz), 5.36 (1H, m).

### Example 86

The product of Example 85 (1.34g, 3.55mM) was benzoylated as described in example 6 and purified by silica gel column chromatography using benzene-ethyl acetate (10:1) as an eluent to obtain 0.66g (36%) of 5-benzoyloxy-1-ethoxycarbonymethyloxy-2,6,6-trimethyl-2-cyclohepten-4-ol (AU147) as a colorless oily product.
IR (KBr, cm⁻¹): 3524, 1751, 1718, 1275, 1118, 712.
1H-NMR (CDCl₃, ppm): 1.03,(3H, s), 1.30 (3H, s), 1.30 (3H, t, J=7Hz), 1.73 (1H, dd, J=14, 2Hz), 1.79 (1H, d, J=14Hz), 1.82 (1H, dd, J=14, 10Hz), 1.85 (3H, t, J=2Hz), 4.00-4.20 (3H, m), 4.23 (2H, q, J=7Hz), 4.46 (1H, brd, J=10Hz), 4.86 (1H, d, J=10Hz), 5.45 (1H, m), 7.46 (2H, t, J=7Hz), 7.58 (1H, t, J=7Hz), 8.07 (2H, d, J=7Hz).

### Example 87

4,5-0-(4-methoxybenzylidene)-2,6,6-trimethyl-2-cyclohepten-1,4,5-triol (5.0g, 16.4mM) obtained as an intermediate in Example 76 was treated with 4-nitrocinnamoyl chloride according to the procedures described in Example 17, hydrolyzed with 80% acetic acid and crystallized from ethyl acetate to obtain 2.54g (83%) of 1-(4-nitrocinnamoyloxy)-2,6,6-trimethyl-2-cyclohepen-4,5-diol (AU149) as yellow needles. melting point: 152.5 to 154 °C.
IR (KBr, cm⁻¹): 3379, 1711, 1518, 1342, 844.
1H-NMR (CDCl₃, ppm): 1.12 (3H, s), 1.16 (3H, s), 1.59 (1H, dd, J=14, 2Hz), 1.75 (3H, brs), 1.84 (1H, dd, J=14. 10Hz), 3.18 (1H, d, J=10Hz), 4.35 (1H, brd, J=10Hz), 5.46 (1H, m), 5.66 (1H, brd, J=10Hz), 6.57 (1H, d, J=16Hz), 7.69 (2H, d, J=9Hz), 7.73 (1H, d, J=16Hz), 8.26 (2H, d, J=9Hz).

### Example 88

The product of Example 87 (2.44g, 6.75mM) was benzoylated as described in Example 6 and fractionated by silica gel column chromatography using hexane-ethyl acetate (10:1) as an eluent. The first eluted portion was crystallized from a mixed solvent of ethyl acetate and hexane to obtain 1.36g (43%) of 4-benzoyloxy-1-(4-nitrocinnamoyloxy)-2,6,6-trimethyl-2-cyclohepten-5-ol (AU405) as light yellow needles. melting point: 156.5 to 157.5 °C.
IR (KBr, cm⁻¹): 3500, 1732, 1715, 1682, 1668, 1580, 1471, 1296, 763.
1H-NMR (CDCl₃, ppm): 1.17 (3H, s), 1.26 (3H, s), 1.69 (1H, dd, J=14, 2Hz), 1.79 (3H, brs), 1.92 (1H, dd, J=14, 10Hz), 2.19 (1H, d, J=5Hz; disappeared by the addition of heavy water). 3.55 (1H, dd, J=10, 5Hz; d by the addition of heavy water. J=10Hz), 5.46 (1H, m), 5.78 (2H, m), 6.59 (1H, d, J=16Hz), 7.58 (5H, m), 7.75 (1H, d, J=16Hz), 8.09 (2H, m), 8.28 (2H, m).

The successive eluted portion was crystallized from a mixed solvent of ethyl acetate and hexane to obtain 0.35g (11%) of 5-benzoyloxy-1-(4-nitrocinnamoyloxy)-2,6,6-trimethyl-2-cyclohepten-4-ol (AU406) as light yellow needles. melting point: 179 to 181 °C.
IR (KBr, cm⁻¹): 3517, 1707, 1639, 1518, 1340, 1280, 716.
1H-NMR CDCl₃, ppm): 1.04 (3H, s), 1.36 (3H, s), 1.69 (1H, dd, J=13, 2Hz), 1.81 (3H, brs), 1.99 (1H, d, J=13, 10Hz), 2.02 (1H, d, J=6Hz; disappeared by the addition of heavy water), 4.61 (1H, m; brd by the addition of heavy water, J=9Hz), 4.99 (1H, d, J=10Hz), 5.56 (1H, m), 5.72 (1H, brd, J=10Hz), 6.60 (1H, d, J=16Hz), 7.61 (5H, m), 7.77 (1H, d, J=16Hz), 8.10 (2H, d, J=8Hz), 8.27 (2H, d, J=8Hz).

### Example 89

4,5-0-(4-methoxybenzylidene)-2,6,6-trimethyl-2-cyclohepten-1,4,5-triol (2.42g, 8.0mM) obtained as an intermediate in Example 76 was benzoylated as described in Example 6 to obtain a crude benzoate.
1H-NMR (CDCl₃, ppm): 1.17 (3H, s), 1.28 (3H, s), 1.68 (1H, dd, J=14, 1Hz), 1.82 (3H, brs), 1.92 (1H, dd, J=14, 10Hz), 3.56 (1H, d, J=9Hz), 3.82 (3H, s), 4.65 (1H, dm, J=9Hz), 5.82 (2H, m), 5.99 (1H, s), 6.92 (2H, d, J=9Hz), 7.42 (2H, d, J=9Hz), 7.46 (2H, m), 7.59 (1H, m), 8.07 (2H, m).

The above crude benzoate was hydrolyzed with 80% acetic acid (13ml), purified by silica gel column chrmatography using hexane-ethyl acetate (2:1) as an eluent and crystallized from a mixed solvent of ethyl acetate and hexane to obtain 1.46g (63%) of 1-benzoyloxy-2,6,6-trimethyl-2-cyclohepten-4,5-diol (AU229) as colorless plates. melting point: 94 to 96°C.
IR (KBr, cm⁻¹): 3600-3200, 1716, 1281, 1115, 1071, 713.
1H-NMR (CDCl₃, ppm): 1.11 (3H, s), 1.18 (3H, s), 1.64 (1H, dd, J=14, 2Hz), 1.80 (3H, t, J=2Hz), 1.87 (1H, dd, J=14, 10Hz), 2.61 (1H, brs; disappeared by the addition of heavy water), 2.83 (1H, brs; disappeared by the addition of heavy water), 3.21 (1H, dd, J=9, 4Hz; d by the addition of heavy water, J=9Hz), 3.87 (1H, brd, J=9Hz), 5.46 (1H, m), 5.76 (1H, brd, J=10Hz), 7.46 (2H, m), 7.58 (1H, m), 8.05 (2H, m).

### Example 90

To a solution of 5-hydroxy-4-(4-methoxybenzyloxy)-2,6,6-trimethyl-2-cyclohepten-1-one (30.4g, 0.1M) obtained in Example 1 in tetrahydrofuran (200ml), dihydropyran (43ml, 0.5M) and p-toluenesulfonic acid (0.5g) were added and stirred for four hours at 0 °C, concentrated, diluted with ethyl acetate, washed with a saturated sodium bicarbonate solution, dried over magnesium sulfate, filtered and concentrated to obtain 54.7g of crude 4-(4-methoxybenzyloxy)-5-tetrahydropyranyloxy-2,6,6-trimethyl-2-cyclohepten-1-one.
IR (KBr, cm⁻¹): 1671, 1514, 1249, 1034, 819.

Sodium borohydride (7g, 0.19M) was added to a solution of the above product in methanol (200ml) at 0 °C, stirred for four hours, then concentrated, diluted with ethyl acetate washed with brine, dried over magnesium sulfate, filtered and concentrated. The residue was purified by silica gel column chromatography using hexane-ethyl acetate (10:1) to obtain 28.3g of 4-(4-methoxybenzyloxy)-5-tetrahydropyranyloxy-2,6,6-trimethyl-2-cyclohepten-1-ol as a colorless oily product.
IR (KBr, cm⁻¹): 3444, 1613, 1514, 1248, 1032, 820.

The above product (4.69g, 12mM) was benzoylated according to the procedures described in Example 6, purified by silica gel column chromatography using hexane-ethyl acetate (10:1) as an eluent to obtain 5.0g of a colorless oily product which was dissolved in methanol (30ml), added by p-toluenesulfonic acid (0.1g), stirred for 30 minutes, and concentrated. The residue was diluted by ethyl acetate, washed with a saturated sodium bicarbonate solution, dried over magnesium sulfate, filtered and concentrated. The residue was crystallized from a mixed solution of ethyl acetate and hexane to obtain 2.30g (46.7%) of 1-benzoyloxy-4-(4-methoxybenzyloxy)-2,6,6-trimethyl-2-cyclohepten-5-ol as colorless prisms. melting point: 106.5 to 107°C.
1H-NMR (CDCl₃, ppm): 1.11 (3H, s), 1.17 (3H, s), 1.60 (1H, dd, J=13, 1Hz), 1.84 (4H, m), 3.21 (1H, brs; disappeared by the addition of heavy water), 3.23 (1H, d, J=10Hz), 3.82 (3H, s), 4.14 (1H, dm, J=10Hz), 4.43 (1H, d, J=11Hz), 4.70 (1H, d, J=11Hz), 5.50 (1H, m), 5.77 (1H, brd, J=11Hz), 6.90 (2H, d, J=9Hz), 7.29 (2H, d, J=9Hz), 7.46 (2H, m), 7.59 (1H, m), 8.06 (2H, m).

The above product (820mg, 2mM) was benzoylated again and purified by silica gel column chromatography using hexane-benzene (1:1) and benzene as an eluent to obtain 740mg (72%) of a colorless oily product. The product so obtained was treated with DDQ according to the procedures described in Example 7, purified by silica gel column chromatography using hexane-ethyl acetate (10:1) as an eluent and crystallized from a mixed solvent of hexane-ethyl acetate to obtain 2.30g (46.7%) of 1,5-dibenzoyloxy-2,6,6-trimethyl-2-cyclohepten-4-ol (AU228) as colorless prisms. melting point: 142 to 143.5°C.
IR (KBr, cm⁻¹): 3503, 1719, 1707, 1285, 709.
1H-NMR (CDCl₃, ppm): 1.03 (3H, s), 1.40 (3H, s), 1.75 (1H, dd, J=14, 2Hz), 1.86 (3H, t, J=2Hz), 1.90 (1H, brs; disappeared by the addition of heavy water), 2.01 (1H, dd, J=14, 10Hz), 4.63 (1H, brd, J=9Hz), 4.97 (1H, d, J=9Hz), 5.55 (1H, m), 5.84 (1H, brd, J=10Hz), 7.47 (4H, m), 7.58 (2H, m), 8.09 (4H, m).

### Example 91

4-(4-methoxybenzyloxy)-5-tetrahydropyranyloxy-2,6,6-trimethyl-2-cyclohepten-1-ol (28.3g) obtained as an intermediate in Example 90 was acetylated by a conventional method and dissolved in methanol(80ml) which was added by p-toluenesulfonic acid (0.2g) and stirred for an hour at room temperature, concentrated, diluted with ethyl acetate, washed with a saturated sodium bicarbonate solution, dried over magnesium sulfate, filtered and concentrated. The residue was purified by silica gel column chromatography using hexane-ethyl acetate (20:1) as an eluent to obtain 17.4g of 1-acetoxy-4-(4-methoxybenzyloxy)-2,6,6-trimethyl-2-cyclohepten-5-ol as a colorless oily product.
1H-NMR (CDCl₃,+D₂O, ppm): 1.08 (3H, s), 1.10 (3H, s), 1.43 (1H, dd, J=14, 2Hz), 1.62 (1H, m), 1.70 (3H, brs), 2.08 (3H, s), 3.16 (1H, dd, J=10, 2Hz), 3.81 (3H, s), 4.18 (1H, dm, J=10Hz), 4.39 (1H, d, J=11Hz), 4.47 (1H, d, J=11Hz), 5.43 (1H, m), 5.50 (1H, brd, J=11Hz), 6.89 (2H, d, J=8Hz), 7.26 (2H, d, J=8Hz).

A solution of the above product in tetrahydrofuran (80ml) was methylated according to the procedures described in Example 69 and purified by silica gel column chromatography using hexane-ethyl acetate (30:1) as an eluent to obtain 1-acetoxy-5-methoxy-4-(4-methoxybenzyloxy)-2,6,6-trimethyl-2-cycloheptene as a colorless oily product.
1H-NMR (CDCl₃, ppm): 1.06 (3H, s), 1.09 (3H, s), 1.45 (1H, dd, J=14, 2Hz), 1.68 (3H, brs), 1.73 (1H, dd, J=14, 10Hz), 2.07 (3H, s), 2.82 (1H, dd, J=10Hz), 3.54 (3H, s), 3.81 (3H, s), 4.13 (1H, dm, J=10Hz), 4.57 (1H, d, J=11Hz), 4.65 (1H, d, J=11Hz), 5.45 (2H, m), 6.88 (2H, d, J=8Hz), 7.30 (2H, d, J=8Hz).

The above product was dissolved in methanol (30ml), added by a solution (1ml) of 28% sodium methoxide in methanol, left to stand overnight at room temperature, added by dilute hydrochloric acid, extracted with ethyl acetate, washed with brine, dried over magnesium sulfate, filtered and concentrated to obtain 5-methoxy-4-(4-methoxybenzyloxy)-2,6,6-trimethyl-2-cyclohepten-1-ol. The product was dissolved in methylene chloride (50ml), added by activated manganese dioxide (30g), stirred at room temperature overnight and filtered. The filtrate was concentrated and the residue was purified by silica gel column chromatography using hexane-ethyl acetate (10:1) as an eluent to obtain 7.64g (24%) of 5-methoxy-4-(4-methoxybenzyloxy)-2,6,6-trimethyl-2-cyclohepten-1-one (AU221) as obtained in Example 69.

### Example 92

To a solution of 5-benzoyloxy-4-(4-methoxybenzyloxy)-2,6,6-trimethyl-2-cyclohepten-1-one (2.31g, 5.6mM) obtained in Example 6 in ethanol (10ml), sodium borohydride (0.11g, 2.8mM) was added with stirring at room temperature and stirred for further an hour. The mixture was added by ice-water, stirred for ten minutes, then extracted with ethyl acetate, washed with brine, dried over magnesium sulfate, filtered, concentrated and purified by silica gel column chromatography using benzene-ethyl acetate (20:1) as an eluent to obtain 1.52g (66%) of 5-benzoyloxy-4-(4-methoxybenzyloxy)-2,6,6-trimethyl-2-cyclohepten-1-ol (AU184) as a colorless oily prodct.
IR (KBr, cm⁻¹): 3474, 1719, 1514, 1274, 1250, 1118.
1H-NMR (CDCl₃, ppm): 1.02 (3H, s), 1.38 (3H, s), 1.74 (1H, dd, J=14, 1Hz), 1.86 (3H, brs), 2.01 (1H, dd, J=14, 11Hz), 2.09 (1H, d, J=7Hz), 3.88 (3H, s), 4.63 (1H, br), 4.93 (1H, d, J=10Hz), 5.55 (1H, m), 5.83 (1H, brd, J=11Hz), 6.95 (2H, d, J=9Hz), 7.47 (2H, m), 7.59 (1H, m), 8.06 (3H, m).

### Example 93

The product of Example 92 (2.0g, 4.87mM) was acetylated by a conventional method to obtain 1.63g (74%) of 1-acetoxy-5-benzoyloxy-4-(4-methoxybenzyloxy)-2,6,6-trimethyl-2-cyclohepten (AU135) as a colorless oily product.
1H-NMR (CDCl₃, ppm): 1.02 (3H, s), 1.27 (3H, s), 1.55 (1H, dd, J=14.2Hz), 1.74 (3H, t, J=2Hz), 1.88 (1H, dd, J=14, 11Hz), 2.09 ( 3H, s), 3.74 (3H, s), 4.24 (1H, dm, J=10Hz), 4.34 (1H, d, J=12Hz), 4.52 (1H, d, J=12Hz), 5.01 (1H, d, J=10Hz), 5.56 (2H, m), 6.65 (2H, d, J=9Hz), 6.96 (2H, d, J=9Hz), 7.46 (2H, m), 7.58 (1H, m), 8.05 (1H, m).

The product (1.62g, 3.58mM) was treated with DDQ according to the procedures described in Example 7, purified by silica gel column chromatography using benzene-hexane (5:1) and crystallized from hexane to obtain 0.69g (58%) of 1-acetoxy-5-benzoyloxy-2,6,6-trimethyl-2-cyclohepten-4-ol (AU137) as colorless needles. melting point: 113 to 114.5 °C.
IR (KBr, cm⁻¹): 3541, 3458, 1734, 1717, 1273, 1249, 1116, 712.
1H-NMR (CDCl₃, ppm): 1.02 (3H, s), 1.32 (3H, s), 1.58 (1H, dd, J=14, 2Hz), 1.75 (3H, brs), 1.88 (1H, dd, J=14, 11Hz), 2.10 (3H, s), 4.55 (1H, brd, J=10Hz), 4.90 (1H, d, J=10Hz), 5.49 (1H, m), 5.57 (1H, brd, J=11Hz), 7.47 (2H, m), 7.59 (1H, m), 8.08 (2H, m).

### Example 94

5-benzoyloxy-4-(4-methoxybenzyloxy)-2,6,6-trimethyl-2-cycloheptene-1-ol (3.0g, 7.3mM) obtained in Example 92 was methylated according to the procedures described in Example 69 and purified by silica gel column chromatography using hexane-ethyl acetate (20:1) as an eluent to obtain 1.01g (33%) of 5-benzoyloxy-1-methoxy-4-(4-methoxybenzyloxy)-2,6,6-trimethyl-2-cycloheptene (AU136) as a colorless oily product.
IR (KBr, cm⁻¹): 1723, 1613, 1514, 1271, 711.
1H-NMR (CDCl₃, ppm): 1.01 (3H, s), 1.24 (3H, s), 1.59 (1H, dd, J=13.2Hz), 1.70 (1H, dd, J=13, 10Hz), 1.80 (3H, brs), 3.33 (3H, s), 3.74 (3H, s), 3.90 (1H, brd, J=10Hz), 4.19 (1H, dm, J=10Hz), 4.33 (1H, d, J=12Hz), 4.52 (1H, d, J=12Hz), 4.96 (1H, d, J=10Hz), 5.48 (1H, m), 6.66 (2H, d, J=9Hz), 6.97 (2H, d, J=9Hz), 7.45 (2H, m), 7.56 (1H, m), 8.08 (2H, m).

### Example 95

5-methoxy-4-(4-methoxybenzyloxy)-2,6,6-trimethyl-2-cyclohepten-1-ol (980mg, 3mM) obtained as an intermediate in Example 91 was treated with benzoyl chloride (0.38ml, 3.3mM) according to the procedures described in Example 17 and purified by silica gel column chromatography using hexane-ethyl acetate (50:1) as an eluent to obtain 715mg of 1-benzoyloxy-5-methoxy-4-(4-methoxybenzyloxy)-2,6,6-trimethyl-2-cycloheptene as a colorless oily product.
1H-NMR (CDCl₃, ppm): 1.08 (3H, s), 1.15 (3H, s), 1.58 (1H, dd, J=14, 2Hz), 1.79 (3H, brs), 1.86 (1H, dd, J=14, 11Hz), 2.87 (1H, d, J=10Hz), 3.57 (3H, s), 3.81 (3H, s), 4.21 (1H, brd, J=10Hz), 4.61 (1H, d, J=11Hz), 4.68 (1H, d, J=11Hz), 5.53 (1H, m), 5.72 (1H, brd, J=11Hz), 6.88 (2H, d, J=9Hz), 7.32 (2H, d, J=9Hz), 7.47 (2H, m), 7.55 (1H, m), 8.05 (2H, m).

The above product (687mg, 1.6mM) was treated with DDQ according to the procedures described in Example 7, purified by silica gel column chromatography using benzene-ethyl acetate (20:1) and crystallized from a mixed solvent of ethyl acetate and hexane to obtain 197mg (40%) of 1-benzoyloxy-5-methoxy-2,6,6-trimethyl-2-cyclohepten-4-ol (AU227) as colorless plates. melting point: 82.5 to 83.5°C.
IR (KBr, cm⁻¹): 3488, 1716, 1282, 1099, 713.
1H-NMR (CDCl₃, ppm): 1.09 (3H, s), 1.14 (3H, s), 1.60 (1H, dd, J=14, 2Hz), 1.80 (3H, t, J=2Hz), 1.88 (1H, dd, J=14, 10Hz), 2.73 (1H, brs; disappeared by the addition of heavy water), 2.75 (1H, d, J=10Hz), 3.57 (3H, s), 4.40 (1H, dm, J=10Hz), 5.52 (1H, m), 5.75 (1H, brd, J=10Hz), 7.45 (2H, m), 7.58 (1H, m), 8.05 (2H, m).

### Example 96

To a solution of Saishin N (9.2g, 50mM) in methanol (700ml), p-toluenesulfonic acid (0.4g) was added and heated under reflux for five hours, diluted with ethyl acetate, washed with a saturated sodium bicarbonate solution and with brine, dried over magnesium sulfate, filtered and concentrated. The residue was fractionated by silica gel column chromatography using benzene-acetone (30:1 to 10:1) as an eluent. The first eluted portion was crystallized from hexane to obtain 3.27g (33%) of 1-methoxy-2,6,6-trimethyl-8-oxabicyclo [3.2.1] oct-2-en-4-ol (AU152) as colorless needles. melting point: 52 to 54 °C.
IR (KBr, cm⁻¹): 3455, 1452, 1277, 1178.
1H-NMR (CDCl₃+D₂O, ppm): 1.26 (3H, s), 1.29 (3H, s), 1.67 (3H, t, J=2Hz), 1.81 (2H, s), 3.36 (3H, s), 3.92 (1H, dd, J=5, 2Hz), 4.82 (1H, m), 5.44 (1H, m).
13C-NMR (CDCl₃, ppm): 16.33 (q), 26.37 (q), 33.69 (q), 40.69 (s), 50.45 (t), 51.43 (q), 70.00 (d), 85.29 (d), 108.19 (s), 126.27 (d), 139.35 (s).

The successive eluted portion was crystallized from hexane to obtain 1.41g (14%) of 1-methoxy-3,3,7-trimethyl-8-oxabicyclo [3.2.1] oct-6-en-4-ol (AU153) as colorless needles. melting point: 82.5 to 83.5 °C. IR (KBr, cm⁻¹): 3471, 1335, 1158, 1005.
1H-NMR (CDCl₃+D₂O, ppm): 0.95 (3H, s), 1.06 (3H, s), 1.62 (2H, s), 1.76 (3H, brs), 3.30 (3H, s), 3.71 (1H, d, J=4Hz), 4.56 (1H, m), 6.00 (1H, m).
13C-NMR (CDCl₃, ppm): 12.52 (q), 24.53 (q), 33.58 (q), 34.32 (s), 41.67 (t), 50.10 (q), 73.80 (d), 78.90 (d), 110.69 (s), 127.86 (d), 142.51 (s).

### Example 97

1-methoxy-3,3,7-trimethyl-8-oxabicyclo [3.2.1] oct-6-en-4-ol (396mg, 2mM) obtained in Example 96 was acetylated by a conventional method and crystallized from a mixed solution of methanol and water to obtain 299mg (62%) of 4-acetoxy-1-methoxy-3,3,7-trimethyl-8-oxabicyclo [3.2.1] oct-6-ene (AU159) as colorless needles. melting point: 54 to 55 °C.
IR (KBr, cm⁻¹): 1735, 1244, 1163, 1020.
1H-NMR (CDCl₃, ppm): 0.97 (3H, s), 1.02 (3H, s), 1.63 (1H, d, J=14Hz), 1.72 (1H, d, J=14Hz), 1.77 (3H, brs), 2.07 (3H, s), 3.30 (3H, s), 4.61 (1H, m), 4.90 (1H, d, J=4Hz), 5.89 (1H, m).

### Example 98

To a solution of 4-benzyloxy-5-hydroxy-2,6,6-trimethyl-2-cyclohepten-1-one (412mg, 1.5mM) obtained in Example 2 in methanol (20ml), concentrated sulfuric acid (0.1ml) was added and heated under reflux for three hours. The reaction mixture was diluted with ethyl acetate, washed with a saturated sodium bicarbonate solution and with brine, dried over magnesium sulfate and purified by silica gel column chromatography using hexane-ethyl acetate (20:1) as an eluent to obtain 144mg (33%) of 1-methoxy-4-benzyloxy-2,6,6-trimethyl-8-oxabicyclo [3.2.1] oct-2-ene (AU157) as a colorless oily product.
IR (KBr, cm⁻¹): 1720, 1453, 1272, 1214, 1060.
1H-NMR (CDCl₃, ppm): 1.24 (3H, s), 1.30 (3H, s), 1.66 (3H, brs), 1.81 (2H, s), 3.35 (3H, s), 4.02 (1H, dd, J=5, 2Hz), 4.55 (3H, m), 5.52 (1H, m), 7.33 (5H, m).

### Example 99

To a solution of 5-hydroxy-4-(4-nitrobenzoyloxy)-2,6,6-trimethyl-2-cyclohepten-1-one (2.00g, 6.0mM) obtained in Example 44 in methanol (30ml), p-toluenesulfonic acid (0.1g) was added. The mixture was heated under reflux for three hours and concentrated to a half amount. The separated crystal was collected by filtration and recrystallized from methanol to obtain 1.16g (74%) of 1-methoxy-4-(4-nitrobenzoyloxy)-2,6,6-trimethyl-8-oxabicyclo [3.2.1] oct-2-ene (AU244) as colorless needles. melting point: 140 to 141 °C.
IR (KBr, cm⁻¹): 1713, 1528, 1348, 1288, 1277, 714.
1H-NMR (CDCl₃, ppm): 1.28 (3H, s), 1.33 (3H, s), 1.74 (3H, t, J=2Hz), 1.89 (2H, s), 3.41 (3H, s), 4.25 (1H, dd, J=5, 2Hz), 5.49 (1H, m), 6.05 (1H, m), 8.19 (2H, d, J=9Hz), 8.31 (2H, d, J=9Hz).

### Example 100

The product of Example 99 (690mg, 2mM) was reduced at nitro group as described in Example 19 and crystallized from a mixed solvent of ethyl acetate and hexane to obtain 390mg (61%) of 4-(4-aminobenzoyloxy)-1-methoxy-2,6,6-trimethyl-8-oxabicyclo [3.2.1] oct-2-ene (AU245) as light yellow prisms. melting point: 117.5 to 119 °C.
IR (KBr, cm⁻¹): 3452, 3418, 3362, 3247, 1702, 1641, 1599, 1270, 1170.
1H-NMR (CDCl₃, ppm): 1.25 (3H, s), 1.33 (3H, s), 1.71 (3H, t, J=2Hz), 1.87 (2H, s), 3.40 (3H, s), 4.08 (2H, brs), 4.21 (1H, dd, J=5, 2Hz), 5.48 (1H, m), 5.96 (1H, m), 6.64 (2H, d, J=9Hz), 7.83 (2H, d, J=9Hz).

### Example 101

5-Hydroxy-4-(4-nitrobenzoyloxy)-2,6,6-trimethyl-2-cyclohepten-1-one (1.00g, 3.0mM) was treated with ethanol (20ml) according to the procedures described in Example 99 and crystallized from a mixed solution of ethyl acetate and hexane to obtain 0.7g (64%) of 1-ethoxy-4-(4-nitrobenzoyloxy)-2,6,6-trimethyl-8-oxabicyclo [3.2.1] oct-2-ene (AU246) as a light yellow flakes. meling point: 144 to 146 °C.
IR (KBr, cm⁻¹): 1713, 1527, 1346, 1287, 1277, 714.
1H-NMR (CDCl₃, ppm): 1.25 (3H, t, J=7Hz), 1.27 (3H, s), 1.33 (3H, s), 1.74 (3H, t, J=2Hz), 1.89 (1H, d, J=12Hz), 1.92 (1H, d, J=12Hz), 3.54 (1H, m), 3.74 (1H, m), 4.23 (1H, dd, J=5, 2Hz), 5.49 (1H, m), 6.04 (1H, m), 8.19 (2H, d, J=9Hz), 8.31 (2H, d, J=9Hz).

### Example 102

The product of Example 101 (578mg, 1.6mM) was reduced at nitro group according to the procedures described in Example 19 and crystallized from a mixed solvent of ethyl acetate and hexane to obtain 293mg (55%) of 4-(4-aminobenzoyloxy)-1-ethoxy-2,6,6-trimethyl-8-oxabicyclo [3.2.1] oct-2-ene (AU247) as orange plates. melting point: 130.5 to 131.5 °C.
IR (KBr, cm⁻¹): 3444, 3359, 3244, 1698, 1606, 1310, 1267, 1171, 1100.
1H-NMR (CDCl₃, ppm): 1.24 (3H, t, J=7Hz), 1.24 (3H, s), 1.32 (3H, s), 1.70 (3H, t, J=2Hz), 1.86 (1H, d, J=12Hz), 1.91 (1H, d, J=12Hz), 3.54 (1H, m), 3.74 (1H, m), 4.08 (2H, brs), 4.19 (1H, dd, J=5, 2Hz), 5.47 (1H, m), 5.96 (1H, m), 6.64 (2H, d, J=9Hz), 7.82 (2H, d, J=9Hz).

### Example 103

5-Hydroxy-4-(4-nitrobenzoyloxy)-2,6,6-trimethyl-2-cyclohepten-1-one (1.00g, 3.0mM) was treated with a mixed solution of ethylene glycol (5ml) and tetrahydrofuran (5ml) according to the prcedures described in Example 99 and crystallized from a mixed solvent of ethyl acetate and hexane to obtain 0.355g (31%) of 1-(2-hydroxyethoxy)-4-(4-nitrobenzoyloxy)-2,6,6-trimethyl-8-oxabicyclo [3.2.1] oct-2-ene (AU248) as colorless fine crystals. melting point: 166 to 167.54°C.
IR (KBr, cm⁻¹): 3446, 1713, 1527, 1346, 1277, 715.
1H-NMR (CDCl₃, cm): 1.28 (3H, s), 1.33 (3H, s), 1.77 (3H, t, J=2Hz), 1.91 (1H, d, J=12Hz), 1.96 (1H, d, J=12Hz), 2.58 (1H, brs), 3.76 (4H, m), 4.27 (1H, dd, J=5, 2Hz), 5.51 (1H, m), 6.04 (1H, m), 8.19 (2H, d, J=9Hz), 8.31 (2H, d, J=9Hz).

### Example 104

The product of Example 103 (367mg, 0.97mM) was reduced at nitro group according to the procedures described in Example 19 and purified by silica gel column chromatography using hexane-ethyl acetate (3:1) to obtain 4-(4-aminobenzoyloxy)-1-(2-hydroxyethoxy)-2,6,6-trimethyl-8-oxabicyclo [3.2.1] oct-2-ene (AU249) as a colorless oily product.
1H-NMR (CDCl₃, ppm): 1.26 (3H, s), 1.33 (3H, s), 1.73 (3H, t, J=2Hz), 1.86 (1H, d, J=12Hz), 1.95 (1H, d, J=12Hz), 2.73 (1H, brs), 3.76 (4H, m), 4.11 (2H, brs), 4.23 (1H, dd, J=5, 2Hz), 5.50 (1H, m), 5.96 (1H, m), 6.64 (2H, d, J=9Hz), 7.82 (2H, d, J=9Hz).

The product was dissolved in ethyl acetate (10ml) and added by 4N hydrogen chloride in ethyl acetate and a small amount of methanol and was solidified in a refrigerator to obtain 160mg (44%) of a hydrochloride as a colorless powder. melting point: 157 to 159 °C.
IR (KBr, cm⁻¹): 3600-3300, 3000-2500, 1716, 1311, 1276, 1177, 1120.

### Example 105

To a solution of 5-hydroxy-4-(4-tetrahydropyranyloxy cinnamoyloxy)-2,6,6-trimethyl-2-cyclohepten-1-one (1.0g, 2.4mM) obtained as an intermediate in Example 65 in methanol (60ml), p-toluenesulfonic acid (25mg) was added. The mixture was stirred at room temperature for a day, concentrated, diluted with ethyl acetate, washed with a saturated sodium bicarbonate solution and with brine, dried over magnesium sulfate, filtered and concentrated. The residue was crystallized from a mixed solvent of ethyl acetate and hexane to obtain 0.35g (42%) of 4-(4-hydroxy cinnamoyloxy)-1-methoxy-2,6,6-trimethyl-8-oxabicyclo [3.2.1] oct-2-ene (AU508) as colorless crystals. melting point: 143 to 145 °C.
IR (KBr, cm⁻¹): 3378, 1677, 1629, 1599, 1278, 1205, 1170.
1H-NMR (CDCl₃, ppm): 1.24 (3H, s), 1.31 (3H, s), 1.72 (3H, t, J=2Hz), 1.88 (2H, s), 3.40 (3H, s), 4.20 (1H, dd, J=6, 2Hz), 5.46 (1H, t, J=2Hz), 5.89 (1H, m), 6.26 (1H, d, J=16Hz), 6.87 (2H, d, J=9Hz), 7.18 (1H, brs), 7.42 (2H, d, J=9Hz), 7.63 (1H, d, J=16Hz).

### Example 106

5-Hydroxy-4-(3-methoxy-4-tetrahydropyranyloxy cinnamoyloxy)-2,6,6-trimethyl-2-cyclohepten-1-one (1.94g, 4.36mM) obtained as an intermediate in Example 61 was treated according to the procedures described in Example 105 and crystallized from hexane-ethyl acetate to obtain 0. 74g (45%) of 4-(4-hydroxy-3-methoxycinnamoyloxy)-1-methoxy-2,6,6-trimethyl-8-oxabicyclo [3.2.1 ] oct-2-ene (AU417) as colorless crystals.
IR (KBr, cm⁻¹): 3531, 3434, 1702, 1629, 1603, 1513, 1264, 1172.
1H-NMR (CDCl₃, ppm): 1.26 (3H, s), 1.29 (3H, s), 1.71 (3H, t, J=2Hz), 1.86 (2H, s), 3.39 (3H, s), 3.94 (3H, s), 4.19 (1H, dd, J=5, 2Hz), 5.45 (1H, q, J=2Hz), 5.86 (1H, s; disappeared by the addition of heavy water), 5.86 (1H, m), 6.25 (2H, d, J=16Hz), 6.92 (1H, d, J=8Hz), 7.02 (1H, d, J=2Hz), 7.08 (1H, dd, J=8, 2Hz), 7.60 (2H, d, J=16Hz).

### Example 107

5-Hydroxy-4-(4-methoxybenzyloxy)-2,6,6-trimethyl-2-cycloheptene-1-one (3.04g, 10mM) obtained in Example 1 was methylated according to the proceduces described in Example 69 and purified by silica gel column chromatography using hexane-ethyl acetate (20:1) as an eluent to obtain 1.85g (58%) of 1-methoxy-4-(4-methoxybenzyloxy)-2,6,6-trimethyl-8-oxabicyclo [3.2.1] octa-2-ene as a colorless oily product.
1H-NMR (CDCl₃, ppm): 1.23 (3H, s), 1.28 (3H, s), 1.66 (3H, dd, J=2, 1Hz), 1.81 (2H, s), 3.35 (3H, s), 3.81 (3H, s), 4.00 (1H, dd, J=5, 2Hz), 4.40-4.60 (3H, m), 5.50 (1H, m), 6.88 (2H, d, J=7Hz), 7.26 (2H, d, J=7Hz).

The above product (3.18g, 10mM) was treated with 2.6 M equivalent of DDQ according to the procedures described in Example 7 and purified by silica gel column chromatography using benzene-ethyl acetate (30:1) as an eluent to obtain 1.63g (83%) of 1-methoxy-2,6,6-trimethyl-8-oxabicyclo [3.2.1] oct-2-en-4-one (AU253) as a colorless oily product.
IR (KBr, cm⁻¹): 1686, 1625, 1438, 1265, 1055.
1H-NMR (CDCl₃, ppm): 0.99 (3H, s), 1.36 (3H, s), 1.65 (1H, d, J=13Hz), 1.94 (1H, d, J=13Hz), 2.00 (3H, s), 3.43 (3H, s), 4.03 (1H, s), 5.84 (1H, brs).

To a solution of the above product in methanol (10ml) was added sodium borohydride (0.20g, 5.3mM) at 0 °C and stirred for an hour. The mixture was then diluted with water, extracted with ethyl acetate, washed with brine, dried over magnesium sulfate, filtered and concentrated. The residue was purified by silica gel column chromatography using hexane-ethyl acetate (5:1) as an eluent and crystallized from hexane to obtain 1.30g (79%) of 1-methoxy-2,6,6-trimethyl-8-oxabicyclo [3.2.1] oct-2-en-4-ol (AU152) as obtained in Example 96.

### Example 108

1-methoxy-2,6,6-trimethyl-8-oxabicyclo [3.2.1] oct-2-en-4-ol (297mg, 1.5mM) which was obtained in Example 107 was acetylated by a conventional method and purified by silica gel column chromatography using hexane-ethyl acetate (20:1) as an eluent to obtain 312mg (86%) of 4-acetoxy-1-methoxy-2,6,6-trimethyl-8-oxabicyclo [3.2.1] oct-2-ene (AU158) as a colorless oily product.
IR (KBr, cm⁻¹): 1742, 1237, 1042.
1H-NMR (CDCl₃, ppm): 1.22 (3H, s), 1.24 (3H, s), 1.69 (3H, t, J=2Hz), 1.83 (2H, s), 2.06 (3H, s), 3.37 (3H, s), 4.09 (1H, dd, J=5, 2Hz), 5.38 (1H, m), 5.72 (1H, m).

### Example 109

To a solution of 5-hydroxy-4-(4-methoxybenzyloxy)-2,6,6-trimethyl-2-cycloheptene-1-one (3.04g, 10mM) which was obtained in Example 1 in tetrahydrofuran (30ml), 55% sodium hydride (0.52g, 12mM) was added under ice-cooling with stirring. After ten minutes, the mixture was heated to 60 C for ten minutes, ice-cooled, added by benzoyl chloride (1.39ml, 12mM), then cooled down to room temperature and stirred for an hour. The reaction mixture was concentrated, diluted with ethyl acetate, washed with water and with brine, dried over magnesium sulfate, filtered and concentrated. The residue was crystallized from a mixed solvent of ethyl acetate and hexane to obtain 2.75g (67%) of 1-benzoyloxy-4-(4-methoxybenzyloxy)-2,6,6-trimethyl-8-oxabicyclo [3.2.1] oct-2-en as a colorless prisms. melting point: 106.5 to 107°C.
Ir (KBr, cm⁻¹): 1736, 1613, 1514, 1267, 1148, 710.
1H-NMR (CDCl₃, ppm): 1.31 (3H, s), 1.37 (3H, s), 1.68 (3H, t, J=2Hz), 2.19 (2H, s), 3.82 (3H, s), 4.15 (1H, dd, J=5, 2Hz), 4.52 (1H, d, J=12Hz), 4.57 (1H, d, J=12Hz), 4.75 (1H, m), 5.51 (1H, m), 6.89 (2H, d, J=9Hz), 7.28 (2H, d, J=9Hz), 7.45 (2H, m), 7.59 (1H, m), 8.06 (2H, m).

The above product (0.4g, 1mM) was treated with DDQ according to the procedures described in Example 17 and fractionated by silica gel column chromatography using benzene-ethyl acetate (50:1 to 5:1) to obtain 130mg (45%) of 1-benzoyloxy-2,6,6-trimethyl-8-oxabicyclo [3.2.1] oct-2-en-4-one (AU168) as a colorless oily product.
IR (KBr, cm⁻¹: 1737, 1684, 1674, 1454, 1276, 1144, 710.
1H-NMR (CDCl₃, ppm): 1.06 (3H, s), 1.44 (3H, s), 2.01 (3H, d, J=2Hz), 2.09 (1H, d, J=12Hz), 2.33 (1H, d, J=12Hz), 4.21 (1H, d, J=2Hz), 5.91 (1H, m), 7.48 (2H, m), 7.62 (1H, m), 8.08 (2H, m).

109mg (38%) of 1-benzoyloxy-2,6,6-trimethyl-8-oxabicyclo [3.2.1] oct-2-en-4-ol (AU169) as a colorless oily product was obtained from an eluted portion of 10:1 to 5:1.
IR (KBr, cm⁻¹): 3445, 1732, 1689, 1452, 1262, 1144, 711.
1H-NMR (CDCl₃, ppm): 1.34 (3H, s), 1.37 (3H, s), 1.68 (3H, t, J=2Hz), 2.20 (2H, s), 4.90 (1H, dd, J=5, 2Hz), 5.03 (1H, m), 5.44 (1H, m), 7.44 (2H, m), 7.57 (1H, m), 8.05 (2H, m).

### Example 110

A solution of 5-benzoyloxy-4-methoxy-2,6,6-trimethyl-2-cyclohepten-1-one (1.51g, 5.0mM) obtained in Example 16 in methanol (20ml) was catalytically hydrogenated by the addition of 10% palladium carbon (0.02g), and then the catalyst was filtered off. The filtrate was concentrated and the residue was fractionated by silica gel column chromatography using benzene-ethyl acetate (25:1) to obtain 0.26g (17%) of 2,4-trans-5-benzoyloxy-4-methoxy-2,6,6-trimethylcycloheptan-1-one (AU123A) as colorless plates from the first eluted portion. melting point: 101 to 102 °C.
IR (KBr, cm⁻¹): 1718, 1698, 1272, 1112, 713.
1H-NMR (CDCl₃, ppm): 1.02 (3H, s), 1.15 (3H, s), 1.18 (3H, d, J=7Hz), 2.06 (2H, m), 2.33 (1H, d, J=13Hz), 2.65 (1H, m), 2.95 (1H, d, J=13Hz), 3.38 (3H, s), 3.56 (3H, m), 5.22 (1H, d, J=5Hz), 7.48 (2H, m), 7.58 (1H, m), 8.07 (2H, m).

The successive eluted portion was crystallized from hexane to obtain 0.47g (31%) of 2,4-cis-5-benzoyloxy-4-methoxy-2,6,6-trimethyl cycloheptan-1-one (AU123B) as colorless needles. melting point: 133 to 134.5°C.
IR (KBr, cm⁻¹): 1725, 1694, 1270, 1094, 714.
1H-NMR (CDCl₃, ppm): 1.06 (3H, s), 1.08 (3H, s), 1.19 (3H, d, J=7Hz), 1.84 (1H, m), 2.09 (1H, m), 2.24 (1H, d, J=13Hz), 2.38 (1H, m), 2.87 (1H, d, J=13Hz), 3.29 (3H, s), 3.29 (1H, m), 5.13 (1H, d, J=9Hz), 7.47 (2H, m), 7.58 (1H, m), 8.08 (2H, m).

### Example 111

5- (3-chlorobenzoyloxy)-4-(4-methoxybenzyloxy)-2,6,6-trimethyl-2-cyclohepten-1-one (3.00g, 6.78mM) obtained in Example 11 was catalytically hydrogenated according to the procedures described in Example 110, purified by silica gel column chromatogrphy using hexane-ethyl acetate (10:1) as an eluent and crystallized from a mixed solvent of ethyl acetate and hexane (10:1) as an eluent and crystallized from a mixed solvent of ethyl acetate and hexane to obtain 0.81g (27%) of 2,4-cis-5-(3-chlorobenzoyloxy)-4(4-methoxybenzyloxy)-2,6,6-trimethyl cycloheptan-1-one (AU131) as colorless needles. melting point: 104 to 105°C.
IR (KBr, cm⁻¹): 1713, 1615, 1514, 1257, 1069, 820, 745.
1H-NMR (CDCl₃, ppm): 1.01 (3H, s), 1.06 (3H, s), 1.18 (3H, d, J=7Hz), 1.89 (1H, m), 2.10 (1H, m), 2.22 (1H, d, J=13Hz), 2.32 (1H, m), 2.87 (1H, d, J=13Hz), 3.50 (1H, brd, J=9Hz), 3.75 (3H, s), 4.30 (1H, d, J=11Hz), 4.50 (1H, d, J=11Hz), 5.17 (1H, d, J=9Hz), 6.66 (2H, d, J=9Hz), 6.97 (2H, d, J=9Hz), 7.40 (1H, t, J=8Hz), 7.55 (1H, dm, J=8Hz), 7.89 (1H, dt, J=8, 2Hz), 7.94 (1H, t, J=2Hz).

### Example 112

The product of Example 111 (795mg, 1.76mM) was treated with DDQ according to the procedures described in Example 7 and crystallized from a mixed solvent of ethyl acetae and hexane to obtain 405mg (70%) of 2,4-cis-5-(3-chlorobenzoyloxy)-4-hydroxy-2,6,6-trimethyl cycloheptan-1-one (AU132) as colorless plates. melting point: 136 to 137 °C.
IR (KBr, cm⁻¹): 3457, 1722, 1675, 982, 746.
1H-NMR (CDCl₃, ppm): 1.05 (3H, s), 1.07 (3H, s), 1.18 (3H, d, J=7Hz), 1.94 (1H, d, J=6Hz; disappeared by the addition of heavy water), 1.94 (1H, m), 2.10 (1H, m), 2.21 (1H, d, J=13Hz), 2.44 (1H, m), 2.89 (1H, d, J=13Hz), 3.82 (1H, m; brt by the addition of heavy water, J=12, 9Hz), 5.03 (1H, d, J=9Hz), 7.42(1H, t, J=8Hz), 7.57 (1H, dm, J=8Hz), 7.96 (1H, dt, J=8, 2Hz), 8.04 (1H, t, J=2Hz).

### Example 113

A solution of Saishin N (11.05g, 60mM) in methanol (50ml) was catalytically hydrogenated by the addition of 10% palladium/carbon (0.2g) at room temperature and filtered. The filtrate was added by a solution (2ml) of 28% sodium methoxide in methanol and stirred for a day at room temperature. The reaction mixture was concentrated, diluted with ethyl acetate, washed with brine, dried over sodium sulfate, filtered and concentrated. The residue was purified by silica gel column chromatography using hexane-ethyl acetate (10:1) as an eluent to obtain 8.40g (76%) of 2,4-cis-2,6,6-trimethyl-8-oxabicyclo [3.2.1] octane-1,4-diol (AUM11) as a colorless oily product.
IR (KBr, cm⁻¹): 3404, 1694, 1458, 1052, 1034.

### Example 114

5-hydroxy-4-methoxy-2,6,6-trimethyl-2-cyclohepten-1-one (24g, 0.12mM) obtained in Example 4 was catalytically hydrogenated and treated with base according to the procedures described in Example 113 and crystallized from hexane to obtain 15.2 (63%) of 2,4-cis-4-methoxy-2,6,6-trimethyl-8-oxabicyclo [3.2.1] octan-1-ol (AUM13) as a colorless crystalline product.
IR (KBr, cm⁻¹): 3356, 1270, 1114, 1064, 1008, 985, 974.

### Example 115

5-hydroxy-4-(4-methoxybenzyloxy)-2,6,6-trimethyl-2-cyclohepten-1-one (16.6g, 54.5mM) obtained in Example 1 was catalytically hydrogenated and treated with base according to the procedures described in Example 113 to obtain 12.2g (73%) of 2,4-cis-4-(4-methoxybenzyloxy)-2,6,6-trimethyl-8-oxabicyclo [3.2.1] octan-1-ol (AUM15) as a colorless oily product.
IR (KBr, cm⁻¹): 3384, 1514, 1247, 1062, 1036, 752.

### Example 116

A solution of Saishin N (25.8g, 0.1M) in methanol was catlytically hydrogenated and treated with base according to the procdureds described in Example 113, then added by p-toluenesulfonic acid and further left to stand for two days. Pottasium carbonate was added to the reaction mixture, stirred, concentrated at a reduced pressure, diluted with ethyl acetae, washed with bride, dried over magnesium sulfate, filtered and concentrated. The residue was crystallized from hexane to obtain 18.1g (64%) of 2,4-cis-1-methoxy-2,6.6-trimethyl-8-oxabicyclo [3.2.1] octan-4-ol (AU105B) as colorless cottony crystals. melting point: 74 to 75° C.
IR (KBr, cm⁻¹): 3242, 1464, 1349, 1056, 1011.
1H-NMR (CDCl₃, ppm): 0.85 (3H, d, J=6Hz), 1.25 (3H, s), 1.26 (3H, s), 1.43 (1H, t, J=14Hz), 1.57 (1H, d, J=14Hz) 1.68 (1H, d, J=14Hz), 2.02 (2H, m), 3.36 (3H, s), 3.60 (1H, brd, J=4Hz), 4.00 (1H, m).

The mother liquor of the crystal was purified by silica gel column chromatography using hexane-ethyl acetate (10:1) as an eluent and crystallized from hexane to obtain 4.65g (16%) of 2,4-trans-1-methoxy-2,6,6-trimethyl-8-oxabicyclo [3.2.1] octan-4-ol (AU105A) as colorless cottony crystals. melting point: 93 to 93.5 °C.
IR (KBr, cm⁻¹): 3217, 1464, 1354, 1280, 1048.
1H-NMR (CDCl₃, ppm): 1.09 (3H d, J=7Hz), 1.24 (3H, s), 1.29 (3H, s), 1.41 (1H, d, J=5Hz; disappeared by the addition of heavy water), 1.62 (1H, d, J=14Hz), 1.77 (1H, m), 1.88-2.06 (3H, m), 3.35 (3H, s), 3.60 (1H, brd, J=4Hz), 4.15 (1H, m).

### Example 117

2,4-cis-1-methoxy-2,6,6-trimethyl-8-oxabicyclo [3.2.1] octan-4-ol (0.3g, 1.5mM) obtained in Example 116 was acetylated by a conventional method to obtain 349mg (96.1%) of 2,4-cis-4-acetoxy-1-methoxy-2,6,6-trimethyl-8-oxabicyclo [3.2.1] octane (AU108B) as a crystalline product. melting point: 52 to 53 °C.
IR (KBr, cm⁻¹): 1734, 1472, 1248.
1H-NMR (CDCl₃, ppm): 0.84 (1H, d, J=6Hz), 1.20 (3H, s), 1.23 (3H, s), 1.42 (1H, q, J=12Hz), 1.58 (1H, d, J=13Hz), 1.70 (1H, d, J=13Hz), 2.03 (3H, s), 2.08 (2H, m), 3.37 (3H, s), 3.71 (1H, d, J=4Hz), 4.95 (1H, m).

### Example 118

2,4-trans-1-methoxy-2,6,6-trimethyl-8-oxabicyclo [3.2.1] octan-4-ol (0.3g, 1.5mM) obtained in Example 116 was acetylated by a conventional method to obtain 317mg (87.3%) of 2,4-trans-1-methoxy-4-acetoxy-2,6,6-trimethyl-8-oxabicyclo [3.2.1] octane (AU108A) as a colorless oily product.
IR (KBr, cm⁻¹): 1738, 1241, 1041.
1H-NMR (CDCl₃, ppm): 1.13 (3H, d, J=7Hz), 1.22 (3H, s), 1.23 (3H, s), 1.63 (1H, d, J=13Hz), 1.79 (1H, m), 2.03 (3H, s), 1.90-2.10 (3H, m), 3.35 (3H, s), 3.70 (1H, d, J=4Hz), 5.11 (1H, m).

### Example 119

2,4-cis-1-methoxy-2,6,6-trimethyl-8-oxabicyclo [3.2.1] octan-4-ol (0.30g, 1.5mM) obtained in Example 116 was methylated according to the procedures described in Example 69 to obtain 0.235g (73.2%) of 2,4-cis-1,4-dimethoxy-2,6,6-trimethyl-8-oxabicyclo [3.2.1] octane (AU109B) as a colorless oily product.
1H-NMR (CDCl₃, ppm): 0.84 (3H, d, J=6Hz), 1.20 (3H, s), 1.23 (3H, s), 1.31 (1H, d, J=12Hz), 1.55 (1H, d, J=13Hz), 1.66 (1H, d, J=13Hz), 1.94-2.11 (2H, m), 3.34 (3H, s), 3.37 (3H, s), 3.47-3.55 (1H, m), 3.75 (1H, d, J=4Hz).

### Example 120

2,4-trans-1-methoxy-2,6,6-trimethyl-8-oxabicyclo [3.2.1] octan-4-ol (0.30g, 1.5mM) obtained in Example 116 was treated as described in Example 119 to obtain 0.256g (80.7%) of 2,4-trans-1,4-dimethoxy-2,6,6-trimethyl-8-oxabicyclo [3.2.1] octane (AU109A) as a colorless oily product.
IR (KBr, cm⁻¹): 1468, 1219, 1109.
1H-NMR (CDCl₃, ppm): 1.09 (3H, d, J=7Hz), 1.22 (3H, s), 1.23 (3H, s), 1.60 (1H, d, J=14Hz), 1.50-2.00 (4H, m), 3.34 (3H, s), 3.35 (3H, s), 3.64 (1H, m), 3.74 (1H, m).

### Example 121

To a solution of 2,4-cis-1-methoxy-2,6,6-trimethyl-8-oxabicyclo [3.2.1] octan-4-ol (1.0g, 5.0mM) obtained in Example 116 in tetrahydrofuran (50ml), a solution (1.59M/1; 4.7ml) of n-butyl lithium in hexane was added under argon atmosphere at -60°C. After stirring at -60 C for 30 minutes, the benzoyl chloride (0.84g, 7.2mM) was added to the mixture, stirred for 30 minutes, and warmed up to room temperature. The reaction mixture was poured onto ice-water and saturated with salt, extracted with ethyl acetate, dried, purified by silica gel column chromatography using hexane-ethyl acetate (50:1) as an eluent and crystallized from hexane to obtain 753mg (49.5%) of 2,4-cis-4-benzoyloxy-1-methoxy-2,6,6-trimethyl-8-oxabicyclo [3.2.1] octane (AU119) as colorless needles. melting point: 89 to 90 °C.
IR (KBr, cm⁻¹): 1709, 1599, 1469, 1278, 1110, 1013, 711.
1H-NMR (CDCl₃, ppm): 0.88 (3H, d, J=7Hz), 1.27 (3H, s), 1.35 (3H, s), 1.50-1.70 (2H, m), 1.78 (1H, d, J=14Hz), 2.20 (2H, m), 3.40 (3H, s), 3.83 (1H, d, J=4Hz), 5.24 (1H, m), 7.44 (2H, m), 7.57 (1H, m), 8.00 (2H, m).

### Example 122

To a solution of 2,4-cis-1-methoxy-2,6,6-trimethyl-8-oxabicyclo [3.2.1] octan-4-ol (4.52g, 22mM) obtained in Example 116 in tetrahydrofuran, 55% sodium hydride (1.05g, 24mM) and benzyl bromide (2.85ml, 24mM) were added under ice-cooling, stirred for two hours at room temperature and heated under reflux for 30minutes. The reaction mixture was added by ice-water, extracted with ethyl acetate, washed with brine, then dried over magnesium sulfate, filtered and concentrated. The residue was fractionated by silica gel column chromatography using hexane-ethyl acetate (100:1 to 30:1) as an eluent. From the first eluted portion 1.00g (15.7%) of 2,4-trans-4-benzyloxy-1-methoxy-2,6,6-trimethyl-8-oxabicyclo [3.2.1] octane (AU162A) was obtained as a colorless oily product.
IR (KBr, cm⁻¹): 1466, 1217, 1110.
1H-NMR (CDCl₃, ppm): 1.07 (3H, d, J=7Hz), 1.22 (3H, s), 1.30 (3H, s), 1.63 (1H, d, J=14Hz), 1.91 (1H, d, J=14Hz), 1.82-2.10 (3H, m), 3.34 (3H, s), 3.73 (1H, brd, J=4Hz), 3.84 (1H, m), 4.53 (2H, s), 7.31 (5H, m).

2.36g (37.0%) of 2,4-cis-4-benzyloxy-1-methoxy-2,6,6-trimethyl-8-oxabicyclo [3.2.1] octane (AU162B) was obtained as a colorless oily product from the successive eluted portion.
IR (KBr, cm⁻¹): 1466, 1220, 1114, 1071.
1H-NMR (CDCl₃, ppm): 0.84 (3H, d, J=7Hz), 1.24 (3H, s), 1.28 (3H, s), 1.42 (1H, m), 1.57 (1H, d, J=14Hz), 1.68 (1H, d, J=14Hz), 1.98-2.09 (2H, m), 3.36 (3H, s), 3.70 (1H, m), 3.75 (1H, m), 4.53 (2H, s), 7.30 (5H, m).

### Example 123

2,4-cis-2,6,6-trimethyl-8-oxabicyclo [3.2.1] octan-1,4-diol (0.93g, 5mM) obtained in example 113 was benzoylated as described in Example 6 and crystallized from hexane to obtain 1.04g (71%) of 2,4-cis-4-benzoyloxy-2,6,6-trimethyl-8-oxabicyclo [3.2.1] octan-1-ol (AU502) as colorless needles. melting point: 157 to 158°C.
IR (KBr, cm⁻¹): 3467, 1695, 1317, 1307, 1291, 1128, 720.

### Example 124

The product of example 123 (0.5g, 1.72mM) was acetylated by a conventional method and crystallized from water to obtain 0.43g (75%) of 2,4-cis-5-acetoxy-4-benzoyloxy-2,6,6-trimethylcycloheptan-1-one (AU194) as colorless crystals. melting point: 166 to 200°C.
IR (KBr, cm⁻¹): 1737, 1710, 1451, 1374, 1273, 1241, 1112, 715.
1H-NMR (CDCl₃, ppm): 1.01 (3H, s), 1.05 (3H, s), 1.16 (3H, d, J=7Hz), 1.90 (3H, s), 2.06 (2H, m), 2.26 (1H, d, J=13Hz), 2.54 (1H, m), 2.88 (1H, d, J=13Hz), 5.17 (2H, m), 7.45 (2H, m), 7.58 (1H, m), 7.98 (2H, m).

### Example 125

2,4-cis-2,6,6-trimethyl-8-oxabicyclo [3.2.1] octane-1,4-diol (0.93g, 5mM) obtained in Example 113 was treated with 3-chlorobenzoyl chloride (0.96g, 5.5mM) according to the procedures described in Example 17 and then acetylated by a conventional method and crystallized from a mixed solvent of ethyl acetate and hexane to obtain 0.59g (32%) of 2,4-cis-5-acetoxy-4-(3-chlorobenzoyloxy)-2,6,6-trimethylcycloheptan-1-one (AU199) as colorless needles. melting point: 191 to 192 °C.
IR (KBr, cm⁻¹): 1743, 1718, 1703, 1263, 1237, 1124, 754, 740.
1H-NMR (CDCl₃, ppm): 1.01 (3H, s), 1.05 (3H, s), 1.17 (3H, d, J=7Hz), 1.92 (3H, s), 2.04 (2H, m), 2.27 (1H, d, J=13Hz), 2.54 (1H, m), 2.88 (1H, d, J=13Hz), 5.15 (2H, m), 7.40 (1H, t, J=8Hz), 7.55 (1H, brd, J=8Hz), 7.86 (1H, brd, J=8Hz), 7.98 (1H, m).

### Example 126

2,4-cis-2,6,6-trimethyl-8-oxabicyclo [3.2.1] octane-1,4-diol (5.59g, 30mM) obtained in Example 113 was acetylated by a conventional method and crystallized from ethyl acetate and hexane to obtain 6.73g (83%) of 2,4-cis-4,5-diacetoxy-2,6,6-trimethylcycloheptan-1-one (AU117) as colorless needles. melting point: 77 to 78 °C.
IR (KBr, cm⁻¹): 1744, 1700, 1246, 1229.
1H-NMR (CDCl₃, ppm): 0.96 (3H, s), 1.00 (3H, s), 1.13 (3H, d, J=7Hz), 1.91 (2H, m), 2.02 (3H, s), 2.07 (3H, s), 2.22 (1H, d, J=13Hz), 2.47 (1H, m), 2.81 (1H, d, J=13Hz), 4.95 (2H, m).

### Example 127

2,4-cis-4-methoxy-2,6,6-trimethyl-8-oxabicyclo [3.2.1] octan-1-ol (1.0g, 5mM) obtained in Example 114 was acetylated by a conventional method and crystallized from hexane to obtain 0.9g (75%) of 2,4-cis-5-acetoxy-4-methoxy-2,6,6-trimethylcycloheptan-1-one (AU120) as colorless crystals. melting poing: 80 to 82 °C.
IR (KBr, cm⁻¹): 1742, 1699, 1239, 1092, 1026.
1H-NMR (CDCl₃, ppm): 0.92 (3H, s), 1.00 (3H, s), 1.16 (3H, d, J=7Hz), 1.74 (1H, m), 2.03 (1H, m), 2.11 (3H, s), 2.18 (1H, d, J=13Hz), 2.33 (1H, m), 2.78 (1H, d, J=13Hz), 3.13 (1H, m), 3.34 (3H, s), 4.85 (1H, d, J=9Hz).

### Example 128

2,4-cis-4-methoxy-2,6,6-trimethyl-8-oxabicyclo [3.2.1] octan-1-ol (1g, 1.5mM) obtained in Example 114 was benzoylated according to the procedures described in Example 6 and purified by silica gel column chromatography using benzene-ethyl acetate (50:1) as an eluent to obtain 272mg (17.9%) of 2,4-cis-1-benzoyloxy-4-methoxy-2,6,6-trimethyl-8-oxabicyclo [3.2.1] octane (AU122) as a colorless oily product.
1H-NMR (CDCl₃, ppm): 0.94 (3H, d, J=7Hz), 1.25 (3H, s), 1.38 (3H, s), 1.41 (1H, q, J=12Hz), 2.10 (3H, m), 2.72 (1H, m), 3.37 (3H, s), 3.65 (1H, m), 3.95 (1H, d, J=4Hz), 7.43 (2H, m), 7.55 (1H, m), 8.03 (2H, m).

### Example 129

2,4-cis-4-(4-methoxybenzyloxy)-2,6,6-trimethyl-8-oxabicyclo [3.2.1] octan-1-ol (1.83g, 6mM) was acetylated by a conventional method and fractionated by silica gel column chromatography using hexaneethyl acetate (10:1 to 5:1) as an eluent. From the first eluted portion 0.28g (13%) of 2,4-cis-1-acetoxy-4-(4-methoxybenzyloxy)-2,6,6-trimethyl-8-oxabicyclo [3.2.1] octane (AU186) was obtained as a colorless oily product.
IR (KBr, cm⁻¹): 1753, 1612, 1514, 1249.
1H-NMR (CDCl₃, ppm): 0.83 (3H, d, J=6Hz), 1.25 (3H, s), 1.29 (3H, s), 1.41 (1H, q, J=12Hz), 1.91 (1H, d, J=13Hz), 1.94 (1H, d, J=13Hz), 2.02 (1H, m), 2.05 (3H, s), 2.55 (1H, m), 3.75 (1H, m), 3.80 (3H, d), 3.84 (1H, m), 4.46 (2H, s), 6.86 (2H, d, J-9Hz), 7.22 (2H, d, J=9Hz).

1.41g (67%) of 2,4-cis-5-acetoxy-4-(4-methoxybenzyloxy)-2,6,6-trimethylcycloheptan-1-one (AU185) was obtained from the successive eluted portion.
IR (KBr, cm⁻¹): 1740, 1700, 1613, 1514, 1244, 1031.
1H-NMR (CDCl₃, ppm): 0.91 (3H, s) 1.01 (3H, s), 1.13 (3H, d, J=7Hz), 1.82 (1H, m), 2.04 (3H, s), 2.05 (1H, m), 2.17 (1H, d, J=13Hz), 2.28 (1H, m), 2.77 (1H, d, J=13Hz), 3.38 (1H, m), 3.80 (3H, s), 4.42 (1H, d, J=13Hz), 4.53 (1H, d, J=13Hz), 4.93 (1H, d, J=9Hz), 6.86 (2H, d, J=9Hz), 7.19 (2H, d, J=9Hz).

### Example 130

2,4-cis-4-(4-methoxybenzyloxy)-2,6,6-trimethyl-8-oxabicyclo [3.2.1] octan-1-ol (2.13g, 6.9mM) obtained in Example 115 was benzoylated according to the procedures described in Example 6 and fractionated by silica gel column chromatography using benzene and benzene-ethyl acetate (20:1) as an eluent. The first eluted part was crystallized from a mixed solvent of ethyl acetate and hexane to obtain 0.40g (14%) of 2,4-cis-1-benzoyloxy-4-(4-methoxybenzyloxy)-2,6,6-trimethyl-8-oxabicyclo [3.2.1] octane (AU188) as colorless plates. melting point: 89.5 to 90.5 °C.
IR (KBr, cm⁻¹): 1742, 1614, 1512, 1248.
1H-NMR (CDCl₃, ppm): 0.92 (3H, d, J=7Hz), 1.30 (3H, s), 1.37 (3H, s), 1.48 (1H, q, J=12Hz), 2.09 (3H, m), 2.71 (1H, m), 3.80 (3H, s), 3.84 (1H, m), 3.91 (1H, m), 4.47 (1H, d, J=12Hz), 4.51 (1H, d, J=12Hz), 6.87 (2H, d, J=9Hz), 7.24 (2H, d, J=9Hz), 7.42 (2H, m), 7.52 (1H, m), 8.01 (2H, m).

The successive eluted portion was crystallized from a mixed solvent of ethyl acetate and benzene to obtain 0.65g (23%) of 2,4-cis-5-benzoyloxy-4-(4-methoxybenzyloxy)-2,6,6-trimethylcycloheptan-1-one (AU187) as colorless needles. melting point: 70.5 to 71.5 °C.
IR (KBr, cm⁻¹): 1717, 1701, 1612, 1515, 1272, 713.
1H-NMR (CDCl₃, ppm): 1.02 (3H, s). 1.07 (3H, s), 1.16 (3H, d, J=7Hz), 1.89 (1H, m), 2.08 (1H, m), 2.23 (1H, d, J=13Hz), 2.34 (1H, m), 2.86 (1H, d, J=13Hz), 3.51 (1H, m), 3.73 (3H, s), 4.34 (1H, d, J=13Hz), 4.48 (1H, d, J=13Hz), 5.18 (1H, d, J=9Hz), 6.64 (2H, d, J=9Hz), 6.98 (2H, d, J=9Hz), 7.46 (2H, m), 7.58 (1H, m), 8.02 (2H, m).

### Example 131

2,4-cis-5-benzoyloxy-4-(4-methoxybenzyloxy)-2,6,6-trimethyl cycloheptan-1-one (574mg, 1.4mM) obtained in Example 130 was treated with DDQ according to the procedures described in Example 7 and crystallized from a mixed solvent of ethyl acetate and hexane to obtain 330mg (81%) of 2,4-cis-5-benzoyloxy-4-hydroxy-2,6,6-trimethyl cycloheptan-1-one (AU191) as colorless plates. melting point: 166 to 167.5°C.
IR (KBr, cm⁻¹): 3436, 1717, 1682, 1270, 706.
1H-NMR (CDCl₃ +D₂O, ppm): 1.06 (3H, s), 1.07 (3H, s), 1.18 (3H, d, J=7Hz), 1.96 (1H, m), 2.10 (1H, m), 2.20 (1H, d, J=13Hz), 2.44 (1H, m), 2.89 (1H, d, J=13Hz), 3.82 (1H, m), 5.03 (1H, d, J=9Hz), 7.48 (2H, m), 7.60 (1H, m), 8.09 (2H, m).

### Example 132

The product (206mg, 0.7mM) of Example 131 was acetylated by a conventional method and crystallized from water to obtain 190mg (81%) of 2,4-cis-4-acetoxy-5-benzoyloxy-2,6,6-trimethylcycloheptan-1-one (AU192) as colorless crystals. melting opint: 119.5 to 121°C.
IR (KBr, cm⁻¹): 1732, 1694, 1270, 1106, 712.
1H-NMR (CDCl₃ +D₂O, ppm): 1.07 (3H, s), 1.08 (3H, s), 1.17 (3H, d, J=7Hz), 1.98 (2H, m), 2.27 (1H, d, J=13Hz), 2.51 (1H, m), 2.90 (1H, d, J=13Hz), 5.13 (1H, m), 5,23 (1H, d, J=9Hz), 7,45 (2H, m), 7.58 (1H, m), 7.99 (2H, m).

### Example 133

2,4-cis-4-(4-methoxybenzyloxy)-2,6,6-trimethyl-8-oxabicyclo [3.2.1] octan-1-ol (1.2g, 3.9mM) obtained in Example 115 was treated with benzoyl chloride according to the procedures described in Example 17 to obtain 0.97g (60%) of 2,4-cis-1-benzoyloxy-4-(4-methoxybenzyloxy) 2,6,6-trimethyl-8-oxabicyclo [3.2.1] octane (AU188) as obtained in Example 130.

The product so obtained (821mg, 2.0mM) was treated with DDQ according to the procedures described in Example 7 and crystallized from a mixed solvent of ethyl acetate and hexane to obtain 410mg (71%) of 2,4-cis-1-benzoyloxy-2,6,6-trimethyl-8-oxabicyclo [3.2.1] octan-4-ol (AU252) as colorless needles. melting point: 87 to 88 °C.
IR (KBr, cm⁻¹): 3516, 1726, 1276, 997.
1H-NMR (CDCl₃, ppm): 0.95 (3H, d, J=7Hz), 1.31 (3H, s), 1.40 (3H, s), 1.51 (1H, q, J=12Hz), 1.58 (1H, brs; disappeared by the addition of heavy water), 2.07 (1H, d, J=13Hz), 2.08 (1H, m), 2.16 (1H, d, J=13Hz), 2.74 (1H, m), 3.80 (1H, d, J=4Hz), 4.13 (1H, m), 7.43 (2H, m), 7.56 (1H, m), 8.02 (2H, m).

### Example 134

To a solution of 2,4-cis-4-(4-methoxybenzyloxy)-2,6,6-trimethyl-8-oxabicyclo [3.2.1] octan-1-ol (1.2g, 3.9mM) obtained in Example 115 in tetrahydrofuran (10ml), 55% sodium hydride (0.18g, 4.1mM) was added at 0 °C. After 10 minutes, 4-nitrobenzoyl chloride (0.76g, 4.1mM) was added to the mixture, stirred for an hour and stirred at room temperature overnight. The reaction mixture was diluted with ethyl acetate, washed with an aqueous diluted potassium carbonate solution and with brine, dried over magnesium sulfate, filtered and concentrated. The residue was crystallized from a mixed solvent of ethyl acetate and hexane to obtain 1.1g (62%) of 2,4-cis-4-(4-methoxybenzyloxy)-1-(4-nitrobenzoyloxy)-2,6,6-trimethyl-8-oxabicyclo [3.2.1] octane as light yellow prisms. melting point: 116.5 to 117.5°C.
IR (KBr, cm⁻¹): 1740, 1530, 1350, 1276.
1H-NMR (CDCl₃, ppm): 0.93 (3H, d, J=7Hz), 1.32 (3H, s), 1.38 (3H, s), 1.50 (1H, q, J=12Hz), 2.11 (3H, m), 2.69 (1H, m), 3.81 (3H, s), 3.85 (1H, m), 3.92 (1H, m), 4.48 (1H, d, J=12Hz), 4.51 (1H, d, J=12Hz), 6.87 (2H, d, J=8Hz), 7.24 (2H, d, J=8Hz), 8.18 (2H, d, J=9Hz), 8.28 (2H, d, J=9Hz).

The product so obtained (1.00g, 2.2mM) was treated with DDQ according to the procedures described in Example 7 and crystallized from a mixed solvent of ethyl acetate and hexane to obtain 0.58g (78%) of 2,4-cis-1-(4-nitrobenzoyloxy):2,6,6-trimethyl-8-oxabicyclo [3.2.1 ] octan-4-ol (AU250) as colorless needles. melting point: 103 to 104°C.
IR (KBr, cm⁻¹): 3600-3100, 1740, 1530, 1350, 1276.
1H-NMr (CDCl₃, ppm): 0.98 (3H, d, J=7Hz), 1.33 (3H, s), 1.41 (3H, s), 1.53 (1H, q, J=12Hz), 2.12 (3H, m), 2.72 (1H, m), 3.82 (1H, d, J=4Hz), 4.16 (1H, m), 8.18 (2H, d, J=9Hz), 8.28 (2H, d, J=9Hz).

### Example 135

The product of Example 134 (436mg, 1.3mM) was reduced at nitro group according to the procedures described in Example 19 to obtain 360mg (90%) of 2,4-cis-1-(4-aminobenzoyloxy)-2,6,6-trimethyl-8-oxabicyclo [3.2.1] octan-4-ol (AU251) as an orange caramel.
IR (KBr, cm⁻¹): 3462, 3367, 1706, 1628, 1603, 1279, 1170.
1H-NMr (CDCl₃, ppm): 0.93 (3H, d, J=7Hz), 1.30 (3H, s), 1.39 (3H, s), 1.49 (1H, q, J=12Hz), 1.58 (1H, brs), 2.08 (3H, m), 2.72 (1H, m), 3.78 (1H, d, J=4Hz), 4.11 (3H, m; 1H by the addition of heavy water, m), 6.63 (2H, d, J=9Hz), 7.83 (2H, d, J=9Hz).

### Example 136

A solution of trifluoroacetic anhydride (4.16ml, 30mM) in methylenechloride (10ml) was added over 20minutes to a solution of dimethyl sulfoxide (2.84ml, 40mM) in methylene chloride (20ml) while it is maintained at -65 °C. After ten minutes, a solution of 2,4-cis-1-methoxy-2,6,6-trimethyl-8-oxabicyclo [3.2.1] octan-4-ol (4.00g, 20mM) obtained in Example 116 was added dropwise to the mixture at the same temperature, and after 30 minutes triethylamine was added at the same temperature. The mixture was then cooled down to room temperature and separated by the addition of water. The aqueous layer was extracted with methylene chloride, and organic layer was dried over magnesium sulfate, filtered and concentrated. The residue was purified by silica gel column chromatography using hexane-ethyl acetate (100:1 to 10:1) as an eluent and crystallized from hexane to obtain 3.25g (82%) of 1-methoxy-2,6,6-trimethyl-8-oxabicyclo [3.2.1] octan-4-one (AU163) as colorless plates . melting point: 38 to 39 °C.
IR (KBr, cm⁻¹): 1729, 1458, 1246, 1018.
1H-NMR (CDCl₃, ppm): 0.93 (3H, d, J=7Hz), 1.01 (3H, s), 1.30 (3H, s), 1.75 (1H, d, J=14Hz), 1.82 (1H, d, J=14Hz), 1.88 (1H, m), 2.48 (1H, m), 2.60 (1H, m), 3.42 (3H, s), 3.73 (1H, brs).
13C-NMR (CDCl₃, ppm): 15.40 (q), 22.29 (q), 29.95 (q), 38.27 (d), 38.35 (t), 39.74 (s), 42.33 (t), 49.59 (q), 90.76 (d), 110.14 (s), 206.27(s).

## Claims

1. A compound of formula I 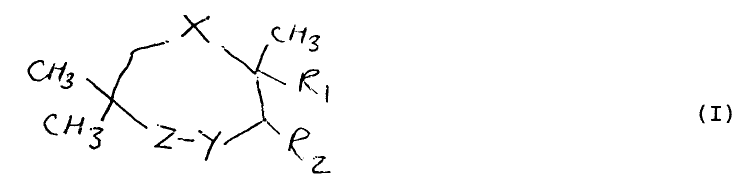 wherein
X represents a group -CR₃R₅-;
R₁ and R₂ each represent hydrogen atoms or R₁ and R₂ together represent a carbon-carbon bond;
Y and Z independently represent carbonyl groups or groups CHR₃;
R₅ represents a hydrogen atom, and each R₃ independently represents a group OR₄, or R₅ and a group R₃ represent an oxygen atom; and
each R₄ independently represents a hydrogen atom or an optionally substituted alkyl, alkenyl, aralkyl, aralkenyl or acyl group; with the proviso that where Y and Z each represent CHOH groups and R₁ and R₂ together represent a carbon-carbon bond, X represents other than a carbonyl group or an isomer thereof.

2. A compound as claimed in claim 1 selected from 5-hydroxy-4-(4-nitrobenzoyloxy)-2,6,6-trimethyl-2-cycloheptene-1-one; 4-(4-aminobenzoyloxy)-5-hydroxy-2,6,6-trimethyl-2-cycloheptene-1-one; 5-hydroxy-4-(1-methyl-2-pyrrolylcarbonyloxy)-2,6,6-trimethyl-2-cycloheptene-1-one; 5-(2,4-diaminobenzoyloxy)-4-methoxy-2,6,6-trimethyl-2-cycloheptene-1-one; 2,4-cis-1-methoxy-2,6,6-trimethyl-8-oxabicyclo [3.2.1] heptane-4-ol; and 4-(4-aminobenzoyloxy)-1-methoxy-2,6,6-trimethyl-8-oxabicyclo [3.2.1] oct-2-ene.

3. A compound as claimed in claim 1 being 4-(4-aminobenzoyloxy)-5-hydroxy-2,6,6-trimethyl-2- cycloheptene-1-one.

4. A compound as claimed in claim 1 being 2,4-cis-1-methoxy-2,6,6-trimethyl-8-oxabicyclo [3.2.1] heptane-4-ol.

5. A compound as claimed in claim 1 for use in the treatment of humans or animals.

6. A pharmaceutical composition comprising a compound of formula I as defined in claim 1 together with at least one pharmaceutical carrier or excipient.

7. The use of a compound of formula I as defined in claim 1 for the manufacture of an agent for use in combatting ulcers.

8. A process for the preparation of compounds of formula I as defined in claim 1 which process comprises at least one of the following steps:
(a) (to prepare compounds of formula I in which X is carbonyl, Z is CHOH and Y is CHOR₄' where R₄' is as defined for R₄ other than hydrogen or acyl) reacting a compound of formula II 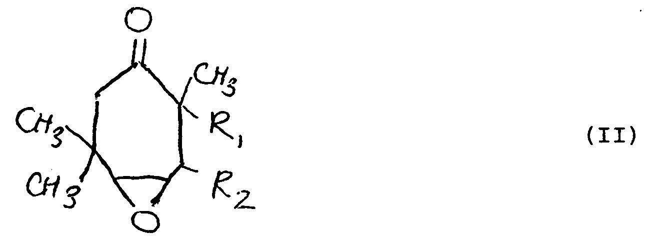 with an alcohol of formula R₄'OH (where R₄' is as hereinbefore defined);
(b) (to prepare compounds of formula I wherein Y and/or Z represents a group CHOR₄'' where R₄'' is an acyl group) acylating a compound of formula I wherein Y and/or Z represents a CHOH group;
(c) (to prepare compounds of formula I in which X is carbonyl, Z is CHOH and Y is CHOR₄′ where R₄′ is as defined for R₄ other than hydrogen or acyl) hydrolysing a compound of formula III 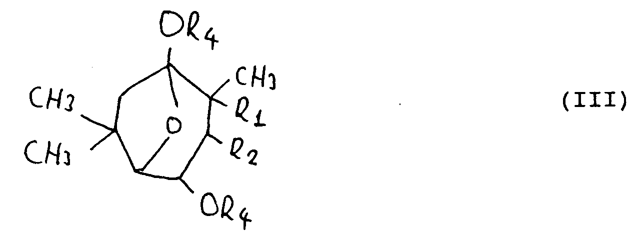
(d) (to prepare compounds of formula I in which X represents a group CR₅OR₄', Y represents a group CHOR₄′, R₄′ is as hereinbefore defined, Z represents a group CHR₃ and R₅ and the R₃ group of Z together represent an oxygen atom) reacting a compound of formula II 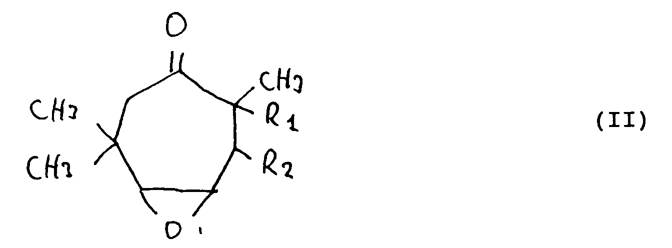 with an alcohol of formula R₄′OH (where R₄′ is as hereinbefore defined);
(e) (to prepare compounds of formula I in which X represents a group CR₅OR₄˝, Y represents a group CHOR₄′, R₄′ and R₄˝ are as hereinbefore defined, Z represents a group CHR₃ and R₅ and the R₃ group of Z together represent an oxygen atom) acylating a compound of formula I wherein X represents a carbonyl group, Y represents a group CHOR₄′ and Z represents a CHOH group;
(f) (to prepare compounds of formula I wherein at least one group R₄ is hydrogen) cleaving a protecting group R₄˝′ (where R₄˝′ is as defined for R₄ other than hydrogen) from a compound of formula I wherein at least one R₄ is a group R₄˝′;
(g) (to prepare compounds of formula I wherein X is carbonyl and Z is a group CHOR′₄) alkylating, alkenylating, aralkylating or aralkenylating a compound of formula IV 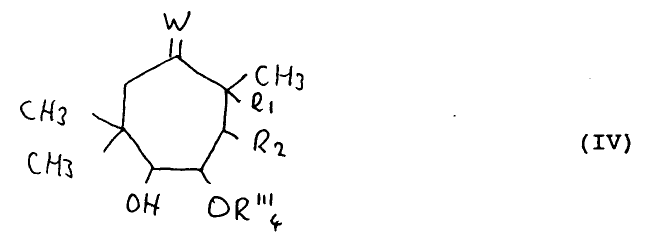 (where R₄˝′ is as hereinbefore defined and W is a carbonyl protecting group),deprotecting the carbonyl group and if desired cleaving protecting group R₄˝′ from the resulting compound of formula I;
(h) (to prepare compounds of formula I wherein X is a group CHOH) reducing a compound of formula I (wherein X is a carbonyl group, Y and Z are both CHOR₄˝′ groups and R₄˝′ is as hereinbefore defined) and if desired cleaving one or both protecting groups R₄˝′ from the resulting compound of formula I;
(i) (to prepare compounds of formula I in which X represents a group CR₅OR₄˝′, one of Y and Z represents a group CHR₃ and the other represents a group CHOR₄ and R₃ and R₅ together represent an oxygen atom) alkylating, alkenylating, aralkylating, aralkenylating or acylating a compound of formula I in which X is a carbonyl group and one of Y and Z is a CHOH group and the other is a CHOR₄ group; (j) (to prepare compounds of formula I wherein R₁ and R₂ are hydrogen) reducing a compound of formula I (as hereinbefore defined with the exclusion of the proviso and in which R₁ and R₂ together represent a carbon-carbon bond); and
(k) (to prepare compounds of formula I wherein Y or Z represents a carbonyl group) oxidizing a compound of formula I in which Y or Z represents a CHOH group.

9. A process as claimed in claim 8 for preparing a compound of formula V 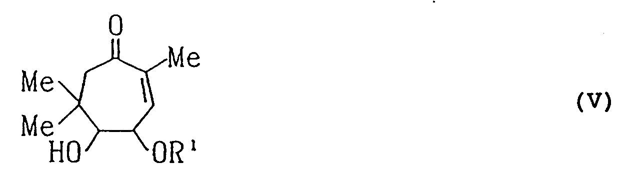 (wherein R¹ represents an alkyl, alkenyl, aralkenyl or aralkyl group) which comprises alcoholyzing eucarvone-4,5-oxide with an alcohol of formula VI
[R¹-OH] (VI)
(wherein R¹ is as hereinbefore defined in the presence of an acid catalyst).

10. A process as claimed in claim 8 for preparing a compound of formula VII 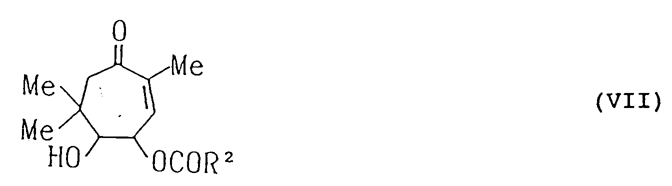 (wherein R² represents an alkyl, alkenyl, aralkenyl aromatic hydrocarbon or heterocyclic group) which comprises condensing Saishin N and a carboxylic acid of formula VIII
[R²COOH] (VIII)
(wherein R² is as hereinbefore defined) by acylation in the presence of carbodiimide.

11. A process as claimed in claim 8 for preparing a compound of formula IX 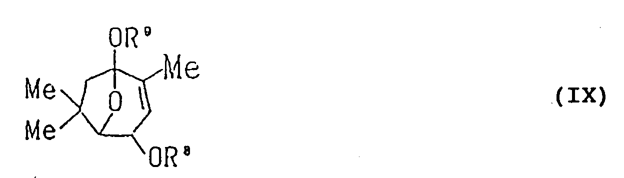 (wherein R⁸ represents an alkyl, alkenyl, aralkyl, aralkenyl or acyl group, and R⁹ represents an alkyl, alkenyl, aralkenyl or aralkyl group) which comprises reacting a compound of formula X 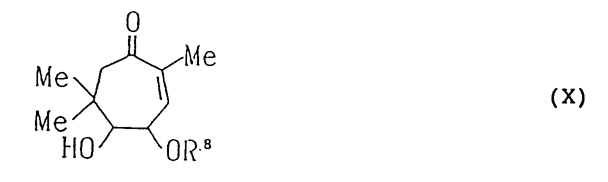 (wherein R⁸ is as hereinbefore defined) with an alcohol of formula XI
[R⁹-OH] (XI)
(wherein R⁹ is as hereinbefore defined) in the presence of an acid catalyst.

12. A process as claimed in claim 8 for preparing a compound of formula XII 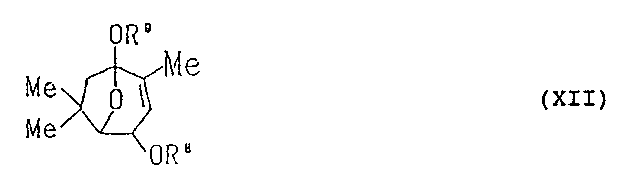 (wherein R⁸ represents an alkyl, alkenyl, aralkyl, aralkenyl or acyl group, and R⁹ represents an alkyl, alkenyl, aralkyl, aralkenyl or acyl group) which comprises alkylating, alkenylating, aralkylating, aralkenylating or acylating a compound of formula XIII 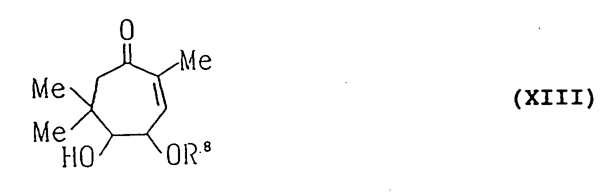 (wherein R⁸ is as hereinbefore defined)

13. A process as claimed in claim 8 for preparing a compound of formula XIV 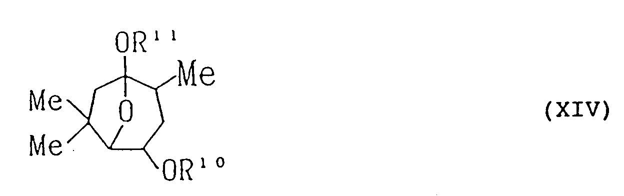 (wherein R¹⁰ represents an alkyl, alkenyl, aralkyl, aralkenyl or acyl group, and R¹¹ represents an alkyl, alkenyl, aralkenyl or aralkyl group) which comprises reacting a compound of formula XV 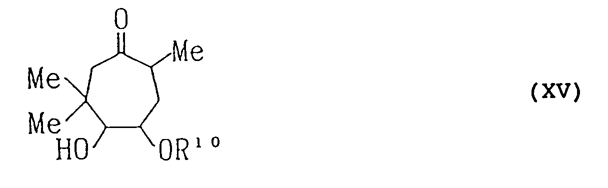 (wherein R¹⁰ is as hereinbefore defined) with an alcohol of formula XVI
[R¹¹-OH] (XVI)
(wherein R¹¹ is as hereinbefore defined) in the presence of an acid catalyst.

## Patentansprüche

1. Verbindung der Formel I 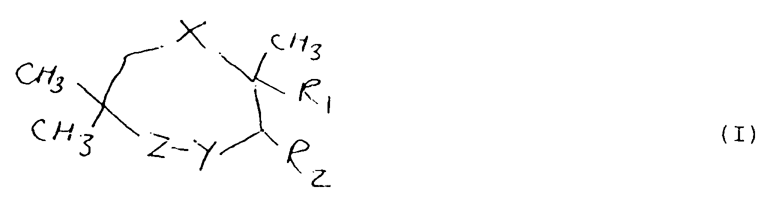 worin
X für eine Gruppe -CR₃R₅- steht;
R₁ und R₂ jeweils Wasserstoffatomen entsprechen oder R₁ und R₂ zusammen einer Kohlenstoff-Kohlenstoff-Bindung entsprechen;
Y und Z unabhängig voneinander Carbonylgruppen oder CHR₃-Gruppen bedeuten;
R₅ ein Wasserstoffatom und jedes R₃ unabhängig eine OR₄-Gruppe ist oder R₅ und eine R₃-Gruppe ein Sauerstoffatom sind; und
jedes R₄ unabhängig für ein Wasserstoffatom oder eine wahlweise substituierte Alkyl-, Alkenyl-, Aralkyl-, Aralkenyl- oder Acylgruppe steht; wobei die Bedingung erfüllt sein muß, daß X eine andere als eine Carbonylgruppe oder ein Isomer davon bedeutet, wenn Y und Z jeweils CHOH-Gruppen und R₁ und R₂ zusammen eine Kohlenstoff-Kohlenstoff-Bindung sind.

2. Verbindung nach Anspruch 1, die ausgewählt wurde unter 5-Hydroxy-4-(4-nitrobenzoyloxy)-2,6,6-trimethyl-2-cyclohepten-1-on; 4-(4-Aminobenzoyloxy)-5-hydroxy-2,6,6-trimethyl-2-cyclohepten-1-on; 5-Hydroxy-4-(1-methyl-2-pyrrolylcarbonyloxy)-2,6,6-trimethyl-2-cyclohepten-1-on; 5-(2,4-Diaminobenzoyloxy)-4-methoxy-2,6,6-trimethyl-2-cyclohepten-1-on; 2,4-cis-1-Methoxy-2,6,6-trimethyl-8-oxabicyclo [3.2.1] heptan-4-ol; und 4-(4-Aminobenzoyloxy)-1-methoxy-2,6,6,-trimethyl-8-oxabicyclo [3.2.1] oct-2-en.

3. Verbindung nach Anspruch 1, bei der es sich um 4-(4-Aminobenzoyloxy)-5-hydroxy-2,6,6-trimethyl-2-cyclohepten-1-on handelt.

4. Verbindung nach Anspruch 1, bei der es sich um 2,4-cis-1-Methoxy-2,6,6-trimethyl-8-oxabicyclo [3.2.1] heptan-4-ol handelt.

5. Verbindung nach Anspruch 1 zur Anwendung bei der human- und veterinärmedizinischen Behandlung.

6. Pharmazeutische Zusammensetzung, die aus einer Verbindung der Formel I, wie sie in Anspruch 1 definiert ist, und mindestens einer pharmazeutischen Trägersubstanz oder einem pharmazeutischen Bindemittel besteht.

7. Verwendung einer Verbindung der Formel I, wie sie in Anspruch 1 definiert ist, für die Herstellung eines Heilmittels zur Anwendung bei der Bekämpfung von Ulzera.

8. Verfahren zur Herstellung von Verbindungen der Formel I, wie sie in Anspruch 1 definiert sind, das mindestens einen der folgenden Schritte umfaßt:
(a) (zur Herstellung von Verbindungen der Formel I, worin X Carbonyl, Z CHOH und Y CHOR₄' bedeutet, wobei es sich bei R₄' im Vergleich zur Definition von R₄ nicht um Wasserstoff oder Acyl handelt) Umsetzen einer Verbindung der Formel II 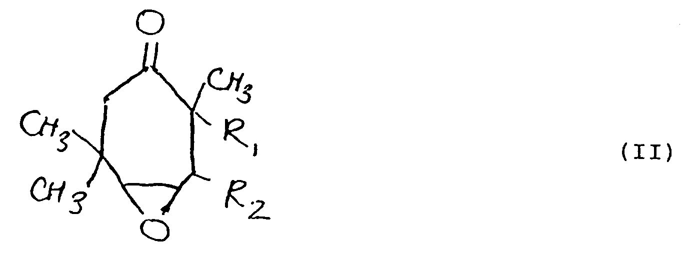 mit einem Alkohol der Formel R₄'OH (wobei R₄' der obigen Definition entspricht);
(b) (zur Herstellung von Verbindungen der Formel I, worin Y und/oder Z für eine CHOR₄''-Gruppe stehen, wobei R₄'' eine Acylgruppe ist) Acylieren einer Verbindung der Formel I, worin Y und/oder Z eine CHOH-Gruppe bedeuten;
(c) (zur Herstellung von Verbindungen der Formel I, worin X Carbonyl, Z CHOH und Y CHOR₄' bedeuten, wobei es sich bei R₄' im Vergleich zur Definition von R₄ nicht um Wasserstoff oder Acyl handelt) Hydrolysieren einer Verbindung der Formel III 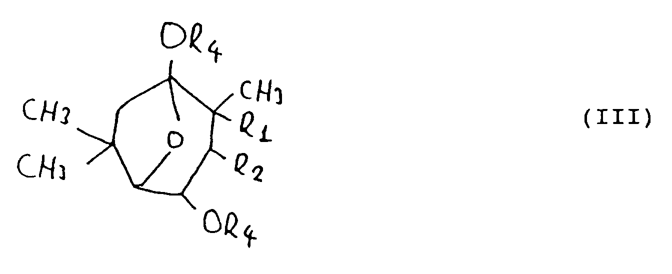
(d) (zur Herstellung von Verbindungen der Formel I, worin X für eine CR₅OR₄'-Gruppe, Y für eine CHOR₄'-Gruppe steht, R₄' wie oben definiert ist, Z für eine CHR₃-Gruppe und R₅ und die R₃-Gruppe von Z zusammen für ein Sauerstoffatom stehen) Umsetzen einer Verbindung der Formel II 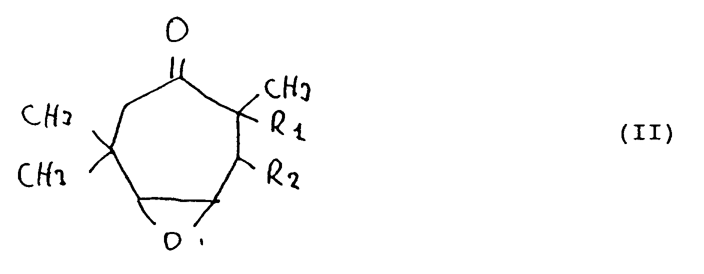 mit einem Alkohol der Formel R₄'OH (worin R₄' der obigen Definition entspricht);
(e) (zur Herstellung von Verbindungen der Formel I, worin X eine CR₅OR₄''-Gruppe, Y eine CHOR₄'-Gruppe ist, R₄' und R₄'' wie oben definiert sind, Z eine CHR₃-Gruppe und R₅ und die R₃-Gruppe von Z zusammen ein Sauerstoffatom sind) Acylieren einer Verbindung der Formel I, worin X eine Carbonylgruppe, Y eine CHOR₄'-Gruppe und Z eine CHOH-Gruppe bedeutet;
(f) (zur Herstellung von Verbindungen der Formel I, worin mindestens eine R₄-Gruppe Wasserstoff ist) Abspalten einer Schutzgruppe R₄''' (wobei es sich bei R₄''' im Vergleich zur Definition von R₄ nicht um Wasserstoff handelt) von einer Verbindung der Formel I, worin mindestens ein R₄ eine R₄'''-Gruppe ist;
(g) (zur Herstellung von Verbindungen der Formel I, worin X für Carbonyl und Z für eine CHOR₄'-Gruppe steht) Alkylieren, Alkenylieren, Aralkylieren oder Aralkenylieren einer Verbindung der Formel IV 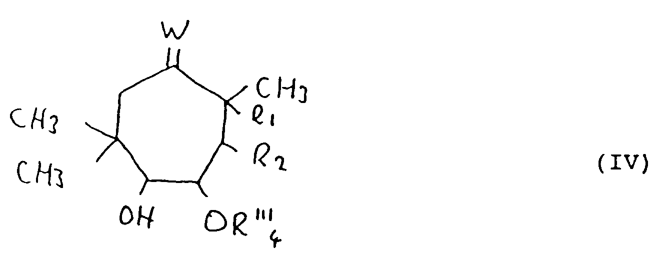 (worin R₄''' der obigen Definition entspricht und W eine Carbonyl-Schutzgruppe bedeutet), Entfernen der Carbonyl-Schutzgruppe und, falls gewünscht, Abspalten der R₄'''-Schutzgruppe von der resultierenden Verbindung der Formel I;
(h) (zur Herstellung von Verbindungen der Formel I, worin X eine CHOH-Gruppe darstellt) Reduzieren einer Verbindung der Formel I (worin X für Carbonyl, Y und Z jeweils für CHOR₄'''-Gruppen stehen und R₄''' der obigen Definition entspricht) und, falls gewünscht. Abspalten einer oder beider R₄'''-Schutzgruppen von der resultierenden Verbindung der Formel I;
(i) (zur Herstellung von Verbindungen der Formel I, worin X eine CR₅OR₄'''-Gruppe, Y oder Z eine CHR₃-Gruppe ist und das jeweils andere Symbol eine CHOR₄-Gruppe und R₃ und R₅ zusammen ein Sauerstoffatom sind) Alkylieren, Alkenylieren, Aralkylieren, Aralkenylieren oder Acylieren einer Verbindung der Formel I (worin X für eine Carbonylgruppe, Y oder Z für eine CHOH-Gruppe und das jeweils andere Symbol für eine CHOR₄-Gruppe steht;
(j) (zur Herstellung von Verbindungen der Formel I, worin R₁ und R₂ Wasserstoff bedeuten) Reduzieren einer Verbindung der Formel I (wie vorstehend definiert, jedoch mit Ausnahme der gestellten Bedingung, und worin R₁ und R₂ gemeinsam eine Kohlenstoff-Kohlenstoff-Bindung bedeuten); und
(k) (zur Herstellung von Verbindungen der Formel I, worin Y oder Z eine Carbonylgruppe darstellen) Oxidieren einer Verbindung der Formel I, worin Y oder Z eine CHOH-Gruppe bedeuten.

9. Verfahren nach Anspruch 8 zur Herstellung einer Verbindung der Formel V 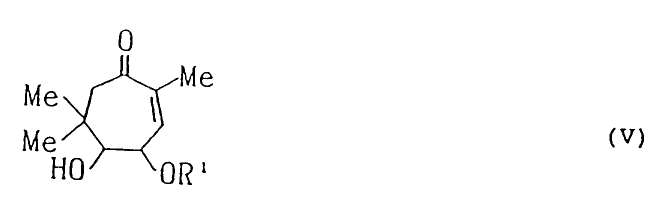 (worin R¹ für eine Alkyl-, Alkenyl-, Aralkenyl- oder Aralkylgruppe steht), das das Alkoholisieren von Eucarvon-4,5-oxid mit einem Alkohol der Formel VI
[R¹-OH] (VI)
(worin R¹ der obigen Definition entspricht) in Gegenwart eines Säurekatalysators umfaßt.

10. Verfahren nach Anspruch 8 zur Herstellung einer Verbindung der Formel VII 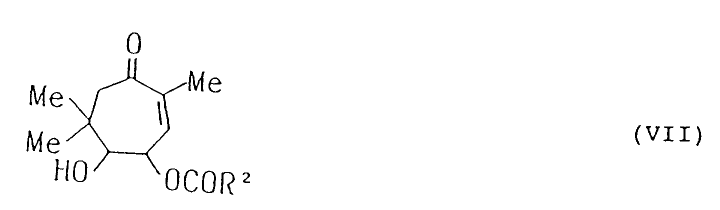 (worin R² eine aromatische Alkyl-, Alkenyl-, Aralkenyl- Kohlenwasserstoffgruppe oder heterocyclische Gruppe bedeutet), das die Kondensierung von Saishin N und einer Carbonsäure der Formel VIII
[R²COOH] (VIII)
(worin R² der obigen Definition entspricht) durch Acylierung in Gegenwart von Carbodiimid umfaßt.

11. Verfahren nach Anspruch 8 zur Herstellung einer Verbindung der Formel IX 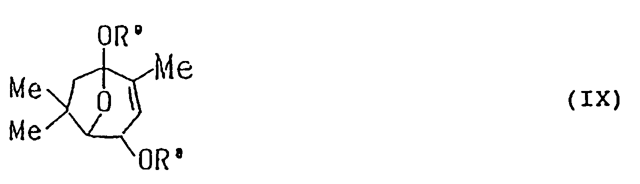 (worin R⁸ für eine Alkyl-, Alkenyl-, Aralkyl-, Aralkenyl- oder Acylgruppe und R⁹ für eine Alkyl-, Alkenyl- Aralkenyl- oder Aralkylgruppe steht), das das Umsetzen einer Verbindung der Formel X 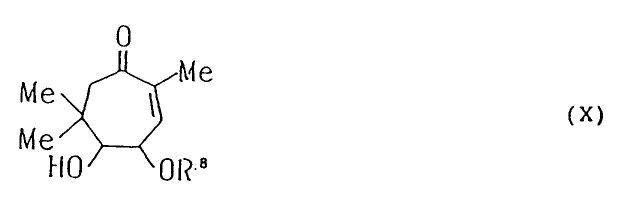 (worin R⁸ der obigen Definition entspricht) mit einem Alkohol der Formel XI
[R⁹-OH] (XI)
(worin R⁹ wie oben definiert ist) in Gegenwart eines Säurekatalysators umfaßt.

12. Verfahren nach Anspruch 8 zur Herstellung einer Verbindung der Formel XII 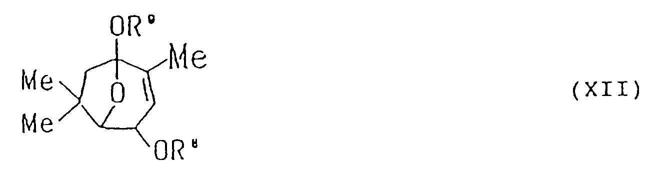 (worin R⁸ eine Alkyl-, Alkenyl-, Aralkyl-, Aralkenyl- oder Acylgruppe und R⁹ eine Alkyl-, Alkenyl-, Aralkyl-, Aralkenyl- oder Acylgruppe ist), das das Alkylieren, Alkenylieren, Aralkylieren, Aralkenylieren oder Acylieren einer Verbindung der Formel XIII 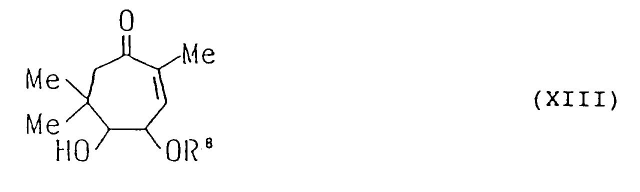 (worin R⁸ wie oben definiert ist) umfaßt.

13. Verfahren nach Anspruch 8 zur Herstellung einer Verbindung der Formel XIV 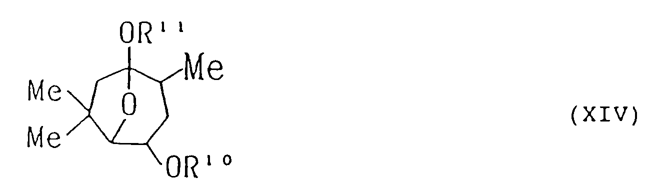 (worin R¹⁰ für eine Alkyl-, Alkenyl-, Aralkyl-, Aralkenyl- oder Acylgruppe und R¹¹ eine Alkyl-, Alkenyl-, Aralkenyl- oder Aralkylgruppe steht), das das Umsetzen einer Verbindung der Formel XV 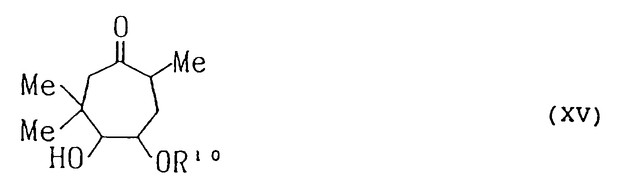 (worin R¹⁰ wie oben definiert ist) mit einem Alkohol der Formel XVI
[R¹¹-OH] (XVI)
(worin R¹¹ der obigen Definition entspricht) in Gegenwart eines Säurekatalysators umfaßt.

## Revendications

1. Composé de formule I 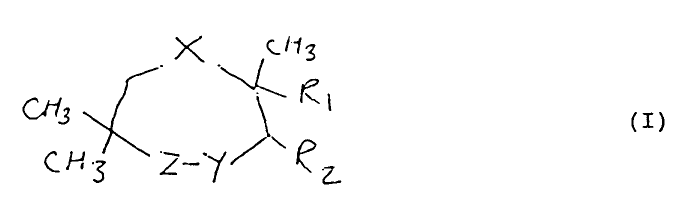 dans laquelle
X représente un groupe -CR₃R₅-;
R₁ et R₂ représentent chacun des atomes d'hydrogène ou R₁ et R₂ représentent ensemble une liaison carbone-carbone;
Y et Z représentent indépendamment des groupes carbonyle ou des groupes CHR₃;
R₅ représente un atome d'hydrogène, et chaque R₃ indépendamment représente un groupe OR₄, ou R₅ et un groupe
R₃ représentent un atome d'oxygène; et
chaque R₄ représente indépendamment un atome d'hydrogène ou un groupe alkyle, alcényle, aralkyle, aralcényle ou acyle éventuellement substitué; avec la condition que lorsque Y et Z représentent chacun des groupes CHOH et R₁ et R₂ représentent ensemble une liaison carbone-carbone, X représente autre chose que un groupe carbonyle ou un isomère de celui-ci.

2. Composé selon la revendication 1 choisi parmi la 5-hydroxy-4-(4-nitrobenzoyloxy)-2,6,6-triméthyl-2-cycloheptène-1-one; la 4-(4-aminobenzoyloxy)-5-hydroxy-2,6,6-triméthyl-2-cycloheptène-1-one; la 5-hydroxy-4-(1-méthyl-2-pyrrolylcarbonyloxy)-2,6,6-triméthyl-2-cycloheptène-1-one; la 5-(2,4-diaminobenzoyloxy)-4-méthoxy-2,6,6-triméthyl-2-cycloheptène-1-one; le 2,4-cis-1-méthoxy-2,6,6-triméthyl-8-oxabicyclo [3.2.1] heptane-4-ol; et le 4-(4-aminobenzoyloxy)-1-méthoxy-2,6,6-triméthyl-8-oxabicyclo [3.2.1] oct-2-ène.

3. Composé selon la revendication 1 qui est la 4-(4-aminobenzoyloxy)-5-hydroxy-2,6,6-triméthyl-2-cycloheptène-1-one.

4. Composé selon la revendication 1 qui est le 2,4-cis-1-méthoxy-2,6,6-triméthyl-8-oxabicyclo [3.2.1] heptane-4-ol.

5. Composé selon la revendication 1 pour utilisation dans le traitement des êtres humains et des animaux.

6. Composition pharmaceutique comprenant un composé de formule I tel que défini dans la revendication 1 avec au moins un support ou un excipient pharmaceutiquement acceptable.

7. Utilisation d'un composé de formule I tel que défini dans la revendication 1 pour la fabrication d'un agent destiné à être utilisé pour combattre les ulcères.

8. Procédé pour la préparation des composés de formule I tels que définis dans la revendication 1, ledit procédé comprenant au moins une des étapes suivantes:
(a) (pour préparer des composés de formule I dans laquelle X est un carbonyle, Z est CHOH et Y est CHOR₄' où R₄' est tel que défini pour R₄ autre qu'un hydrogène ou un acyle) faire réagir un composé de formule II 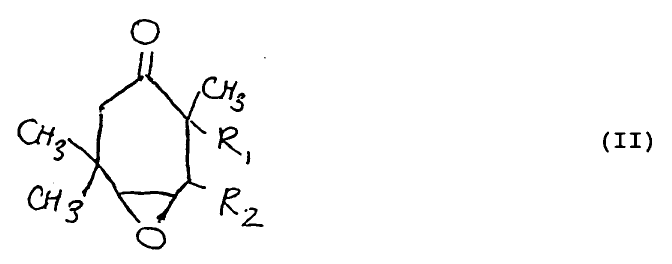 avec un alcool de formule R₄'OH (où R₄' est tel que défini précédemment);
(b) (pour préparer des composés de formule I dans laquelle Y et/ou Z représente un groupe CHOR₄'' où R₄'' est un groupe acyle) acyler un composé de formule I dans laquelle Y et/ou Z représente un groupe CHOH;
(c) pour préparer des composés de formule I dans laquelle X est un carbonyle, Z est CHOH et Y est CHOR₄' où R₄' est tel que défini pour R₄ autre qu'un hydrogène ou un acyle) hydrolyser un composé de formule III 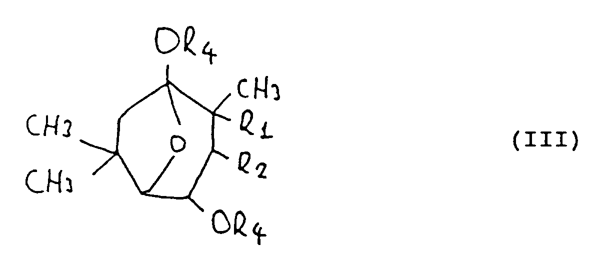
(d) (pour préparer des composés de formule I dans laquelle X représente un groupe CR₅OR₄', Y représente un groupe CHOR₄', R₄' est tel que défini précédemment, Z représente un groupe CHR₃ et R₅ et le groupe R₃ de Z ensemble représentent un atome d'oxygène) faire réagir un composé de formule II 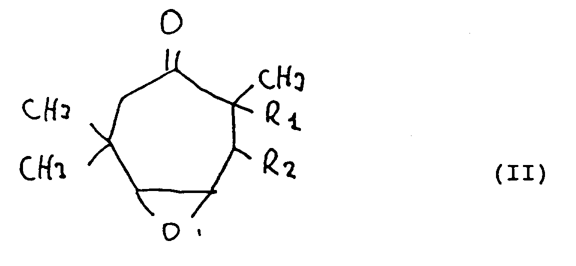 avec un alcool de formule R₄'OH (où R₄' est tel que défini précédemment);
(e) (pour préparer des composés de formule I dans laquelle X représente un groupe CR₅OR₄'', Y représente un groupe CHOR₄', R₄' et R₄'' sont tels que définis précédemment, Z représente un groupe CHR₃ et R₅ et le groupe R₃ de Z ensemble représentent un atome d'oxygène) acyler un composé de formule I dans laquelle X représente un groupe carbonyle, Y représente un groupe CHOR₄' et Z représente un groupe CHOH;
(f) (pour préparer des composés de formule I dans laquelle au moins un groupe R₄ est un hydrogène) effectuer le clivage d'un groupe protecteur R₄''' (où R₄''' est tel que défini pour R₄ autre que un hydrogène) d'un composé de formule I dans laquelle au moins un R₄ est un groupe R₄''';
(g) (pour préparer des composés de formule I dans laquelle X est un groupe carbonyle et Z est un groupe CHOR'₄) alkyler, alcényler, aralkyler ou un aralcényler un composé de formule IV 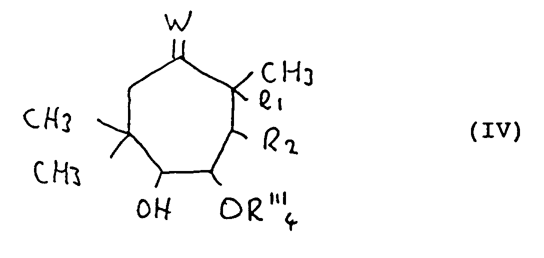 (où R₄''' est tel que défini précédemment et W est un groupe protecteur du carbonyle), déprotéger le groupe carbonyle et si cela est nécessaire effectuer le clivage du groupe protecteur R₄''' du composé de formule I résultant;
(h) (pour préparer des composés de formule I dans laquelle X est un groupe CHOH) réduire un composé de formule I (dans laquelle X est un groupe carbonyle, Y et Z sont les deux des groupes CHOR₄''' et R₄''' est tel que défini précédemment) et si nécessaire effectuer le clivage de l'un ou des deux groupes protecteurs R₄''' du composé de formule I résultant;
(i) (pour préparer des composés de formule I dans laquelle X représente un groupe CR₅OR₄''', un de Y et Z représente un groupe CHR₃ et l'autre représente un groupe CHOR₄ et R₃ et R₅ représentent ensemble un atome d'oxygène) alkyler, alcényler, aralkyler, aralcényler ou acyler un composé de formule I dans laquelle X est un groupe carbonyle et l'un de Y et Z est un groupe CHOH et l'autre est un groupe CHOR₄;
(j) (pour préparer des composés de formule I dans laquelle R₁ et R₂ sont un hydrogène) réduire un composé de formule I (tel que défini précédemment avec l'exclusion de la condition et dans lequel R₁ et R₂ représentent ensemble une liaison carbone-carbone);
(k) pour préparer des composés de formule I dans laquelle Y ou Z représente un groupe carbonyle) oxyder un composé de formule I dans laquelle Y ou Z représente un groupe CHOH.

9. Procédé selon la revendication 8 pour la préparation d'un composé de formule V 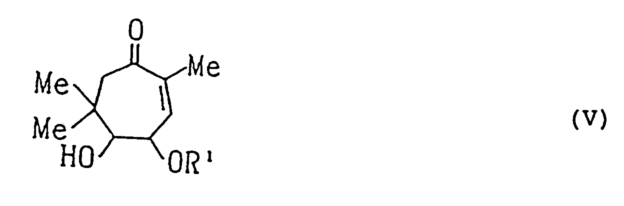 (dans laquelle R¹ représente un groupe alkyle, alcényle, aralcényle ou aralkyle) qui comprend le fait d'alcooliser un eucarvone-4,5-oxide avec un alcool de formule VI
[R¹-OH] (VI)
(dans laquelle R¹ est tel que défini précédemment en présence d'un catalyseur acide).

10. Procédé selon la revendication 8 pour la préparation d'un composé de formule VII 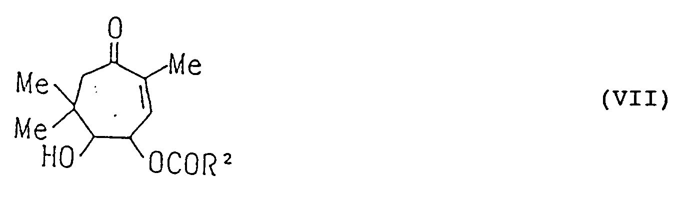 (dans laquelle R² représente un groupe alkyle, alcényle, aralcényle, hydrocarbure aromatique ou hétérocyclique) qui comprend le fait de condenser la Saishin N et un acide carboxylique de formule VIII
[R²COOH] (VIII)
(dans laquelle R² est tel que défini précédemment) par une acylation en présence d'un carbodiimide.

11. Procédé selon la revendication 8 pour la préparation d'un composé de formule IX 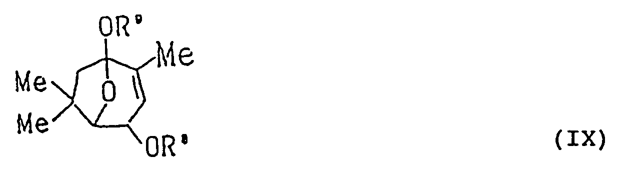 (dans laquelle R⁸ représente un groupe alkyle, alcényle, aralkyle, aralcényle ou acyle, et R⁹ représente un groupe alkyle, alcényle, aralcényle ou aralkyle) qui comprend le fait de faire réagir un composé de formule X 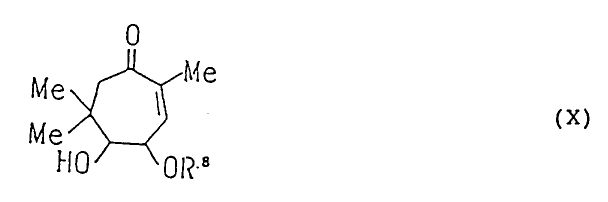 (dans laquelle R⁸ est tel que défini précédemment) avec un alcool de formule XI
[R⁹-OH] (XI)
(dans laquelle R⁹ est tel que défini précédemment) en présence d'un catalyseur acide.

12. Procédé selon la revendication 8 pour la préparation d'un composé de formule XII 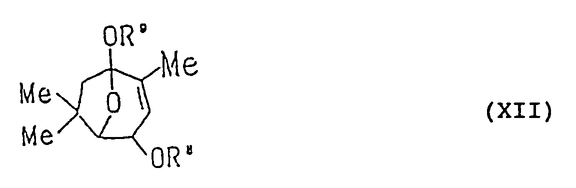 (dans laquelle R⁸ représente un groupe alkyle, alcényle, aralkyle, aralcényle ou acyle, et R⁹ représente un groupe alkyle, alcényle, aralkyle, aralcényle ou acyle) qui comprend l'alkylation, l'alcénylation, l'aralkylation, aralcénylation ou l'acylation d'un composé de formule XIII 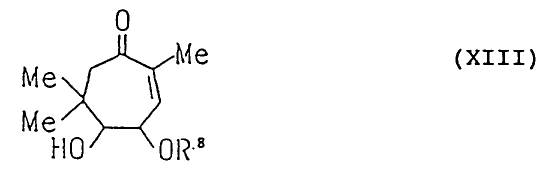 (dans laquelle R⁸ est tel que défini précédemment).

13. Procédé selon la revendication 8 pour la préparation d'un composé de formule XIV 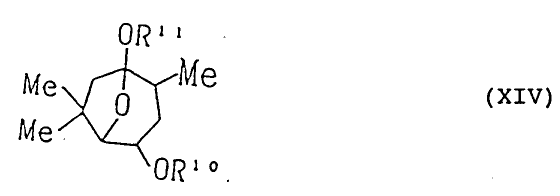 (dans laquelle R¹⁰ représente un groupe alkyle, alcényle, aralkyle, aralcényle ou acyle, et R¹¹ représente un groupe alkyle, alcényle, aralcényle ou aralkyle) qui comprend le fait de faire réagir un composé de formule XV 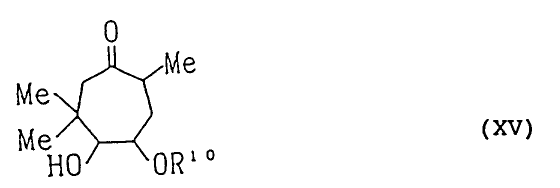 (dans laquelle R¹⁰ est tel que défini précédemment) avec un alcool de formule XVI
[R¹¹-OH] (XVI)
(dans laquelle R¹¹ est tel que défini précédemment) en présence d'un catalyseur acide.
